(19) **Europäisches Patentamt European Patent Office Office européen des brevets**

(11) **EP 4 752 156 A1**

(12) **EUROPEAN PATENT APPLICATION**
published in accordance with Art. 153(4) EPC

(43) Date of publication:
03.06.2026 Bulletin 2026/23

(21) Application number: 24844831.8

(22) Date of filing: 25.07.2024

(51) International Patent Classification (IPC):
*C07K 16/18* (2006.01)          *C12Q 1/68* (2018.01)
*A61K 39/395* (2006.01)       *A61K 31/713* (2006.01)
*A61P 35/00* (2006.01)

(52) Cooperative Patent Classification (CPC):
A61K 31/713; A61K 39/395; A61P 35/00;
C07K 16/18; C12Q 1/68

(86) International application number:
PCT/CN2024/107513

(87) International publication number:
WO 2025/021145 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.07.2023  CN 202310921036

(71) Applicant: Shanghai Yunxuan Biotechnologies,
Inc.
Shanghai 201306 (CN)

(72) Inventors:
• ZHANG, Zhushan
Shanghai 201306 (CN)
• ZHOU, Boya
Shanghai 201306 (CN)

(74) Representative: Vossius & Partner
Patentanwälte Rechtsanwälte mbB
Siebertstraße 3
81675 München (DE)

Remarks:
The complete document including Reference
Table(s) and the Sequence Listing(s) can be
downloaded from the EPO website

(54) **ANTIBODY AND USE THEREOF**

(57)    The present invention belongs to the field of biomedicine. Specifically, the present invention provides an immunogenic polypeptide derived from cTAGE5 protein and use thereof, and also provides an antibody that specifically binds to the immunogenic polypeptide and use thereof.

EP 4 752 156 A1

## Description

### Technical field

The present invention belongs to the field of biomedicine. Specifically, the present invention provides an immunogenic polypeptide derived from cTAGE5 protein and use thereof, and also provides an antibody specifically binding to said immunogenic polypeptide and use thereof.

### Background

Cutaneous T cell lymphoma-associated antigen 5 (cTAGE5), also known as MEA6 or MGEA6, is considered as a tumor marker for various tumors, such as meningioma, glioma, T-cell lymphoma and the like. However, while the cTAGE5 was studied as a tumor marker, it was found that the cTAGE5 also existed in normal cells and was indispensable for the cellular functions, such as participation in the transport and export by the endoplasmic reticulum (ER).

In previous researches on the function of cTAGE5, the intracellular part of cTAGE5/Mea6 is the main focus. It has been found that cTAGE5/Mea6, which is 804 aa in length and forms a complex with TANGO1 on the membrane at the output end (Lumen) of the ER, binds to the frame proteins Sec12, Sec22, Sec23 of COPII and promotes the output of collagen by COPII, but it does not leave the membrane together with COPII. Similar studies mostly study the behavior of cTAGE5/Mea6 within the cell membrane, which is an essential component for normal function of human cells. The amino acid sequence of cTAGE5/MEA6 outside the membrane is considered to be a signal peptide, and there is no report about the function of this sequence.

A variety of commercialized antibodies against cTAGE5 have been available, such as products HPA000387, HPA000922 from Sigma-Aldrich. However, these antibodies target intracellular fragments of cTAGE5/Mea6 (804 amino acids). There is no antibody against the extracellular domain of cTAGE5 at present.

### Brief description of the drawings

FIG. 1. Sequence structure of the Mea6 splicing variant of cTAGES.

FIG. 2 shows representative IHC results of rabbit polyclonal antibodies against ZZS01 and ZZS05.

FIG. 3 shows representative IHC results of 1D6 and F11 monoclonal antibodies.

FIG. 4. F11 monoclonal antibody significantly inhibited the growth of A-375 xenograft tumors in mice. A: Representative tumor morphology; B: Mouse weight change; C: Tumor volume change. The results were expressed as mean$\pm$SD (n=8). *p<0.01, significantly compared with the control group.

FIG. 5. shows that F11 monoclonal antibody significantly inhibited the growth of HCC1937 xenograft tumors in mice. A: representative tumor morphology; B: change in mouse weight; C: change in tumor volume. The results were expressed as mean$\pm$SD (n=8). **p<0.01, significant difference compared with the control group.

FIG. 6. Anti-ZZS05 antibody h32 mediated the apoptosis of tumor organoids in an ADCC model. A: pancreatic cancer organoid; B: small cell lung cancer organoid.

FIG. 7. human anti-ZZS05 antibody h35 mediated apoptosis of different tumor organoids.

FIG. 8. Human anti-ZZS05 antibody h35 mediated LDH release.

### Detailed description of the invention

#### Definitions

In the present disclosure, unless otherwise specified, scientific and technical terms used herein have meanings generally understood by those skilled in the art. Moreover, the terms and laboratory procedures used herein are those widely used in the art. Meanwhile, for better understanding of the present disclosure, the definitions and explanations of the related terms are provided below.

As used herein, the term "and/or" encompasses all combinations of the items connected by the term, and each combination should be regarded as having been listed individually herein. For example, "A and/or B" encompasses "A", "A and B" and "B". For example, "A, B and/or C" encompasses "A", "B", "C", "A and B", "A and C", "B and C" and "A and B and C".

The term "comprising" as used herein to describe a protein or nucleic acid sequence is meant to include a protein or nucleic acid that is composed of the sequence or has additional amino acid(s) or nucleotide(s) at one or both ends but still has the activity described herein. Moreover, it is well known in the art that the N-terminal methionine, encoded by the start codon, may be retained in certain practical cases (for example, when expressed in a particular expression system), but does not substantially affect the function of the polypeptide. Therefore, in the description and claims of the present application, when a specific polypeptide amino acid sequence is described, although it may not include a methionine encoded by a start codon at an N-terminal, the sequence including the methionine is also included, and correspondingly, the encoded nucleotide sequence may also include a start codon.

As used herein, "antibody" refers to an immunoglobulin and an immunoglobulin fragment, no matter it is a natural one or is partially or completely synthesized (e.g., recombinantly produced), including any fragment retaining the binding specificity of the full-length immunoglobulin molecule and at least including a part of the variable region of the immunoglobulin molecule. Therefore, an antibody includes any protein having a binding domain that is homologous or substantially homologous to an immunoglobulin antigen-binding domain (antibody binding site). An antibody includes an antibody fragment. As used herein, the term "antibody" includes synthetic antibodies, recombinantly produced antibodies, polyclonal antibodies, monoclonal antibodies, multi-specific antibodies (e.g., bispecific antibodies), human antibodies, non-human antibodies, camel antibodies, single domain antibodies, humanized antibodies, chimeric antibodies, intracellular antibodies, and antibody fragments, such as, but not limited to, Fab fragments, Fab' fragments, F(ab')$_2$ fragments, Fv fragments, disulfide-linked Fv (dsFv), Fd fragments, Fd' fragments, single chain Fv (scFv), single chain Fab (scFab), bispecific antibodies, anti-idiotypic (anti-Id) antibodies, or antigen-binding fragments of any of the foregoing antibodies. The antibodies provided herein include any member of any immunoglobulin type (e.g., IgG, IgM, IgD, IgE, IgA or IgY), any class (e.g., IgG1, IgG2, IgG3, IgG4, IgA1 or IgA2) or subclass (e.g., IgG2a or IgG2b).

As used herein, an "antibody fragment" or "antigen-binding fragment" of an antibody refers to any portion of a full-length antibody that is less than full length but contains at least a portion of the variable region of the antibody that binds antigen (e.g. one or more CDRs and/or one or more antibody combining sites) and thus retains the binding specificity, and at least a portion of the specific binding ability of the full-length antibody. Hence, an antigen-binding fragment refers to an antibody fragment that contains an antigen-binding portion that binds to the same antigen as the antibody from which the antibody fragment is derived. Antibody fragments include antibody derivatives produced by enzymatic treatment of full-length antibodies, as well as synthetically, e.g. recombinantly produced derivatives. An antibody fragment is included among antibodies. Examples of antibody fragments include, but are not limited to, Fab, Fab', F(ab')$_2$, single-chain Fv (scFv), Fv, dsFv, diabody, Fd and Fd' fragments and other fragments, including modified fragments (see, for example, Methods in Molecular Biology, Vol 207: Recombinant Antibodies for Cancer Therapy Methods and Protocols (2003); Chapter 1; p 3-25, Kipriyanov). The fragment can include multiple chains linked together, such as by disulfide bridges and/or by peptide linkers. An antibody fragment generally contains at least or about 50 amino acids and typically at least or about 200 amino acids. An antigen-binding fragment includes any antibody fragment that when inserted into an antibody framework (such as by replacing a corresponding region) results in an antibody that immune-specifically binds (i.e. exhibits Ka of at least or at least about $10^7$- $10^8$ M$^{-1}$) to the antigen.

As used herein, "monoclonal antibody" refers to a population of identical antibodies, meaning that each individual antibody molecule in a population of monoclonal antibodies is identical to the others. This property is in contrast to that of a polyclonal population of antibodies, which contains antibodies having a plurality of different sequences. Monoclonal antibodies can be produced by a number of well-known methods (Smith et al. (2004) J. Clin. Pathol. 57, 912-917; and Nelson et al., J Clin Pathol (2000), 53, 111-117). For example, monoclonal antibodies can be produced by immortalization of a B cell, for example through fusion with a myeloma cell to generate a hybridoma cell line or by infection of B cells with virus such as EBV. Recombinant technology also can be used to produce antibodies in vitro from clonal populations of host cells by transforming the host cells with plasmids carrying artificial sequences of nucleotides encoding the antibodies.

As used herein, the term "hybridoma" or "hybridoma cell" refers to a cell or cell line (typically a myeloma or lymphoma cell) produced by the fusion of antibody-producing lymphocytes and non-antibody-producing cancer cells. As is known to those of ordinary skill in the art, hybridoma may proliferate and continue to produce specific monoclonal antibodies. Methods for producing a hybridoma are known in the art (see, for example, Harlow & Lane, 1988). When referring to the term "hybridoma" or "hybridoma cell", it also includes subcloned and progeny cells of the hybridoma.

As used herein, a "conventional antibody" refers to an antibody that contains two heavy chains (which can be denoted H and H') and two light chains (which can be denoted L and L') and two antibody combining sites, where each heavy chain can be a full-length immunoglobulin heavy chain or any functional region thereof that retains antigen-binding capability (e.g. heavy chains include, but are not limited to, $V_H$, chains $V_H$-$C_H$1 chains and $V_H$-$C_H$1-$C_H$2-$C_H$3 chains), and each light chain can be a full-length light chain or any functional region of (e.g. light chains include, but are not limited to, $V_L$ chains and $V_L$-$C_L$ chains). Each heavy chain (H and H') pairs with one light chain (L and L', respectively).

As used herein, a full-length antibody is an antibody having two full-length heavy chains (e.g. $V_H$-$C_H$1-$C_H$2-$C_H$3 or $V_H$-$C_H$1-$C_H$2-$C_H$3-$C_H$4) and two full-length light chains ($V_L$-$C_L$) and hinge regions, such as human antibodies produced naturally by antibody secreting B cells and antibodies with the same domains that are synthetically produced.

As used herein, a dsFv refers to an Fv with an engineered intermolecular disulfide bond, which stabilizes the $V_H$-$V_L$ pair.

As used herein, a Fab fragment is an antibody fragment that results from digestion of a full-length immunoglobulin with papain, or a fragment having the same structure that is produced synthetically, e.g. by recombinant methods. A Fab fragment contains a light chain (containing a $V_L$ and $C_L$) and another chain containing a variable domain of a heavy chain ($V_H$) and one constant region domain of the heavy chain ($C_H1$).

As used herein, a F(ab')$_2$ fragment is an antibody fragment that results from digestion of an immunoglobulin with pepsin at pH 4.0-4.5, or a fragment having the same structure that is produced synthetically, e.g. by recombinant methods. The F(ab')$_2$ fragment essentially contains two Fab fragments where each heavy chain portion contains an additional few amino acids, including cysteine residues that form disulfide linkages joining the two fragments.

As used herein, a Fab' fragment is a fragment containing one half (one heavy chain and one light chain) of the F(ab')$_2$ fragment.

As used herein, an scFv fragment refers to an antibody fragment that contains a variable light chain ($V_L$) and variable heavy chain ($V_H$), covalently connected by a polypeptide linker in any order. The linker is of a length such that the two variable domains are bridged without substantial interference. Exemplary linkers are (Gly-Ser)$_n$ residues with some Glu or Lys residues dispersed throughout to increase solubility.

The term "chimeric antibody" is intended to refer to antibodies in which the variable region sequences are derived from one species and the constant region sequences are derived from another species, such as an antibody in which the variable region sequences are derived from a rat antibody and the constant region sequences are derived from a human antibody.

"Humanized" antibody refers to forms of non-human (e.g. rat) antibodies that are chimeric immunoglobulins, immunoglobulin chains, or fragments thereof (such as Fv, Fab, Fab', F(ab')$_2$ or other antigen-binding subsequences of antibodies) that contain minimal sequence derived from non-human immunoglobulin. Preferably, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity.

Furthermore, during humanization, it is also possible to mutate the amino acid residues within the CDR1, CDR2 and/or CDR3 regions of VH and/or VL, thereby improving one or more binding properties (e.g., affinity) of the antibody. A mutation, such as a PCR-mediated mutation, can be performed, the effect of which on antibody binding or other functional properties can be assessed using in vitro or in vivo tests as described herein. Typically, a conservative mutation is introduced. Such mutation can be an amino acid substitution, addition or deletion. In addition, the number of mutations within the CDRs typically does not exceed one or two. Thus, the humanized antibody of the invention also encompasses the antibody comprising one or two amino acid mutations within the CDRs.

As used herein, the term "epitope" refers to any antigenic determinant on an antigen to which the paratope of an antibody binds. Epitopic determinants typically contain chemically active surface groupings of molecules such as amino acids or sugar side chains and typically have specific three dimensional structural characteristics, as well as specific charge characteristics.

As used herein, a variable domain or variable region is a specific Ig domain of an antibody heavy or light chain that contains a sequence of amino acids that varies among different antibodies. Each light chain and each heavy chain has one variable region domain, $V_L$ and $V_H$, respectively. The variable domains provide antigen specificity, and thus are responsible for antigen recognition. Each variable region contains CDRs that are part of the antigen-binding site domain and framework regions (FRs).

As used herein, "antigen-binding domain" and "antigen-binding site," are used synonymously to refer to a domain within an antibody that recognizes and physically interacts with cognate antigen. A native conventional full-length antibody molecule has two conventional antigen-binding sites, each containing portions of a heavy chain variable region and portions of a light chain variable region. A conventional antigen-binding site contains the loops that connect the anti-parallel beta strands within the variable region domains. The antigen combining sites can contain other portions of the variable region domains. Each conventional antigen-binding site contains three hypervariable regions from the heavy chain and three hypervariable regions from the light chain. The hypervariable regions also are called complementarity-determining regions (CDRs).

As used herein, "hypervariable region," "HV," "complementarity-determining region" and "CDR" and "antibody CDR" are used interchangeably to refer to one of a plurality of portions within each variable region that together form an antigen-binding site of an antibody. Each variable region domain contains three CDRs, named CDR1, CDR2 and CDR3. The three CDRs are non-contiguous along the linear amino acid sequence, but are proximate in the folded polypeptide. The CDRs are located within the loops that join the parallel strands of the beta sheets of the variable domain. As described herein, one of skill in the art knows and can identify the CDRs based on Kabat or Chothia numbering (see e.g., Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917). Preferably, the CDR in the present invention is determined by the IMGT method[2,3]. Reference may be made to the methods described in the Examples of the present disclosure for details.

As used herein, framework regions (FRs) are the domains within the antibody variable region domains that are located within the beta sheets; the FR regions are comparatively more conserved, in terms of their amino acid sequences, than the hypervariable regions.

As used herein, a "constant region" domain is a domain in an antibody heavy or light chain that contains a sequence of amino acids that is comparatively more conserved than that of the variable region domain. In conventional full-length antibody molecules, each light chain has a single light chain constant region ($C_L$) domain and each heavy chain contains one or more heavy chain constant region ($C_H$) domains, which include, $C_H1$, $C_H2$, $C_H3$ and $C_H4$. Full-length IgA, IgD and IgG isotypes contain $C_H1$, $C_H2$, $C_H3$ and a hinge region, while IgE and IgM contain $C_H1$, $C_H2$, $C_H3$ and $C_H4$. $C_H1$ and $C_L$ domains extend the Fab arm of the antibody molecule, thus contributing to the interaction with antigen and rotation of the antibody arms. Antibody constant regions can serve effector functions, such as, but not limited to, clearance of antigens, pathogens and toxins to which the antibody specifically binds, e.g., through interactions with various cells, biomolecules and tissues.

As used herein, a functional region of an antibody is a portion of the antibody that contains at least a $V_H$, $V_L$, $C_H$ (e.g. $C_H1$, $C_H2$ or $C_H3$), $C_L$ or hinge region domain of the antibody, or at least a functional region thereof.

As used herein, a functional region of a $V_H$ domain is at least a portion of the full $V_H$ domain that retains at least a portion of the binding specificity of the full $V_H$ domain (e.g. by retaining one or more CDR of the full $V_H$ domain), such that the functional region of the $V_H$ domain, either alone or in combination with another antibody domain (e.g. $V_L$ domain) or region thereof, binds to antigen. Exemplary functional regions of $V_H$ domains are regions containing the CDR1, CDR2 and/or CDR3 of the $V_H$ domain.

As used herein, a functional region of a $V_L$ domain is at least a portion of the full $V_L$ domain that retains at least a portion of the binding specificity of the full $V_L$ domain (e.g. by retaining one or more CDRs of the full $V_L$ domain), such that the function region of the $V_L$ domain, either alone or in combination with another antibody domain (e.g. $V_H$ domain) or region thereof, binds to antigen. Exemplary functional regions of $V_L$ domains are regions containing the CDR1, CDR2 and/or CDR3 of the $V_L$ domain.

As used herein, "specifically bind" or "immunospecifically bind" with respect to an antibody or antigen-binding fragment thereof are used interchangeably herein and refer to the ability of the antibody or antigen-binding fragment to form one or more noncovalent bonds with a cognate antigen, by noncovalent interactions between the antibody combining site(s) of the antibody and the antigen. The antigen can be an isolated antigen or presented in a tumor cell. Typically, an antibody that immunospecifically binds (or that specifically binds) to a antigen is one that binds to the antigen with an affinity constant Ka of about or $1 \times 10^7$ M$^{-1}$ or $1x\ 10^8$ M$^{-1}$ or greater (or a dissociation constant ($K_d$) of $lx\ 10^{-7}$ M or $1 \times 10^{-8}$ M or less). Affinity constants can be determined by standard kinetic methodology for antibody reactions, for example, immunoassays, surface plasmon resonance (SPR) (Rich and Myszka (2000) Curr. Opin. Biotechnol 11:54; Englebienne (1998) Analyst. 123:1599), isothermal titration calorimetry (ITC) or other kinetic interaction assays known in the art (see, e.g., Paul, ed., Fundamental Immunology, 2nd ed., Raven Press, New York, pages 332-336 (1989); see also U.S. Pat. No. 7,229,619 for a description of exemplary SPR and ITC methods for calculating the binding affinity of antibodies). Instrumentation and methods for real time detection and monitoring of binding rates are known and are commercially available (e.g., BiaCore 2000, Biacore AB, Upsala, Sweden and GE Healthcare Life Sciences; Malmqvist (2000) Biochem. Soc. Trans. 27:335).

The term "compete" or "competes", as used herein with regard to an antibody, means that a first antibody, or an antigen-binding fragment thereof, binds to an epitope in a manner sufficiently similar to the binding of a second antibody, or an antigen-binding fragment thereof, such that the result of binding of the first antibody with its cognate epitope is detectably decreased in the presence of the second antibody compared to the binding of the first antibody in the absence of the second antibody. Alternatively, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope or ligand, whether to the same, greater, or lesser extent, the antibodies are said to "cross-compete" with each other for binding of their respective epitope(s). Both competing and cross-competing antibodies are encompassed by the present invention. Regardless of the mechanism by which such competition or cross-competition occurs (e.g., steric hindrance, conformational change, or binding to a common epitope, or fragment thereof), the skilled artisan would appreciate, based upon the teachings provided herein, that such competing and/or cross-competing antibodies are encompassed and can be useful for the methods disclosed herein.

As used herein, "polypeptide" refers to two or more amino acids covalently joined. The terms "polypeptide" and "protein" are used interchangeably herein.

An "isolated protein", "isolated polypeptide" or "isolated antibody" is a protein, polypeptide or antibody that by virtue of its origin or source of derivation (1) is not associated with naturally associated components that accompany it in its native state, (2) is free of other proteins from the same species, (3) is expressed by a cell from a different species, or (4) does not occur in nature. Thus, a polypeptide that is chemically synthesized or synthesized in a cellular system different from the cell from which it naturally originates will be "isolated" from its naturally associated components. A protein may also be rendered substantially free of naturally associated components by isolation, using protein purification techniques well known in the art.

In a peptide or protein, suitable conservative substitutions of amino acids are known to those of skill in this art and generally can be made without altering a biological activity of a resulting molecule. Those of skill in this art recognize that, in general, single amino acid substitutions in non-essential regions of a polypeptide do not substantially alter biological activity (see, e.g., Watson et al., Molecular Biology of the Gene, 4th Edition, 1987, The Benjamin/Cummings Pub. co., p.224).

As used herein, the terms "polynucleotide" and "nucleic acid molecule" refer to an oligomer or polymer containing at least two linked nucleotides or nucleotide derivatives, including a deoxyribonucleic acid (DNA) and a ribonucleic acid (RNA), joined together, typically by phosphodiester linkages.

As used herein, isolated nucleic acid molecule is one which is separated from other nucleic acid molecules which are present in the natural source of the nucleic acid molecule. An "isolated" nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding an antibody or antigen-binding fragments provided.

Sequence "identity" has an art-recognized meaning and the percentage of sequence identity between two nucleic acid or polypeptide molecules or regions can be calculated using published techniques. Sequence identity can be measured along the full length of a polynucleotide or polypeptide or along a region of the molecule. (See, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotides or polypeptides, the term "identity" is well known to skilled artisans (Carrillo, H. & Lipman, D., SIAM J Applied Math 48:1073 (1988)).

As used herein, "operably linked" with reference to nucleic acid sequences, regions, elements or domains means that the nucleic acid regions are functionally related to each other. For example, a promoter can be operably linked to nucleic acid encoding a polypeptide, whereby the promoter regulates or mediates the transcription of the nucleic acid.

As used herein, "expression" refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay and Bradford protein assay.

As used herein, a "host cell" is a cell that is used in to receive, maintain, reproduce and amplify a vector. A host cell also can be used to express the polypeptide encoded by the vector. The nucleic acid contained in the vector is replicated when the host cell divides, thereby amplifying the nucleic acids. The host cell may be a eukaryotic cell or a prokaryotic cell. Suitable host cells include, but are not limited to, CHO cells, various COS cells, HeLa cells, HEK cells such as HEK 293 cells.

Codon optimization refers to the replacement of at least one codon (eg, about or more than about 1, 2, 3, 4, 5, 10, 15, 20, 25, 50 or more codons) of a native sequence by a codon that is used more frequently or most frequently in the gene of the host cell, modifying the nucleic acid sequence while maintaining the native amino acid sequence to enhance expression in the host cell of interest. Different species show specific preferences for certain codons of a particular amino acid. Codon preference (difference in codon usage between organisms) is often associated with the efficiency of translation of messenger RNA (mRNA), which is believed to depend on the nature of the translated codon and the availability of specific transfer RNA (tRNA) molecules. The advantages of selected tRNAs within cells generally reflect the most frequently used codons for peptide synthesis. Therefore, genes can be customized to be best gene expressed in a given organism based on codon optimization. The codon usage table can be easily obtained, for example, in the Codon Usage Database available at www.kazusa.orjp/codon/, and these tables can be adjusted in different ways. See, Nakamura Y. et. al "Codon usage tabulated from the international DNA sequence databases: status for the year2000 Nucl.Acids Res, 28: 292 (2000).

As used herein, a "vector" is a replicable nucleic acid from which one or more heterologous proteins can be expressed when the vector is transformed into an appropriate host cell. Reference to a vector includes those vectors into which a nucleic acid encoding a polypeptide or fragment thereof can be introduced, typically by restriction digest and ligation. Reference to a vector also includes those vectors that contain nucleic acid encoding a polypeptide. The vector is used to introduce the nucleic acid encoding the polypeptide into the host cell for amplification of the nucleic acid or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well known to those of skill in the art.

As used herein, a vector also includes "virus vectors" or "viral vectors." Viral vectors are engineered viruses that are operatively linked to exogenous genes to transfer (as vehicles or shuttles) the exogenous genes into cells.

As used herein, an "expression vector" includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, "treating" a subject with a disease or condition means that the subject's symptoms are partially or totally alleviated, or remain static following treatment. Hence treatment encompasses prophylaxis, therapy and/or cure. Prophylaxis refers to prevention of a potential disease and/or a prevention of worsening of symptoms or progression of a disease. Treatment also encompasses any pharmaceutical use of any antibody or antigen-binding fragment thereof provided or compositions provided herein.

As used herein, a "therapeutic effect" means an effect resulting from treatment of a subject that alters, typically improves or ameliorates the symptoms of a disease or condition or that cures a disease or condition.

As used herein, a "therapeutically effective amount" or a "therapeutically effective dose" refers to the quantity of an agent, compound, material, or composition containing a compound that is at least sufficient to produce a therapeutic effect following administration to a subject. Hence, it is the quantity necessary for preventing, curing, ameliorating, arresting or partially arresting a symptom of a disease or disorder.

As used herein, a "prophylactically effective amount" or a "prophylactically effective dose" refers to the quantity of an agent, compound, material, or composition containing a compound that when administered to a subject, will have the intended prophylactic effect, e.g., preventing or delaying the onset, or reoccurrence, of disease or symptoms, reducing the likelihood of the onset, or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, the term "subject" refers to a mammal, such as a human.

## Polypeptide comprising antigenic epitope in cTAGE5 extracellular region and use thereof

In one aspect, the present invention provides an isolated immunogenic polypeptide comprising an amino acid sequence of SEQ ID NO: 1 or SEQ ID NO: 2, or comprising an amino acid sequence having at least 80%, at least 90%, at least 95% sequence identity to SEQ ID NO: 1 or SEQ ID NO: 2, or comprising an amino acid sequence having one or more (e.g., 1, 2, 3, 4, or 5) amino acid substitutions, deletions or insertions relative to SEQ ID NO: 1 or SEQ ID NO: 2. In some embodiments, the isolated polypeptide may also comprise a tag. The tag may be a tag used for separating and/or purifying the polypeptide, such as a His tag or a GST tag. The tag can also be a moiety for enhancing the immunogenicity of the polypeptide.

In another aspect, the invention provides use of the isolated immunogenic polypeptide of the invention in generating an antibody. In some embodiments, the antibody is capable of specifically binding to the extracellular region of cTAGE5. In some embodiments, the extracellular region of cTAGE5 comprises an amino acid sequence shown in SEQ ID NO: 1, or comprises an amino acid sequence having at least 80%, at least 90%, at least 95% sequence identity with SEQ ID NO: 1, or comprises an amino acid sequence having one or more (e.g., 1, 2, 3, 4, or 5) amino acid substitutions, deletions or additions relative to SEQ ID NO: 1. In some embodiments, the extracellular region of cTAGE5 comprises an amino acid sequence shown in SEQ ID NO: 2, or comprises an amino acid sequence having at least 80%, at least 90%, or at least 95% sequence identity with SEQ ID NO: 2, or comprises an amino acid sequence having one or more (e.g., 1, 2, 3, 4, or 5) amino acid substitutions, deletions, or additions relative to SEQ ID NO: 2.

An exemplary cTAGE5 protein comprises an amino acid sequence of SEQ ID NO: 415 or SEQ ID NO: 416.

In another aspect, the present invention provides a method of generating an antibody, comprising immunizing an animal with the isolated immunogenic polypeptide according to the present invention, and then isolating an antibody specific for the polypeptide from the animal or obtaining sequence information of an antibody specific for the polypeptide. In some embodiments, the antibody is able to specifically bind to the extracellular region of the cTAGE5 protein. In some embodiments, the extracellular region of cTAGE5 comprises an amino acid sequence as shown in SEQ ID NO: 1, or comprises an amino acid sequence having at least 80%, at least 90%, or at least 95% sequence identity to SEQ ID NO: 1, or comprises an amino acid sequence having one or more (e.g., 1, 2, 3, 4, or 5) amino acid substitutions, deletions, or additions relative to SEQ ID NO: 1. In some embodiments, the extracellular region of cTAGE5 comprises an amino acid sequence as shown in SEQ ID NO: 2, or comprises an amino acid sequence having at least 80%, at least 90%, or at least 95% sequence identity to SEQ ID NO: 2, or comprises an amino acid sequence having one or more (e.g., 1, 2, 3, 4, or 5) amino acid substitutions, deletions, or additions relative to SEQ ID NO: 2. In some embodiments, the animal can be any animal used in the art for producing an antibody by immunization, including but not limited to, mice, rats, rabbits, goats, llamas, etc.

In another aspect, the invention provides a pharmaceutical composition comprising the isolated immunogenic polypeptide of the invention and a pharmaceutically acceptable carrier. In some embodiments, the pharmaceutical composition is used for the treatment and/or prevention of a tumor. In some embodiments, the pharmaceutical composition is a vaccine. In some embodiments, the vaccine further comprises an adjuvant.

In another aspect, the present invention provides use of the isolated immunogenic polypeptide of the present invention in the preparation of a medicament. In some embodiments, the medicament is used to treat and/or prevent a tumor. In some embodiments, the medicament is a vaccine. In some embodiments, the vaccine further comprises an adjuvant.

In another aspect, the invention provides a method of preventing and/or treating a tumor in a subject in need thereof, comprising administering to the subject an effective amount of the isolated immunogenic polypeptide of the invention or the pharmaceutical composition of the invention.

In another aspect, the present invention provides a method for detecting the presence and/or amount of an antibody that specifically binds to cTAGE5 in a biological sample, the method comprising: 1) contacting the isolated immunogenic polypeptide of the invention with the biological sample; and 2) detecting the presence and/or amount of an antibody bound to the isolated immunogenic polypeptide. In some embodiments, the biological sample is a biological sample from a subject. In some embodiments, the subject is a subject suspected of having a tumor. In some embodiments, the biological sample is a tissue sample, a blood sample, or a lymph sample. In some embodiments, the biological sample is a blood sample, such as whole blood, plasma, or serum. In some embodiments, the antibody specifically binds to the extracellular region of cTAGE5 protein. In some embodiments, the cTAGE5 extracellular region comprises an amino acid sequence as shown in SEQ ID NO: 1, or comprises an amino acid sequence having at least 80%, at least 90%, or at least 95% sequence identity with SEQ ID NO: 1, or comprises an amino acid sequence having one or more (such as 1, 2, 3, 4 or 5) amino acid substitutions, deletions or additions relative to SEQ ID NO: 1. In some embodiments, the extracellular domain of cTAGE5 comprises the amino acid sequence shown in SEQ ID NO:2, or comprises an amino acid sequence having at least 80%, at least 90%, at least 95% sequence identity with SEQ ID NO:2, or comprises an amino acid sequence having one or more (such as 1, 2, 3, 4 or 5) amino acid substitutions, deletions or additions relative to SEQ ID NO:2.

In another aspect, the invention provides the use of the isolated immunogenic polypeptide of the invention in the preparation of a kit for use in a method for detection of the presence and/or amount of an antibody specific for cTAGE5 protein in a biological sample. In some embodiments, the biological sample is a biological sample from an individual. In some embodiments, the subject is a subject suspected of having a tumor. In some embodiments, the biological sample is a tissue sample, a blood sample, or a lymph sample. In some embodiments, the biological sample is a blood sample, e.g., whole blood, plasma, or serum. In some embodiments, the antibody specifically binds to the extracellular region of cTAGE5. In some embodiments, the extracellular region of cTAGE5 comprises an amino acid sequence shown in SEQ ID NO:1, or comprises an amino acid sequence having at least 80%, at least 90%, at least 95% sequence identity to SEQ ID NO:1, or comprises an amino acid sequence having one or more (such as 1, 2, 3, 4, or 5) amino acid substitutions, deletions or additions relative to SEQ ID NO:1. In some embodiments, the extracellular region of cTAGE5 comprises an amino acid sequence as shown in SEQ ID NO: 2, or comprises an amino acid sequence having at least 80%, at least 90%, or at least 95% sequence identity with SEQ ID NO: 2, or comprises an amino acid sequence having one or more (e.g., 1, 2, 3, 4, or 5) amino acid substitutions, deletions, or additions relative to SEQ ID NO: 2.

In another aspect, the present invention provides the use of the cTAGE5 protein comprising an amino acid sequence as shown in SEQ ID NO: 1 or 2, or comprising an amino acid sequence having at least 80%, at least 90%, or at least 95% sequence identity to SEQ ID NO: 1, or comprising an amino acid sequence having one or more (e.g., 1, 2, 3, 4, or 5) amino acid substitutions, deletions, or insertions relative to SEQ ID NO: 1, preferably comprising an amino acid sequence as shown in SEQ ID NO: 2, as a tumor marker.

In another aspect, the present invention provides a tumor screening method, comprising detecting the presence or amount of a cTAGE5 protein in a biological sample from a subject, wherein the cTAGE5 protein comprises an amino acid sequence shown in SEQ ID NO: 1 or 2, or comprises an amino acid sequence having at least 80%, at least 90%, at least 95% sequence identity with SEQ ID NO: 1, or comprises an amino acid sequence having one or more (such as 1, 2, 3, 4, or 5) amino acid substitutions, deletions, or insertions relative to SEQ ID NO: 1, preferably comprising an amino acid sequence as shown in SEQ ID NO: 2.

In another aspect, the present invention provides a tumor screening method, the method comprising detecting the presence and/or amount of an antibody that specifically binds to the cTAGE5 protein in a biological sample from a subject, wherein the antibody specifically binds to the extracellular region of the cTAGE5 protein, and the extracellular region of the cTAGE5 protein comprises an amino acid sequence shown in SEQ ID NO: 1 or 2, or comprises an amino acid sequence having at least 80%, at least 90%, at least 95% sequence identity to SEQ ID NO: 1, or comprises an amino acid sequence having one or more (such as 1, 2, 3, 4, or 5) amino acid substitutions, deletions or additions relative to SEQ ID NO: 1, preferably comprising an amino acid sequence as shown in SEQ ID NO: 2.

In some embodiments of various aspects of the present invention, the tumor is a cTAGE5 protein-associated tumor, such as a tumor overexpressing cTAGE5 protein. In some embodiments, the cTAGE5 protein has an extracellular region. In some embodiments, the cTAGE5 extracellular region comprises an amino acid sequence shown in SEQ ID NO: 1, or comprises an amino acid sequence having at least 80%, at least 90%, at least 95% sequence identity to SEQ ID NO: 1, or comprises an amino acid sequence having one or more (such as 1, 2, 3, 4 or 5) amino acid substitutions, deletions or additions relative to SEQ ID NO: 1. In some embodiments, the extracellular region of cTAGE5 comprises an amino acid sequence shown in SEQ ID NO: 2, or comprises an amino acid sequence having at least 80%, at least 90%, at least 95% sequence identity with SEQ ID NO: 2, or comprises an amino acid sequence having one or more (such as 1, 2, 3, 4 or 5) amino acid substitutions, deletions or additions relative to SEQ ID NO: 2. Examples of the tumor include, but are not limited to, nasopharyngeal cancer, malignant melanoma, lung cancer, glioma, skin cancer, glossopharyngeal cancer, lymphoma (including B-cell lymphoma or T-cell lymphoma), liver cancer, cervical cancer, endometrial cancer, thyroid cancer, colon cancer, ovarian cancer, brain tumor, prostate cancer, breast cancer (such as triple-negative breast cancer), esophageal cancer, gastric cancer, pancreatic cancer, rectal cancer, colorectal cancer. In some embodiments, the tumor is an adenocarcinoma. In some embodiments, the tumor is squamous cell carcinoma. In some embodiments (e.g., where the cTAGE5 extracellular region comprises the amino acid sequence shown in SEQ ID NO:1), the tumor is selected from nasopharyngeal cancer, malignant melanoma, lung squamous cell carcinoma, cervical cancer, skin cancer, glossopharyngeal cancer, esophageal squamous cell carcinoma. In some embodiments (e.g., where thecTAGE5 extracellular region comprises the amino acid sequence shown in SEQ ID NO: 2), the tumor is selected from B-cell lymphoma, T-cell lymphoma, malignant melanoma, lung squamous carcinoma, lung adenocarcinoma, liver cancer, cervical squamous carcinoma, thyroid carcinoma, colon adenocarcinoma, ovarian cancer, brain tumor, skin squamous carcinoma, prostate cancer, breast cancer (e.g., triple negative breast cancer), esophageal squamous carcinoma, esophageal adenocarcinoma, gastric cancer, pancreatic cancer, rectal cancer, endometrial cancer, and colorectal cancer.

## Antibodies against the extracellular region of cTAGES

In one aspect, the present invention provides an antibody or an antigen binding fragment thereof that specifically binds to the extracellular region of cTAGE5. In some embodiments, the cTAGE5 extracellular region comprises an amino acid sequence as shown in SEQ ID NO: 1, or comprises an amino acid sequence having at least 80%, at least 90%, at least 95% sequence identity with SEQ ID NO: 1, or comprises an amino acid sequence having one or more (such as 1, 2, 3, 4, or 5) amino acid substitutions, deletions, or insertions relative to SEQ ID NO: 1. In some embodiments, the extracellular region of cTAGE5 comprises an amino acid sequence shown in SEQ ID NO:2, or comprises an amino acid sequence having at least 80%, at least 90%, at least 95% sequence identity to SEQ ID NO:2, or comprises an amino acid sequence having one or more (such as 1, 2, 3, 4 or 5) amino acid substitutions, deletions or additions relative to SEQ ID NO:2. In some embodiments, the antibody is a polyclonal antibody. In some preferred embodiments, the antibody is a monoclonal antibody.

In one aspect, the present invention provides an antibody or an antigen-binding fragment thereof that specifically binds to an immunogenic polypeptide, wherein the immunogenic polypeptide comprises an amino acid sequence of SEQ ID NO: 1 or SEQ ID NO: 2, or comprises an amino acid sequence having at least 80%, at least 90%, at least 95% sequence identity to SEQ ID NO: 1 or SEQ ID NO: 2, or comprises an amino acid sequence having one or more (e.g., 1, 2, 3, 4, or 5) amino acid substitutions, deletions or insertions relative to SEQ ID NO: 1 or SEQ ID NO: 2. In some embodiments, the antibody is a polyclonal antibody. In some preferred embodiments, the antibody is a monoclonal antibody.

In one aspect, the present invention provides an isolated monoclonal antibody or an antigen-binding fragment thereof, wherein the monoclonal antibody comprises the heavy chain CDRs and light chain CDRs, and/or the heavy chain variable regions and light chain variable regions, of any one of the antibodies listed in Table 2 and Table 6.

In one aspect, the present invention provides an isolated monoclonal antibody or an antigen-binding fragment thereof, wherein the monoclonal antibody comprises a heavy chain variable region and a light chain variable region, and comprises the heavy chain CDRs and light chain CDRs selected from any group of the following:

| Group | Description | SEQ ID NO |
|-------|-------------|-----------|
| 1 | HCDR1 | 7 |
| | HCDR2 | 8 |
| | HCDR3 | 9 |
| | LCDR1 | 10 |
| | LCDR2 | 11 |
| | LCDR3 | 12 |
| 2 | HCDR1 | 17 |
| | HCDR2 | 18 |
| | HCDR3 | 19 |
| | LCDR1 | 20 |
| | LCDR2 | 21 |
| | LCDR3 | 22 |
| 3 | HCDR1 | 33 |
| | HCDR2 | 34 |
| | HCDR3 | 35 |
| | LCDR1 | 36 |
| | LCDR2 | 37 |
| | LCDR3 | 38 |
| 4 | HCDR1 | 41 |
| | HCDR2 | 42 |
| | HCDR3 | 43 |
| | LCDR1 | 44 |
| | LCDR2 | 45 |
| | LCDR3 | 46 |
| 5 | HCDR1 | 49 |
| | HCDR2 | 50 |
| | HCDR3 | 51 |
| | LCDR1 | 52 |
| | LCDR2 | 53 |
| | LCDR3 | 54 |
| 6 | HCDR1 | 57 |
| | HCDR2 | 58 |
| | HCDR3 | 59 |
| | LCDR1 | 60 |
| | LCDR2 | 61 |
| | LCDR3 | 62 |

(continued)

| Group | Description | SEQ ID NO |
|---|---|---|
| 7 | HCDR1 | 65 |
| | HCDR2 | 66 |
| | HCDR3 | 67 |
| | LCDR1 | 68 |
| | LCDR2 | 69 |
| | LCDR3 | 70 |
| 8 | HCDR1 | 73 |
| | HCDR2 | 74 |
| | HCDR3 | 75 |
| | LCDR1 | 76 |
| | LCDR2 | 77 |
| | LCDR3 | 78 |
| 9 | HCDR1 | 81 |
| | HCDR2 | 82 |
| | HCDR3 | 83 |
| | LCDR1 | 84 |
| | LCDR2 | 85 |
| | LCDR3 | 86 |
| 10 | HCDR1 | 89 |
| | HCDR2 | 90 |
| | HCDR3 | 91 |
| | LCDR1 | 92 |
| | LCDR2 | 93 |
| | LCDR3 | 94 |
| 11 | HCDR1 | 97 |
| | HCDR2 | 98 |
| | HCDR3 | 99 |
| | LCDR1 | 100 |
| | LCDR2 | 101 |
| | LCDR3 | 102 |
| 12 | HCDR1 | 105 |
| | HCDR2 | 106 |
| | HCDR3 | 107 |
| | LCDR1 | 108 |
| | LCDR2 | 109 |
| | LCDR3 | 110 |

(continued)

| Group | Description | SEQ ID NO |
|---|---|---|
| 13 | HCDR1 | 113 |
| | HCDR2 | 114 |
| | HCDR3 | 115 |
| | LCDR1 | 116 |
| | LCDR2 | 117 |
| | LCDR3 | 118 |
| 14 | HCDR1 | 121 |
| | HCDR2 | 122 |
| | HCDR3 | 123 |
| | LCDR1 | 124 |
| | LCDR2 | 125 |
| | LCDR3 | 126 |
| 15 | HCDR1 | 129 |
| | HCDR2 | 130 |
| | HCDR3 | 131 |
| | LCDR1 | 132 |
| | LCDR2 | 133 |
| | LCDR3 | 134 |
| 16 | HCDR1 | 137 |
| | HCDR2 | 138 |
| | HCDR3 | 139 |
| | LCDR1 | 140 |
| | LCDR2 | 141 |
| | LCDR3 | 142 |
| 17 | HCDR1 | 145 |
| | HCDR2 | 146 |
| | HCDR3 | 147 |
| | LCDR1 | 148 |
| | LCDR2 | 149 |
| | LCDR3 | 150 |
| 18 | HCDR1 | 153 |
| | HCDR2 | 154 |
| | HCDR3 | 155 |
| | LCDR1 | 156 |
| | LCDR2 | 157 |
| | LCDR3 | 158 |

(continued)

| Group | Description | SEQ ID NO |
|---|---|---|
| 19 | HCDR1 | 161 |
| | HCDR2 | 162 |
| | HCDR3 | 163 |
| | LCDR1 | 164 |
| | LCDR2 | 165 |
| | LCDR3 | 166 |
| 20 | HCDR1 | 169 |
| | HCDR2 | 170 |
| | HCDR3 | 171 |
| | LCDR1 | 172 |
| | LCDR2 | 173 |
| | LCDR3 | 174 |
| 21 | HCDR1 | 177 |
| | HCDR2 | 178 |
| | HCDR3 | 179 |
| | LCDR1 | 180 |
| | LCDR2 | 181 |
| | LCDR3 | 182 |
| 22 | HCDR1 | 185 |
| | HCDR2 | 186 |
| | HCDR3 | 187 |
| | LCDR1 | 188 |
| | LCDR2 | 189 |
| | LCDR3 | 190 |
| 23 | HCDR1 | 193 |
| | HCDR2 | 194 |
| | HCDR3 | 195 |
| | LCDR1 | 196 |
| | LCDR2 | 197 |
| | LCDR3 | 198 |
| 24 | HCDR1 | 201 |
| | HCDR2 | 202 |
| | HCDR3 | 203 |
| | LCDR1 | 204 |
| | LCDR2 | 205 |
| | LCDR3 | 206 |

(continued)

| Group | Description | SEQ ID NO |
|---|---|---|
| 25 | HCDR1 | 209 |
| | HCDR2 | 210 |
| | HCDR3 | 211 |
| | LCDR1 | 212 |
| | LCDR2 | 213 |
| | LCDR3 | 214 |
| 26 | HCDR1 | 217 |
| | HCDR2 | 218 |
| | HCDR3 | 219 |
| | LCDR1 | 220 |
| | LCDR2 | 221 |
| | LCDR3 | 222 |
| 27 | HCDR1 | 225 |
| | HCDR2 | 226 |
| | HCDR3 | 227 |
| | LCDR1 | 228 |
| | LCDR2 | 229 |
| | LCDR3 | 230 |
| 28 | HCDR1 | 233 |
| | HCDR2 | 234 |
| | HCDR3 | 235 |
| | LCDR1 | 236 |
| | LCDR2 | 237 |
| | LCDR3 | 238 |
| 29 | HCDR1 | 241 |
| | HCDR2 | 242 |
| | HCDR3 | 243 |
| | LCDR1 | 244 |
| | LCDR2 | 245 |
| | LCDR3 | 246 |
| 30 | HCDR1 | 249 |
| | HCDR2 | 250 |
| | HCDR3 | 251 |
| | LCDR1 | 252 |
| | LCDR2 | 253 |
| | LCDR3 | 254 |

(continued)

| Group | Description | SEQ ID NO |
|---|---|---|
| 31 | HCDR1 | 257 |
| | HCDR2 | 258 |
| | HCDR3 | 259 |
| | LCDR1 | 260 |
| | LCDR2 | 261 |
| | LCDR3 | 262 |
| 32 | HCDR1 | 265 |
| | HCDR2 | 266 |
| | HCDR3 | 267 |
| | LCDR1 | 268 |
| | LCDR2 | 269 |
| | LCDR3 | 270 |
| 33 | HCDR1 | 273 |
| | HCDR2 | 274 |
| | HCDR3 | 275 |
| | LCDR1 | 276 |
| | LCDR2 | 277 |
| | LCDR3 | 278 |
| 34 | HCDR1 | 281 |
| | HCDR2 | 282 |
| | HCDR3 | 283 |
| | LCDR1 | 284 |
| | LCDR2 | 285 |
| | LCDR3 | 286 |
| 35 | HCDR1 | 289 |
| | HCDR2 | 290 |
| | HCDR3 | 291 |
| | LCDR1 | 292 |
| | LCDR2 | 293 |
| | LCDR3 | 294 |
| 36 | HCDR1 | 297 |
| | HCDR2 | 298 |
| | HCDR3 | 299 |
| | LCDR1 | 300 |
| | LCDR2 | 301 |
| | LCDR3 | 302 |

(continued)

| Group | Description | SEQ ID NO |
|---|---|---|
| 37 | HCDR1 | 305 |
| | HCDR2 | 306 |
| | HCDR3 | 307 |
| | LCDR1 | 308 |
| | LCDR2 | 309 |
| | LCDR3 | 310 |
| 38 | HCDR1 | 313 |
| | HCDR2 | 314 |
| | HCDR3 | 315 |
| | LCDR1 | 316 |
| | LCDR2 | 317 |
| | LCDR3 | 318 |
| 39 | HCDR1 | 321 |
| | HCDR2 | 322 |
| | HCDR3 | 323 |
| | LCDR1 | 324 |
| | LCDR2 | 325 |
| | LCDR3 | 326 |
| 40 | HCDR1 | 329 |
| | HCDR2 | 330 |
| | HCDR3 | 331 |
| | LCDR1 | 332 |
| | LCDR2 | 333 |
| | LCDR3 | 334 |
| 41 | HCDR1 | 337 |
| | HCDR2 | 338 |
| | HCDR3 | 339 |
| | LCDR1 | 340 |
| | LCDR2 | 341 |
| | LCDR3 | 342 |
| 42 | HCDR1 | 345 |
| | HCDR2 | 346 |
| | HCDR3 | 347 |
| | LCDR1 | 348 |
| | LCDR2 | 349 |
| | LCDR3 | 350 |

(continued)

| Group | Description | SEQ ID NO |
|-------|-------------|-----------|
| 43 | HCDR1 | 353 |
| | HCDR2 | 354 |
| | HCDR3 | 355 |
| | LCDR1 | 356 |
| | LCDR2 | 357 |
| | LCDR3 | 358 |
| 44 | HCDR1 | 361 |
| | HCDR2 | 362 |
| | HCDR3 | 363 |
| | LCDR1 | 364 |
| | LCDR2 | 365 |
| | LCDR3 | 366 |
| 45 | HCDR1 | 369 |
| | HCDR2 | 370 |
| | HCDR3 | 371 |
| | LCDR1 | 372 |
| | LCDR2 | 373 |
| | LCDR3 | 374 |
| 46 | HCDR1 | 377 |
| | HCDR2 | 378 |
| | HCDR3 | 379 |
| | LCDR1 | 380 |
| | LCDR2 | 381 |
| | LCDR3 | 382 |
| 47 | HCDR1 | 385 |
| | HCDR2 | 386 |
| | HCDR3 | 387 |
| | LCDR1 | 388 |
| | LCDR2 | 389 |
| | LCDR3 | 390 |
| 48 | HCDR1 | 393 |
| | HCDR2 | 394 |
| | HCDR3 | 395 |
| | LCDR1 | 396 |
| | LCDR2 | 397 |
| | LCDR3 | 398 |

(continued)

| Group | Description | SEQ ID NO |
|---|---|---|
| 49 | HCDR1 | 401 |
| | HCDR2 | 402 |
| | HCDR3 | 403 |
| | LCDR1 | 404 |
| | LCDR2 | 405 |
| | LCDR3 | 406 |
| 50 | HCDR1 | 409 |
| | HCDR2 | 410 |
| | HCDR3 | 411 |
| | LCDR1 | 412 |
| | LCDR2 | 413 |
| | LCDR3 | 414 |

In some embodiments, the monoclonal antibody comprises a light chain variable region and a heavy chain variable region, the heavy chain variable region (VH) and the light chain variable region (VL) respectively comprise an amino acid sequence selected from any one of the following groups, or each comprise an amino acid sequence having at least 70%, at least 75%, at least 80%, at least 85%, at least 90%, at least 95% or more sequence identity to an amino acid sequence selected from any one of the following groups:

| Group | Description | SEQ ID NO |
|---|---|---|
| 1 | $V_H$ | 5 |
| | VL | 6 |
| 2 | VH | 15 |
| | VL | 16 |
| 3 | VH | 31 |
| | VL | 32 |
| 4 | VH | 39 |
| | VL | 40 |
| 5 | VH | 47 |
| | VL | 48 |
| 6 | VH | 55 |
| | VL | 56 |
| 7 | VH | 63 |
| | VL | 64 |
| 8 | VH | 71 |
| | VL | 72 |
| 9 | VH | 79 |
| | VL | 80 |
| 10 | VH | 87 |
| | VL | 88 |

(continued)

| Group | Description | SEQ ID NO |
|---|---|---|
| 11 | VH | 95 |
| | VL | 96 |
| 12 | VH | 103 |
| | VL | 104 |
| 13 | VH | 111 |
| | VL | 112 |
| 14 | VH | 119 |
| | VL | 120 |
| 15 | VH | 127 |
| | VL | 128 |
| 16 | VH | 135 |
| | VL | 136 |
| 17 | VH | 143 |
| | VL | 144 |
| 18 | VH | 151 |
| | VL | 152 |
| 19 | VH | 159 |
| | VL | 160 |
| 20 | VH | 167 |
| | VL | 168 |
| 21 | VH | 175 |
| | VL | 176 |
| 22 | VH | 183 |
| | VL | 184 |
| 23 | VH | 191 |
| | VL | 192 |
| 24 | VH | 199 |
| | VL | 200 |
| 25 | VH | 207 |
| | VL | 208 |
| 26 | VH | 215 |
| | VL | 216 |
| 27 | VH | 223 |
| | VL | 224 |
| 28 | VH | 231 |
| | VL | 232 |
| 29 | VH | 239 |
| | VL | 240 |

(continued)

| Group | Description | SEQ ID NO |
|---|---|---|
| 30 | VH | 247 |
| | VL | 248 |
| 31 | VH | 255 |
| | VL | 256 |
| 32 | VH | 263 |
| | VL | 264 |
| 33 | VH | 271 |
| | VL | 272 |
| 34 | VH | 279 |
| | VL | 280 |
| 35 | VH | 287 |
| | VL | 288 |
| 36 | VH | 295 |
| | VL | 296 |
| 37 | VH | 303 |
| | VL | 304 |
| 38 | VH | 311 |
| | VL | 312 |
| 39 | VH | 319 |
| | VL | 320 |
| 40 | VH | 327 |
| | VL | 328 |
| 41 | VH | 335 |
| | VL | 336 |
| 42 | VH | 343 |
| | VL | 344 |
| 43 | VH | 351 |
| | VL | 352 |
| 44 | VH | 359 |
| | VL | 360 |
| 45 | VH | 367 |
| | VL | 368 |
| 46 | VH | 375 |
| | VL | 376 |
| 47 | VH | 383 |
| | VL | 384 |
| 48 | VH | 391 |
| | VL | 392 |

(continued)

| Group | Description | SEQ ID NO |
|---|---|---|
| 49 | VH | 399 |
| | VL | 400 |
| 50 | VH | 407 |
| | VL | 408 |

In some embodiments, the monoclonal antibody is a humanized antibody. In some embodiments, the heavy chain variable region of the humanized antibody comprises an amino acid sequence as shown in SEQ ID NO: 23, and the light chain variable region of the humanized antibody comprises an amino acid sequence as shown in SEQ ID NO: 24. In some embodiments, the heavy chain variable region of the humanized antibody comprises the amino acid sequence of SEQ ID NO: 25, and the light chain variable region of the humanized antibody comprises the amino acid sequence of SEQ ID NO: 26. In some embodiments, the heavy chain variable region of the humanized antibody comprises the amino acid sequence shown in SEQ ID NO:27 and a light chain variable region of the humanized antibody comprises the amino acid sequence shown in SEQ ID NO:28. In some embodiments, the heavy chain variable region of the humanized antibody comprises the amino acid sequence shown in SEQ ID NO: 29, and the light chain variable region of the humanized antibody comprises the amino acid sequence shown in SEQ ID NO: 30.

In some embodiments, the monoclonal antibody comprises a heavy chain constant region as shown in SEQ ID NO: 417. In some embodiments, the monoclonal antibody comprises a light chain constant region as shown in SEQ ID NO: 418. In some embodiments, the monoclonal antibody comprises a heavy chain constant region shown in SEQ ID NO: 417 and a light chain constant region shown in SEQ ID NO: 418.

In some embodiments, the monoclonal antibody comprises a heavy chain as shown in SEQ ID NO:3, and a light chain as shown in SEQ ID NO:4. In some embodiments, the monoclonal antibody comprises a heavy chain as shown by SEQ ID NO:13 and a light chain as shown by SEQ ID NO:14.

In some embodiments, the monoclonal antibody specifically binds to the extracellular region of the cTAGE5 protein. In some embodiments, the extracellular region of the cTAGE5 protein comprises the amino acid sequence shown in SEQ ID NO: 1. In some embodiments, the extracellular region of the cTAGE5 protein comprises the amino acid sequence of SEQ ID NO: 2.

In some embodiments, the monoclonal antibody specifically binds to an immunogenic polypeptide comprising the amino acid sequence of SEQ ID NO: 1 or 2.

In one aspect, the present invention further comprises a monoclonal antibody or an antigen binding fragment thereof that is capable of competing with the monoclonal antibody comprising the heavy chain of SEQ ID NO: 3 and the light chain of SEQ ID NO: 4 for binding to the extracellular region of cTAGE5 protein, or competing with the monoclonal antibody comprising the heavy chain of SEQ ID NO: 13 and the light chain of SEQ ID NO: 14 for binding to the extracellular region of cTAGE5 protein. In some embodiments, the extracellular region of the cTAGES protein comprises an amino acid sequence shown in SEQ ID NO: 1. In some embodiments, the extracellular region of the cTAGE5 protein comprises an amino acid sequence as shown in SEQ ID NO: 2.

In another aspect, the invention further includes a monoclonal antibody or an antigen binding fragment thereof which is capable of competing with the monoclonal antibody comprising the heavy chain of SEQ ID NO: 3 and the light chain of SEQ ID NO: 4 for binding to the extracellular region of cTAGE5 protein, or competing with the monoclonal antibody comprising the heavy chain of SEQ ID NO: 13 and the light chain of SEQ ID NO: 14 for binding to an immunogenic polypeptide comprising an amino acid sequence of SEQ ID NO: 1 or 2.

The antibody of the present invention, such as the monoclonal antibody or antigen binding fragment thereof, can inhibit tumor growth by at least about 10%, preferably at least about 20%, more preferably at least about 30%, more preferably at least about 40%, more preferably at least about 50%, more preferably at least about 60%, more preferably at least about 70%, more preferably at least about 80%.

The $K_D$ value of the antibody of the invention, such as a monoclonal antibody or an antigen-binding fragment thereof of the invention, for binding to the extracellular region of cTAGES protein (e.g., the extracellular region of the cTAGE5 protein comprising an amino acid sequence shown in SEQ ID NO:1 or 2) or the isolated immunogenic polypeptide of the invention (e.g., an immunogenic polypeptide comprising an amino acid sequence shown in SEQ ID NO:1 or 2) can be less than about $1\times10^{-7}$ M, preferably less than about $1\times10^{-8}$ M, more preferably less than about $1\times10^{-9}$ M, more preferably less than about $1\times10^{-10}$ M.

**Nucleic Acid, Vector, and Method for Producing Antibody**

In another aspect, the present invention provides an isolated nucleic acid molecule that encodes the antibody or antigen-binding fragment thereof of the present invention. In some embodiments, the nucleotide sequence of the nucleic acid molecule is codon-optimized for expression in a host cell. In some embodiments, the nucleic acid molecule is operably linked to an expression regulatory sequence.

The present invention also provides an expression vector comprising at least one of the above-described nucleic acid molecule of the invention.

The present invention also provides a host cell transformed with at least one of the above nucleic acid molecule or expression vector of the present invention.

In another aspect, the present invention provides a method of producing the antibody or antigen-binding fragment thereof of the present invention, comprising:

(i) culturing the host cell of the present invention under a condition suitable for expression of the nucleic acid molecule or the expression vector;
(ii) isolating and purifying the antibody or antigen-binding fragment expressed by the host cell.

The present invention also relates to an isolated antibody or antigen-binding fragment thereof obtained by the method described herein, which is capable of specifically binding to the extracellular region of cTAGE5 protein. In some embodiments, the cTAGE5 extracellular region comprises an amino acid sequence as shown in SEQ ID NO: 1, or comprises an amino acid sequence having at least 80%, at least 90%, at least 95% sequence identity with SEQ ID NO: 1, or comprises an amino acid sequence having one or more (such as 1, 2, 3, 4 or 5) amino acid substitutions, deletions or additions relative to SEQ ID NO: 1. In some embodiments, the extracellular region of cTAGE5 comprises the amino acid sequence of SEQ ID NO: 2, or comprises an amino acid sequence having at least 80%, at least 90%, at least 95% sequence identity with SEQ ID NO: 2, or comprises an amino acid sequence having one or more (such as 1, 2, 3, 4, or 5) amino acid substitutions, deletions, or additions relative to SEQ ID NO: 2.

**Antibody Conjugates**

The invention also provides an antibody conjugate comprising the antibody or antigen binding fragment of the invention and a therapeutic moiety conjugated to the antibody or antigen binding fragment. In some embodiments, the therapeutic portion comprises, for example, a cytotoxin, a radioactive isotope, or a biologically active protein.

A cytotoxin includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Therapeutic agents that can be conjugated also include, for example, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

Other preferred examples of therapeutic cytotoxins which can be conjugated to the antibody of the present invention include duocarmycins, calicheamicins, maytansines and auristatins, and derivatives thereof.

Cytotoxins can be conjugated to the antibody of the invention using linker technology available in the art. Examples of linker types that have been used to conjugate a cytotoxin to antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D).

The antibody of the invention may also be conjugated to a radioisotope to produce a cytotoxic radiopharmaceutical, also known as a radioactive antibody conjugate. Examples of radioactive isotopes that can be conjugated to antibodies for use diagnostically or therapeutically include, but are not limited to, iodine[131], indium[111], yttrium[90] and lutetium[177]. Methods for preparing a radioactive antibody conjugate have been established in the art.

The antibody of the invention may also be conjugated to a protein with desired biological activity to modify a given biological response. Such proteins may include, for example, an enzymatically active toxin, or active fragment thereof, such as abrin, ricin A, pseudomonas exotoxin, or diphtheria toxin; a protein such as tumor necrosis factor or interferon-γ; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other immune factors such as IFN..

## Disease Treatment and/or Prevention

Thus, the present invention provides a method of preventing and/or treating a tumor in a subject, comprising administering to the subject an effective amount of the antibody or antigen-binding fragment thereof of the present invention or the antibody conjugate of the present invention.

The tumor that can be treated and/or prevented by the method of the present invention is a cTAGE5 protein-associated tumor, such as a tumor overexpressing cTAGE5 protein. In some embodiments, the cTAGE5 protein has an extracellular region. In some embodiments, the cTAGE5 extracellular region comprises an amino acid sequence shown in SEQ ID NO: 1, or comprises an amino acid sequence having at least 80%, at least 90%, at least 95% sequence identity to SEQ ID NO:1, or comprises an amino acid sequence having one or more (e.g., 1, 2, 3, 4 or 5) amino acid substitutions, deletions or additions relative to SEQ ID NO:1. In some embodiments, the extracellular region of cTAGE5 comprises an amino acid sequence as shown in SEQ ID NO: 2, or comprises an amino acid sequence having at least 80%, at least 90%, at least 95% sequence identity to SEQ ID NO: 2, or comprises an amino acid sequence having one or more (e.g., 1, 2, 3, 4, or 5) amino acid substitutions, deletions or additions relative to SEQ ID NO: 2.

Examples of tumors that can be treated and/or prevented by the method of the present invention include, but are not limited to, nasopharyngeal cancer, malignant melanoma, lung cancer, glioma, skin cancer, glossopharyngeal cancer, lymphoma (including B-cell lymphoma or T-cell lymphoma), liver cancer, cervical cancer, endometrial cancer, thyroid cancer, colon cancer, ovarian cancer, brain tumor, prostate cancer, breast cancer (such as triple-negative breast cancer), esophageal cancer, gastric cancer, pancreatic cancer, rectal cancer, colorectal cancer. In some embodiments, the tumor is an adenocarcinoma. In some embodiments, the tumor is squamous cell carcinoma. In some embodiments (e.g., where the cTAGE5 extracellular region comprises the amino acid sequence shown in SEQ ID NO: 1), the tumor is selected from nasopharyngeal cancer, malignant melanoma, lung squamous cell carcinoma, cervical cancer, skin cancer, glossopharyngeal cancer, esophageal squamous cell carcinoma. In some embodiments (e.g., where thecTAGE5 extracellular region comprises the amino acid sequence shown in SEQ ID NO: 2), the tumor is selected from B-cell lymphoma, T-cell lymphoma, malignant melanoma, lung squamous carcinoma, lung adenocarcinoma, liver cancer, cervical squamous carcinoma, thyroid carcinoma, colon adenocarcinoma, ovarian cancer, brain tumor, skin squamous carcinoma, prostate cancer, breast cancer (e.g., triple negative breast cancer), esophageal squamous carcinoma, esophageal adenocarcinoma, gastric cancer, pancreatic cancer, rectal cancer, endometrial cancer, and colorectal cancer.

## Pharmaceutical composition

The present invention also provides a pharmaceutical composition comprising the antibody or antigen-binding fragment thereof of the present invention, or the antibody conjugate of the present invention, and a pharmaceutically acceptable carrier. The pharmaceutical composition is used for prevention and/or treatment of a tumor in a subject.

The term "pharmaceutically acceptable carrier" as used herein includes any and all physiologically compatible solvents, dispersing media, coatings, antibacterial agents, antifungal agents, isotonic agents, absorption delaying agents, etc. Preferably, the carrier is suitable for intravenous, intramuscular, subcutaneous, parenteral, spinal or epidermal administration (such as by injection or infusion). Depending on the route of administration, the active compound, that is, the antibody molecule or conjugate, may be encapsulated in a material to protect the compound from the action of acids and other natural conditions that may inactivate the compound.

The pharmaceutical composition of the present invention may also comprise a pharmaceutically acceptable antioxidant. Examples of pharmaceutically acceptable antioxidants include: (1) water-soluble antioxidants, such as ascorbic acid, cysteine hydrochloride, sodium bisulfate, sodium metabisulfite, sodium sulfite, etc.; (2) oil-soluble antioxidants, such as ascorbyl palmitate, butylated hydroxyanisole (BHA), butylated hydroxytoluene (BHT), lecithin, propyl gallate, $\alpha$-tocopherol, etc.; and (3) metal chelating agents, such as citric acid, ethylenediamine tetraacetic acid (EDTA), sorbitol, tartaric acid, phosphoric acid, etc.

These compositions may also contain auxiliary agents such as preservatives, wetting agents, emulsifying agents and dispersing agents.

Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, this amount will range from about 0.01% to about 99% of active ingredient, preferably from about 0.1% to about 70%, most preferably from about 1 %to about 30 %of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody molecule, the dosage ranges from about 0.0001 to 100 mg/kg, and more usually 0.01 to 20 mg/kg, of the subject body weight. For example dosages can be 0.3 mg/kg body weight, 1 mg/kg body weight, 3 mg/kg body weight, 5 mg/kg body weight, 10 mg/kg body weight or 20 mg/kg body weight or within the range of 1-20 mg/kg. An exemplary treatment regime entails administration once per week, once every 2 weeks, once every 3 weeks, once every 4 weeks, once a month, once every 3 months or once every 3 to 6 months, or with a short administration interval at the beginning (such as once per week to once every 3 weeks), and then an extended interval later (such as once a month to once every 3 to 6 months).

Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective amount" of the antibody or antigen-binding fragment thereof of the invention preferably results in a decrease in severity of disease symptoms, an increase in frequency and duration of disease symptom-free periods, or a prevention of impairment or disability due to the disease affliction. For example, for the treatment of tumors, a "therapeutically effective amount " preferably inhibits cell growth or tumor growth by at least about 10%, at least about 20%, more preferably by at least about 40%, even more preferably by at least about 60%, and still more preferably by at least about 80% relative to untreated subjects. The ability to inhibit tumor growth can be evaluated in an animal model system predictive of efficacy in human tumors. Alternatively, this property of a composition can be evaluated by examining the ability of the compound to inhibit cell growth; such inhibition can be determined in vitro by assays known to the skilled practitioner. A therapeutically effective amount of the antibody or antigen-binding fragment thereof can decrease tumor size, or otherwise ameliorate symptoms, such as preventing and/or treating metastasis or recurrence, in a subject. One of ordinary skill in the art would be able to determine such amounts based on such factors as the subject's size, the severity of the subject's symptoms, and the particular composition or route of administration selected.

The antibody or antigen-binding fragment thereof of the invention or the pharmaceutical composition of the invention can be administered via one or more routes of administration using one or more of a variety of methods known in the art. As will be appreciated by the skilled artisan, the route and/or mode of administration will vary depending upon the desired results. The antibody of the present invention is preferably administered by intravenous, intramuscular, intradermal, intraperitoneal, subcutaneous, spinal or other parenteral administration routes, such as injection or infusion, or bladder perfusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, the antibody or an antigen-binding fragment thereof of the invention, or the antibody conjugate or the pharmaceutical composition of the invention may also be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J.R. Robinson, ed., Marcel Dekker, Inc., New York, 1978.

In some embodiments, the antibody of the invention can be formulated to ensure proper distribution in vivo. For example, the blood-brain barrier (BBB) blocks many highly hydrophilic compounds. To ensure that the therapeutic compounds of the invention cross the BBB (if desired), they can be formulated, for example, in liposomes.

### Chimeric Antigen Receptor (CAR)

The antibody or antigen-binding fragment thereof can also be used to prepare a chimeric antigen receptor (CAR), which can be used for cell therapy (CAR-T or CAR-NK therapy) of related tumors after being introduced into T cells or NK cells. Thus, the present invention also encompasses a chimeric antigen receptor (CAR) comprising the antibody or antigen-binding fragment thereof of the present invention, a CAR-T cell or CAR-NK cell comprising the CAR, and the use of the CAR-T cell or CAR-NK cell in the treatment of a tumor.

### Combination Therapy

The polypeptide, the antibody or an antigen-binding fragment thereof, the antibody conjugate or the pharmaceutical composition of the invention may be administered in combination with a chemotherapeutic agent or an antibody targeting another tumor antigen.

The chemotherapy agents or the antibodies targeting other tumor antigens used in combination with the polypeptide, antibody or antigen binding fragment thereof, antibody conjugate or pharmaceutical composition of the present invention, are not particularly limited. Examples of the chemotherapy agents and antibodies that target other tumor antigens include, but are not limited to, ifosfamide, cyclophosphamide, dacarbazine, temozolomide, nimustine, busulfan, melphalan, enocitabine, capecitabine, carmofur, cladribine, gemcitabine, cytarabine, tegafur, tegafur-uracil, TS-1, doxifluridine, nelarabine, hydroxyurea, Fluorouracil, Fludarabine, pemetrexed, pentostatin, mercaptopurine, methotrexate, Irinotecan, etoposide, Eribulin, Sobuzoxane, docetaxel, paclitaxel, vinorelbine, Vincristine, Vindesine, Vinblastine, actinomycin D, Aclarubicin, amrubicin, idarubicin, epirubicin, Zinostatin, daunorubicin, doxorubicin, pirarubicin, bleomycin, peplomycin, mitomycin C, mitoxantrone, oxaliplatin, carboplatin, cisplatin, nedaplatin, anastrozole, exemestane, ethinyl estradiol, Chlormadinone, goserelin, tamoxifen, dexamethasone, bicalutamide, toremifene, flutamide, prednisolone, fosfestrol, Mitotane, methyltestosterone, Leuprorelin, letrozole, methylmedroxyprogesterone, ibritumomab tiuxetan, Imatinib, everolimus, Erlotinib, Gefitinib, Sunitinib, cetuximab, Sorafenib, Dasatinib, Tamibarotene, Trastuzumab, retinoic acid, Keytruda, bevacizumab, Bortezomib, and Lapatinib.

The polypeptide, antibody or antigen binding fragment thereof, antibody conjugate and the chemotherapeutic agent or antibody targeting other tumor antigens can be administered in one administration or administered separately. When administered separately (in respective different administration regimens), they can be administered sequentially without intervals or administered at predetermined intervals.

The combination dose of the polypeptide, antibody or antigen-binding fragment thereof, antibody conjugate and the chemotherapeutic agent or antibody targeting other tumor antigens in the pharmaceutical composition of the present invention is not particularly limited. As mentioned above, the dose of the antibody of the present invention can be determined by referring to the dose when the antibody is used alone. The chemotherapy agent and the antibody targeting other tumor antigens may be used at the dosage indicated for respective drug or may be reduced (considering the combined effect with the antibody of the present invention).

The polypeptide, antibody or antigen-binding fragment thereof, antibody conjugate or pharmaceutical composition of the present invention may also be combined with radiotherapy, for example, by administering ionizing radiation to a patient, which may be before, during and/or after the administration of the antibody or pharmaceutical composition of the present invention.

## Detection and Diagnosis

In another aspect, the present invention also provides a method for detecting the presence or expression level of cTAGE5 protein in a biological sample, comprising contacting the biological sample and optionally a control sample with the antibody of the present invention, such as a monoclonal antibody or antigen binding fragment thereof of the present invention, under a condition allowing the formation of a complex between the antibody of the present invention, such as a monoclonal antibody or antigen binding fragment thereof of the present invention, and the cTAGE5 protein. The formation of the complex is then detected, wherein the formation of the complex in the biological sample or the difference in the formation of the complex between the biological sample and the control sample indicates the presence of the cTAGE5 protein or the expression level of the cTAGE5 protein in the sample. In some embodiments, the cTAGE5 protein has an extracellular region. In some embodiments, the cTAGE5 extracellular region comprises an amino acid sequence as shown in SEQ ID NO: 1, or comprises an amino acid sequence having at least 80%, at least 90%, at least 95% sequence identity with SEQ ID NO: 1, or comprises an amino acid sequence having one or more (e.g., 1, 2, 3, 4 or 5) amino acid substitutions, deletions or insertions relative to SEQ ID NO: 1. In some embodiments, the extracellular region of cTAGE5 comprises an amino acid sequence shown in SEQ ID NO: 2, or comprises an amino acid sequence having at least 80%, at least 90%, at least 95% sequence identity to SEQ ID NO: 2, or comprises an amino acid sequence having one or more (e.g., 1, 2, 3, 4 or 5) amino acid substitutions, deletions or additions relative to SEQ ID NO: 2.

In some embodiments, the control biological sample is a biological sample from a healthy individual. In some embodiments, the control biological sample is a biological sample from an individual known not to have a tumor.

In some embodiments, the biological sample is an ex vivo sample. The biological sample includes, but is not limited to, a tissue sample, a blood sample, a lymph sample and the like.

In some embodiments, the biological sample is a tissue section. In some embodiments, the method is an immunohistochemistry assay. In some embodiments, the method is immunofluorescence assay.

Surprisingly, it is found that the cTAGE5 containing the amino acid sequence of SEQ ID NO:1 is highly expressed in malignant melanoma and squamous carcinoma, and the cTAGE5 containing the amino acid sequence of SEQ ID NO:2 is highly expressed in most common tumors. Therefore, the antibody of the present invention, such as a monoclonal antibody or an antigen binding fragment thereof, may be used for screening or diagnosis of cTAGE5-related tumors.

Thus, the present invention also provides a method for detecting the presence of a tumor (e.g., a tumor cell) in a patient, comprising detecting (e.g., by the method of the present invention) the presence or expression level of cTAGE5 in a biological sample from the patient, wherein the presence of cTAGE5 in the biological sample from the patient or the expression level of cTAGE5 in the biological sample from the patient higher than the expression level of cTAGE5 in a control biological sample, indicating the presence of a tumor (e.g., a tumor cell) in the patient. In some embodiments, the cTAGE5 protein has an extracellular region. In some embodiments, the cTAGE5 extracellular region comprises an amino acid sequence as shown in SEQ ID NO:1, or comprises an amino acid sequence having at least 80%, at least 90%, at least 95% sequence identity with SEQ ID NO:1, or comprises an amino acid sequence having one or more (such as 1, 2, 3, 4 or 5) amino acid substitutions, deletions or additions relative to SEQ ID NO:1. In some embodiments, the extracellular region of cTAGE5 comprises an amino acid sequence shown in SEQ ID NO: 2, or comprises an amino acid sequence having at least 80%, at least 90%, at least 95% sequence identity to SEQ ID NO: 2, or comprises an amino acid sequence having one or more (such as 1, 2, 3, 4 or 5) amino acid substitutions, deletions or additions relative to SEQ ID NO: 2. In some embodiments, the extracellular region of the cTAGE5 protein comprises the amino acid sequence shown in SEQ ID NO: 1. In some preferred embodiments, the extracellular region of the cTAGE5 protein comprises an amino acid sequence shown in SEQ ID NO: 2. In some embodiments, the method can also indirectly detect the presence or expression level of the cTAGE5 protein by detecting the presence and/or amount of the mRNA molecule encoding the cTAGE5 extracellular region (e.g., SEQ ID NO:1 or SEQ ID NO:2).

In some embodiments, the control biological sample is a biological sample from a healthy individual. In some embodiments, the control biological sample is a biological sample from an individual that is known not to have a tumor.

In some embodiments, the biological sample is an ex vivo sample. The biological sample includes but is not limited to a tissue sample, a blood sample, a lymph sample and the like.

In some embodiments, the biological sample is a tissue section. In some embodiments, the method is an immunohistochemistry assay. In some embodiments, the method is immunofluorescence assay.

In another aspect, the present invention provides a diagnostic agent for detecting and/or diagnosing a cTAGE5-related disease, such as a tumor, which comprises the antibody, such as the monoclonal antibody or an antigen-binding fragment thereof of the present invention, and optionally a physiologically acceptable carrier. In some embodiments, the diagnostic agent is a contrast agent.

In another aspect, the present invention provides the use of an antibody, such as a monoclonal antibody or an antigen binding fragment thereof of the present invention, in the preparation of a diagnostic agent for the detection and/or diagnosis of a cTAGE5 related disease, such as a tumor. In some embodiments, the diagnostic agent is a contrast agent.

In another aspect, the invention provides a method for detecting and/or diagnosing cTAGE5-related disease, such as a malignant tumor, in an subject, comprising administering to the subject an antibody, such as a monoclonal antibody or an antigen-binding fragment thereof of the invention, or a diagnostic agent of the invention.

In some embodiments of the above aspects of the invention, the antibody, monoclonal antibody or antigen binding fragment of the invention is conjugated to a fluorochrome, chemical substance, peptide, enzyme, isotope, label, or other reagents which is detectable *per se* or can be detected by other reagents.

In some embodiments of the aspects of the present invention, the tumor is a cTAGE5 protein-related tumor, such as a tumor overexpressing cTAGE5 protein. In some embodiments, the cTAGE5 protein has an extracellular region. In some embodiments, the extracellular domain of cTAGE5 comprises an amino acid sequence as shown in SEQ ID NO:1, or comprises an amino acid sequence having at least 80%, at least 90%, at least 95% sequence identity to SEQ ID NO:1, or comprises an amino acid sequence having one or more (e.g., 1, 2, 3, 4 or 5) amino acid substitutions, deletions or additions relative to SEQ ID NO:1. In some embodiments, the extracellular domain of cTAGE5 comprises an amino acid sequence as shown in SEQ ID NO:2, or comprises an amino acid sequence having at least 80%, at least 90%, at least 95% sequence identity to SEQ ID NO:2, or comprises an amino acid sequence having one or more (such as 1, 2, 3, 4, or 5) amino acid substitutions, deletions or additions relative to SEQ ID NO:2. Examples of the tumor include, but are not limited to, nasopharyngeal cancer, malignant melanoma, lung cancer, glioma, skin cancer, glossopharyngeal cancer, lymphoma (including B-cell lymphoma or T-cell lymphoma), liver cancer, cervical cancer, thyroid cancer, colon cancer, ovarian cancer, brain tumor, prostate cancer, breast cancer, esophageal cancer, gastric cancer, pancreatic cancer, and rectal cancer. In some embodiments, the tumor is an adenocarcinoma. In some embodiments, the tumor is squamous cell carcinoma. In some embodiments (e.g., where the cTAGE5 extracellular region contains amino acid sequence as shown in SEQ ID NO:1), the tumor is selected from nasopharyngeal carcinoma, malignant melanoma, lung squamous cell carcinoma, cervical cancer, skin cancer, glossopharyngeal cancer, esophageal squamous cell carcinoma. In some embodiments (e.g., where cTAGE5 extracellular region contains the amino acid sequence shown in SEQ ID NO: 2), the tumor is selected from B-cell lymphoma, T-cell lymphoma, malignant melanoma, lung squamous cell carcinoma, lung adenocarcinoma, liver cancer, cervical squamous cell carcinoma, thyroid cancer, colon adenocarcinoma, ovarian cancer, brain tumor, skin squamous cell carcinoma, prostate cancer, breast cancer, esophageal squamous cell carcinoma, esophageal adenocarcinoma, gastric cancer, pancreatic cancer, and rectal cancer.

## Kit

The invention also includes a kit for use in the method of the invention, comprising the polypeptide, antibody, e.g., monoclonal antibody or antigen binding fragment thereof, or antibody conjugate of the invention, as well as instructions for use. The kit may further include at least one additional detection reagent for detecting the presence of the polypeptide or monoclonal antibody of the present invention. The kit generally comprises a label indicating the intended use and/or use instructions for the contents of the kit. The term label includes any written or recorded material provided on the kit, with the kit, or in any other manner with the kit.

## Examples

Further understanding of the present invention can be obtained by reference to the specific examples given herein. These examples are only intended to illustrate the present invention but are not intended to limit the scope of the present invention in any way. It is obvious that various modifications and variations can be made to the present invention without departing from the essence of the present invention, and therefore, these modifications and variations are also within the scope of protection claimed in the present application.

**Method and Process of IHC Experiment**

1) the slide was baked at 60°C for 30 minutes, and then routinely dewaxed and hydrated;

2) antigen repair: antigens were repaired with 0.01M citric acid buffer (pH6.0) at high pressure for 2 min, cool to room temperature, washed with PBS for 5 min×3 times

3) endogenous peroxidase was blocked with 3% $H_2O_2$-methanol at room temperature for 10 minutes, and washed with PBS for 5 minutes×3 times;

4) normal non-immunized animal serum was added, room temperature for 10 minutes;

5) the serum was removed, diluted antibody (polyclonal antibody or ID6 or F11) solution was added dropwise, and stored overnight at 4°C in a refrigerator;

6) the cells were washed with 0.1% Tween-20 PBS for 5 minutes×3 times;

7) biotinylated goat anti-mouse/rabbit IgG was dropwise added, and incubated at room temperature for 10 minutes;

8) washed with 0.1% Tween-20 PBS for 5 min ×3 times;

9) streptavidin-peroxidase was added and incubated at room temperature for 10 min;

10) DAB for 5 minutes, and washed with distilled water to terminate coloration;

11) after re-staining with hematoxylin, washing with water, and decolorization, the tissue sections were washed with water until the blue color appeared again;

12) normal dehydration, neutral gum sealing and covering.

**Example 1: Identification of the Extracellular Region of cTAGE5**

cTAGE5 gene has more than twenty exons, and due to selective transcription, 28 cTAGE5 splicing variants are present in the database. 28 cTAGE5 splice variants were summarized in Table 1.

Table 1: Extracellular and intracellular structures of 28 splicing variants of cTAGE5. Eight variants had no transmembrane region. Another eight variants had a same short peptide outside the transmembrane region. The other 12 variants had the same short peptide but with a N-terminal extension outside the membrane region.

| cTAGE5 splicing variant types | Name of Splice Variant |
|---|---|
| No transmembrane region. (8) | cTAGE5-variant7, cTAGE5-variant13, cTAGE5-variant14, cTAGE5-variant15, cTAGE5-variant16, cTAGE5-variant17, cTAGE5-variant18, cTAGE5-variant29, |
| Short peptide outside the transmembrane region. (8) | cTAGE5-variant4, cTAGE5-variant5, cTAGE5-va riant10, cTAGE5-variant11, cTAGE5-variant12, cTAGE5-variant20, cTAGE5-variant21, cTAGE5-variant22 |
| Longer peptide outside the transmembrane Region. (12) | cTAGE5-variant1, cTAGE5-variant2, cTAGE5-variant3, cTAGE5-variant6, cTAGE5-variant19, cTAGE5-variant23, cTAGE5-variant24, cTAGE5-variant25, cTAGE5-variant26, cTAGE5-variant27, cTAGE5-variant28, cTAGE5-variant30 |

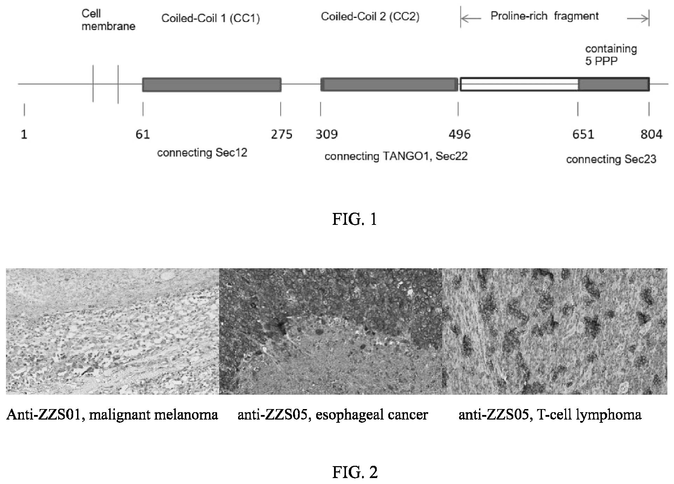

Previously, when studying the function of cTAGE5 for delivering proteins, the sequence of the Mea6 variant of cTAGE5, also known as Mgea6, was mostly used as a research object. It is one of 12 splice variants with a longer peptide outside the transmembrane region, and its structure is roughly shown in FIG. 1.

Previous studies on the function of cTAGE5 mainly focused on the intramembrane region of cTAGE5/Mea6. Studies have found that the cTAGE5/Mea6 of 804 amino acid residues, which forms a complex of TANGO1 on the membrane at the output end of ER (Lumen), is linked to framework proteins Sec12, Sec22, Sec23 of COPII to promote COPII to output collagen, but it does not leave the membrane together with COPII. Similar studies mostly focused on the behavior of cTAGE5/Mea6 within the cell , which is an indispensable component of the normal operation of human cells. The amino acid sequence of the outer (extracellular) part of the cTAGE5/MEA6 is considered as a signal peptide, and there is no report on the function of this part of sequence.

Three different N-terminal extension sequences are comprised in the 12 splicing variants with longer peptide outside of the transmembrane region: 9 variants have the N-terminal extension sequence: MEEPGTTQPYLGLLELEERRVAALPEG (SEQ ID NO: 1); the other two variants have the N-terminal long sequence: MELKSPEEEVVAALPEG(SEQ ID NO: 2); the extension sequence of the other variant is not immunogenic, which is not considered here.

Herein, the sequence MEEPGVTPQPYLGLLELEELRVVALPEG (SEQ ID NO: 1) is named ZZS01; the sequence MELKSPEEEVVAALPEG(SEQ ID NO: 2) is named ZZS05.

The amino acid sequence of an exemplary cTAGE5 protein comprising ZZS01(cTAGE5 variant 1; Protein_id =

28

"NP_005921.2") is as follows:

MEEPGVTPQPYLGLLLEELRRVVAALPEGMRPDSNLYGFPWELVICAAVVGFFAVLFFLW
RSFRSVRSRLYVGREKKLALMLSGLIEEKSKLLEKFSLVQKEYEGYEVESSLKDASFEKEATEA
QSLEATCEKLNRSNSELEDEILCLEKELKEEKSKHSEQDELMADISKRIQSLEDESKSLKSQVAE
AKMTFKIFQMNEERLKIAIKDALNENSQLQESQKQLLQEAEVWKEQVSELNKQKVTFEDSKV
HAEQVLNDKESHIKTLTERLLKMKDWAAMLGEDITDDDNLELEMNSESENGAYLDNPPKGA
LKKLIHAAKLNASLKTLEGERNQIYIQLSEVDKTKEELTEHIKNLQTEQASLQSENTHFENENQ
KLQQKLKVMTELYQENEMKLHRKLTVEENYRLEKEEKLSKVDEKISHATEELETYRKRAKDL
EEELERTIHSYQGQIISHEKKAHDNWLAARNAERNLNDLRKENAHNRQKLTETELKFELLEKD
PYALDVPNTAFGREHSPYGPSPLGWPSSETRAFLSPPTLLEGPLRLSPLLPGGGGRGSRGPGNP
LDHQITNERGESSCDRLTDPHRAPSDTGSLSPPWDQDRRMMFPPPGQSYPDSALPPQRQDRFC
SNSGRLSGPAELRSFNMPSLDKMDGSMPSEMESSRNDTKDDLGNLNVPDSSLPAENEATGPGF
VPPPLAPIRGPLFPVDARGPFLRRGPPFPPPPPGAMFGASRDYFPPGDFPGPPPAPFAMRNVYPP
RGFPPYLPPRPGFFPPPPHSEGRSEFPSGLIPPSNEPATEHPEPQQET (SEQ ID NO:415)

The amino acid sequence of an exemplary cTAGE5 protein comprising ZZS05(cTAGE5 variant 2; Protein_id = "NP_976229.1") is as follows:

MELKSPEEEVVAALPEGMRPDSNLYGFPWELVICAAVVGFFAVLFFLWRSFRSVRSRLYV
GREKKLALMLSGLIEEKSKLLEKFSLVQKEYEGYEVESSLKDASFEKEATEAQSLEATCEKLN
RSNSELEDEILCLEKELKEEKSKHSEQDELMADISKRIQSLEDESKSLKSQVAEAKMTFKIFQM
NEERLKIAIKDALNENSQLQESQKQLLQEAEVWKEQVSELNKQKVTFEDSKVHAEQVLNDKE
SHIKTLTERLLKMKDWAAMLGEDITDDDNLELEMNSESENGAYLDNPPKGALKKLIHAAKLN
ASLKTLEGERNQIYIQLSEVDKTKEELTEHIKNLQTEQASLQSENTHFENENQKLQQKLKVMT
ELYQENEMKLHRKLTVEENYRLEKEEKLSKVDEKISHATEELETYRKRAKDLEEELERTIHSY
QGQIISHEKKAHDNWLAARNAERNLNDLRKENAHNRQKLTETELKFELLEKDPYALDVPNTA

FGREHSPYGPSPLGWPSSETRAFLSPPTLLEGPLRLSPLLPGGGGRGSRGPGNPLDHQITNERGE
SSCDRLTDPHRAPSDTGSLSPPWDQDRRMMFPPPGQSYPDSALPPQRQDRFCSNSGRLSGPAE
LRSFNMPSLDKMDGSMPSEMESSRNDTKDDLGNLNVPDSSLPAENEATGPGFVPPPLAPIRGP
LFPVDARGPFLRRGPPFPPPPPGAMFGASRDYFPPGDFPGPPPAPFAMRNVYPPRGFPPYLPPRP
GFFPPPPHSEGRSEFPSGLIPPSNEPATEHPEPQQET  (SEQ ID NO:416)

Example 2: Polyclonal Antibody Preparation and Immunohistochemistry Experiment

The coding sequences of ZZS01 and ZZS05 were inserted into vector pGEX-6p-1, respectively, and the GST-ZZS01 and GST- ZZS05 fusion proteins were expressed and purified in BL21 E. coli, respectively. The rabbits were immunized with these two fusion proteins respectively, and rabbit serum against ZZS01 and ZZS05 was harvested.
The obtained rabbit serum was used to perform immunohistochemical experiments on tissue sections of 20 common tumors. The results showed that the polyclonal antibody of ZZS01 was sensitive to malignant melanoma and squamous cell carcinoma (insensitive to adenocarcinoma), and the polyclonal antibody of ZZZ05 was sensitive to all these tumors. The IHC results of representative rabbit polyclonal antibodies are shown in FIG. 2.

**Example 3: Mouse Monoclonal Antibody Preparation and Immunohistochemistry Experiment**

**3.1. Preparation of mouse monoclonal antibody**

The fusion proteins GST- ZZS01 and GST- ZZS05 were used to prepare murine monoclonal antibodies, respectively. ZZS01 and ZZS05 polypeptides were used to select hybridoma cells for specific antibodies, respectively. The total RNA of the hybridoma cells highly expressed by the antibody was extracted, and the mRNA sequence of the antibodies was obtained by RT- PCR. The two antibodies themselves and their original signal peptides were respectively inserted into two vectors pCHO1.0 and pcDNA3.1 for expression in CHO. After centrifugation, the expressed cell culture medium was subjected to the protein-G affinity column. The antibodies were eluted with glycine solution at pH 2.6 and further purified by ion exchange resin. The antibodies concentrated with a suitable buffer were placed in a refrigerator (-20°C) for later use.

The sequence information of the obtained murine monoclonal antibodies is shown in Table 2.

Table 2 the sequence information of murine monoclonal antibodies (underlined as CDR).

| Murine monoclonal antibodies | Description | SEQ ID NO | Sequence |
|---|---|---|---|
| 106 (anti-zzs01) | Full length Heavy chain | 3 | QIQLVQSGPELKKPGETVKISCKASGYTFTNYAVNWVIQAPGK GLKWMGWINAYTGEPTYADDFKGRFAFSLETSASTAYLQINNL<br><br>KNEDMGIYFCARGGYDTYALDYWGQGTSVTVSSAKTTPPSVY PLAPGSAAQTNSMVTLGCLVKGYFPEPVTVTWNSGSLSSGVHT FPAVLQSDLYTLSSSVTVPSSTWPSETVTCNVAHPASSTKVDKK IVPRDCGCKPCICTVPEVSSVFIFPPPKPKDVLTITLTPKVTCVVVD ISKDDPEVQFSWFVDDVEVHTAQTQPREEQFNSTFRSVSELPIM HQDWLNGKEFKCRVNSAAFPAPIEKTISKTKGRPKAPQVYTIPP PKEQMAKDKVSLTCMITDFFPEDITVEWQWNGQPAENYKNTQ PIMDTDGSYFVYSKLNVQKSNWEAGNTFTCSVLHEGLHNHHT EKSLSHSPGK |
| | Full length Light chain | 4 | DVVMTQTPLTLSVTIGQPASISCKSSQSLLYSNGKTYLSWFLQR PGQSPKRLIYLVSKLDSGVPDRFTGSGSGTDFTLKISRVEAEDLG VYYCVQGTHFPFTFGSGTKLDIKRADAAPTVSIFPPSSEQLTSGG ASVVCFLNNFYPKDINVKWKIDGSERQNGVLNSWTDQDSKDS TYSMSSTLTLTKDEYERHNSYTCEATHKTSTSPIVKSFNRNEC |
| | VH | 5 | QIQLVQSGPELKKPGETVKISCKAS<u>GYTFTNYA</u>VNWVIQAPGK GLKWMGW<u>INAYTGEPT</u>YADDFKGRFAFSLETSASTAYLQINNL KNEDMGIYFC<u>ARGGYDTYALDY</u>WGQGTSVTVSS |
| | VL | 6 | DVVMTQTPLTLSVTIGQPASISCKSS<u>QSLLYSNGKTY</u>LSWFLQR PGQSPKRLIY<u>LVS</u>KLDSGVPDRFTGSGSGTDFTLKISRVEAEDLG VYYC<u>VQGTHFPFT</u>FGSGTKLDIK |
| | HCDR1 | 7 | GYTFTNYA |
| | HCDR2 | 8 | INAYTGEP |
| | HCDR3 | 9 | ARGGYDTYALDY |
| | LCDR1 | 10 | QSLLYSNGKTY |
| | LCDR2 | 11 | LVS |
| | LCDR3 | 12 | VQGTHFPFT |

(continued)

| Murine monoclonal antibodies | Description | SEQ ID NO | Sequence |
|---|---|---|---|
| F11 (anti-zzs05) | Full length Heavy chain | 13 | EVQLQQSGPELVKPGASVKISCKTSGYTFTEYTMHWVKQSHGK TLEWIGGIYPNNSGTRYNQKFRGKATLTADKSSSTAYMELRSL TTEDSAVYYCHRYELYWYFDVWGAGTTVTVSSAKTTPPSVYP LAPGSAAQTNSMVTLGCLVKGYFPEPVTVTWNSGSLSSGVHTF PAVLQSDLYTLSSSVTVPSSTWPSETVTCNVAHPASSTKVDKKI VPRDCGCKPCICTVPEVSSVFIFPPKPKDVLTITLTPKVTCVVVDI SKDDPEVQFSWFVDDVEVHTAQTQPREEQFNSTFRSVSELPIMH QDWLNGKEFKCRVNSAAFPAPIEKTISKTKGRPKAPQVYTIPPP KEQMAKDKVSLTCMITDFFPEDITVEWQWNGQPAENYKNTQPI MDTDGSYFVYSKLNVQKSNWEAGNTFTCSVLHEGLHNHHTEK SLSHSPGK |
| | Full length Light chain | 14 | DIVLTQSPASLAVSLGQRATISCRASKSVSTAGYSYMHWYQQK PGQPPKLLIYLTSNLESGVPARFSGSGSGTDFTLNIHPVEEEDAA TYYCQHSREVPYTFGGGTKLEIKRADAAPTVSIFPPSSEQLTSGG ASVVCFLNNFYPKDINVKWKIDGSERQNGVLNSWTDQDSKDS TYSMSSTLTLTKDEYERHNSYTCEATHKTSTSPIVKSFNRNEC |
| | VH | 15 | EVQLQQSGPELVKPGASVKISCKTS<u>GYTFTEYT</u>MHWVKQSHGK TLEWIGG<u>IYPNNSGT</u>RYNQKFRGKATLTADKSSSTAYMELRSL TTEDSAVYYC<u>HRYELYWYFDV</u>WGAGTTVTVSS |
| | VL | 16 | DIVLTQSPASLAVSLGQRATISCRAS<u>KSVSTAGYSY</u>MHWYQQK PGQPPKLLIY<u>LTS</u>NLESGVPARFSGSGSGTDFTLNIHPVEEEDAA TYYC<u>QHSREVPYT</u>FGGGTKLEIK |
| | HCDR1 | 17 | GYTFTEYT |
| | HCDR2 | 18 | IYPNNSGT |
| | HCDR3 | 19 | HRYELYWYFDV |
| | LCDR1 | 20 | KSVSTAGYSY |
| | LCDR2 | 21 | LTS |
| | LCDR3 | 22 | QHSREVPYT |

## 3.2. Immunohistochemical Experiment

According to Table 3 and Table 4 below, immunohistochemical examination was performed on pathological tissue sections of tumor patients using 1D6 antibody and F11 antibody, respectively.

Table 3. information of paraffin blocks of the patient's pathological tissue for IHC using antibody 1D6

| Paraffin Block | Organ | Pathological Types | TNM |
|---|---|---|---|
| Des130745B4 | Esophagus | Squamous Cell Carcinoma Grade III | T3N1M0 |
| Des130526B5 | Esophagus | Squamous Cell Carcinoma Grade III | T3N1M0 |
| Rln100124B4 | Lung | Squamous Cell Carcinoma Grade III | T3N2M0 |
| Rln140075B6 | Lung | Squamous Cell Carcinoma Grade III | T3N1M0 |
| Kin070079B3 | Skin | Squamous Cell Carcinoma Grade III | T3N1M0 |
| Kin040028B9 | Skin | Squamous Cell Carcinoma Grade III | T3N0M0 |
| Fdu160107B5 | cervix | Squamous Cell Carcinoma Grade II | T1bN0MO |
| Fdu160110B7 | cervix | Squamous Cell Carcinoma Grade II | T1bN0MO |

(continued)

| Paraffin Block | Organ | Pathological Types | TNM |
|---|---|---|---|
| Doc140380B6 | Tongue base | Squamous Cell Carcinoma Grade II | T2N0M0 |
| Rla130028B6 | Left pharynx | Squamous Cell Carcinoma Grade II | T3N1M0 |
| Rns110055B6 | Nasopharynx | Squamous Cell Carcinoma Grade II | T2N0M0 |
| Kin060124B2 | Skin | Malignant melanoma (right plantar) | T4N0M0 |
| Kin060158B6 | Skin | Malignant Melanoma (Abdominal) | T4N0M0 |

TNM: tumor clinical staging system, T, N, M, respectively represent the primary tumor size state, regional lymphatic metastasis state, and presence or absence of distant metastasis (Tumor, Node, Metastasis).

Table 4 the information of the paraffin blocks of the pathological tissue of patients for IHC using F11 antibody.

| Paraffin Block | Organ | Pathological Types | TNM |
|---|---|---|---|
| | | | |
| Dlv120009B2 | Liver | Hepatocyte Liver Cancer Grade III | T2N0M0 |
| Dlv110125B8 | Liver | Hepatocyte Liver Cancer Grade III | T3N0M0 |
| Dst150026B4 | Gastric | Adenocarcinoma Grade III | T3N1M0 |
| Dst150034B20 | Gastric | Adenocarcinoma Grade III | T4N3M0 |
| Fmg080053B8 | Breast | Non-Special Infiltration Ductal Cancer Grade III | T2N1M0 |
| Fmg150476B13 | Breast | Non-Special Infiltration Ductal Cancer Grade III | T3N1M0 |
| Dre160029B4 | Rectum | Adenocarcinoma Grade II | T3N1M0 |
| Dre160143B4 | Rectum | Adenocarcinoma Grade II | T3N0M0 |
| Etg170007B6 | Thyroid | Thyroid papillary carcinoma | T4N0M0 |
| Etg140027B7 | Thyroid | Thyroid papillary carcinoma | T2N0M0 |
| Mpr130014B9 | Prostate | Prostate Adenocarcinoma Gleason4 Grade (3+ 4) | T2N0M0 |
| Mpr130032B4 | Prostate | Prostate Adenocarcinoma Gleason3 Grade (2+ 4) | T2N0M0 |
| Fov160046B9 | Ovary | Viscous Cancer | T1N0M0 |
| Fov160002B8 | Ovary | High grade serous cancer | T1N0M0 |
| Nct090046B10 | Brain | Oligoglioblastoma Grade II | - |
| Nct130005B1 | Brain | Astroglioblastoma Grade II | - |
| Fdu160107B5 | Cervix | Squamous Cell Carcinoma Grade II | T1bN0MO |
| Fdu160110B7 | Cervix | Squamous Cell Carcinoma Grade II | T1bNOMO |
| Ily060231B2 | Lymph Nodes | (Neck) T Cell Lymphoma | - |
| Ily060244B3 | Lymph Nodes | (Right Neck) Diffuse T Cell Lymphoma | - |
| Ily060329B2 | Lymph Nodes | (Left Neck) Diffuse Large B-Cell Lymphoma | - |
| Ily060385B2 | Lymph Nodes | (Inguinal) Diffuse Large B-Cell Lymphoma | - |
| Rln060561B2 | Lung | Adenocarcinoma Grade III | T2N2M0 |
| Rln070029B3 | Lung | Adenocarcinoma Grade III | T2N2M0 |
| Rln100124B4 | Lung | Squamous Cell Carcinoma Grade III | T3N2M0 |
| Rln100055B2 | Lung | Squamous Cell Carcinoma Grade III | T2N3M0 |
| Des070256B6 | Esophagus | Adenocarcinoma Grade III | T3N1M0 |
| Des051252B10 | Esophagus | Adenocarcinoma Grade III | T40N0M0 |
| Des130745B4 | Esophagus | Squamous Cell Carcinoma Grade III | T3N1M0 |

(continued)

| Paraffin Block | Organ | Pathological Types | TNM |
|---|---|---|---|
| Des130526B5 | Esophagus | Squamous Cell Carcinoma Grade III | T3N1M0 |
| Dpa130297B2 | Pancreas | Adenocarcinoma Grade II | T4N0M0 |
| Dpa030841B15 | Pancreas | Adenocarcinoma Grade III | T2N0M0 |
| Dco120211B5 | Colon | Adenocarcinoma Grade III | T3N1M0 |
| Dco130380B3 | Colon | Adenocarcinoma Grade III | T3N1M0 |
| Kin070079B3 | Skin | (Face) Squamous Cell Carcinoma Grade III | T3N1M0 |
| Kin040028B9 | Skin | (scalp) Squamous Cell Carcinoma Grade III | T3N0M0 |
| Kin060124B2 | Skin | (right plantar) malignant melanoma | T4N0M0 |
| Kin060158B6 | Skin | (Abdominal) Malignant Melanoma | T4N0M0 |
| TNM: tumor clinical staging system, T, N, M, respectively represent the primary tumor size state, regional lymphatic metastasis state, and presence or absence of distant metastasis (Tumor, Node, Metastasis). | | | |

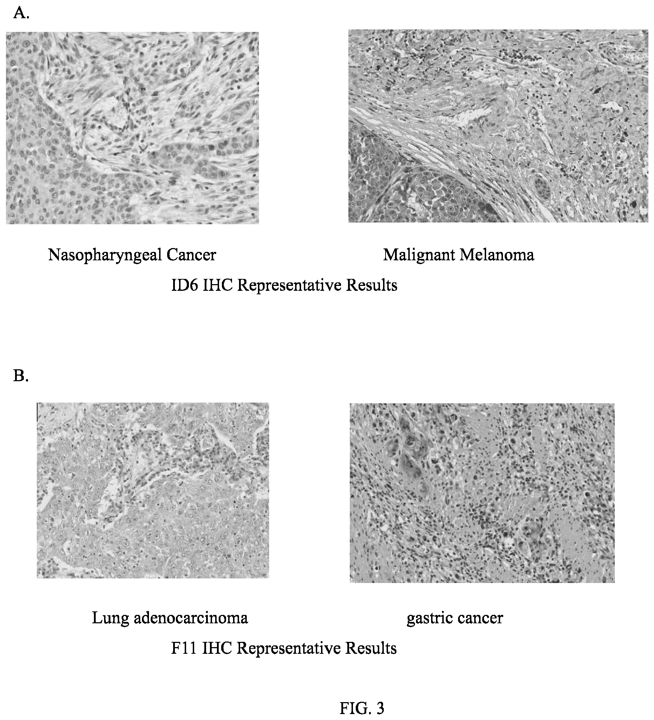

The results showed that the specificity of monoclonal antibodies 1D6 and F11 for tumor cell recognition was consistent with the results of polyclonal antibodies in Example 2. Antibody 1D6 can specifically recognize malignant melanoma and squamous cell carcinoma (including all the tumors in Table 3: nasopharyngeal carcinoma, malignant melanoma, lung squamous cell carcinoma, cervical cancer, skin cancer, glossopharyngeal carcinoma, esophageal squamous cell carcinoma); F11 can specifically recognize all the tumors tested (tumors in Table 4: B-cell lymphoma, T-cell lymphoma, malignant melanoma, lung squamous cell carcinoma, lung adenocarcinoma, liver cancer, cervical squamous cell carcinoma, thyroid cancer, colon adenocarcinoma, ovarian cancer, brain tumor, skin squamous cell carcinoma, prostate cancer, breast cancer, esophageal squamous cell carcinoma, esophageal adenocarcinoma, gastric cancer, pancreatic cancer, rectal cancer). Meanwhile, these antibodies do not bind to paracancerous tissues (normal cells). Representative immunohistochemical results are shown in FIG. 3.

These results suggest that the cTAGE5 protein containing ZZS01 is overexpressed in human malignant melanoma and human squamous cell carcinoma tissue cells, and the cTAGE5 protein containing ZZS05 is overexpressed in all tumor tissue cells tested in the experiments of tumor immunohistochemistry. Therefore, ZZS01 is a cell-specific antigenic epitope for human malignant melanoma and human squamous cell carcinoma tissue, and ZZS05 is a cell-specific antigenic epitope for human tumors.

### 3.3. Mouse Antibody Affinity Assay

Antigen binding affinity of monoclonal murine antibody 1D6 or F11 was determined by Biacore method using antigens ZZS01 and ZZS05, respectively.

General Procedure:

1. Mobile phase buffer preparation: 10-fold dilution of HBS- EB+buffer to 10-fold;
2. Test at 25° C, the mobile phase was HBS- EP+buffer;
3. The antigen was injected into the sensor chip CM5 coated on the goat anti-GST antibody, and the GST- antigen fusion protein was immobilized;
4. Diluted antibodies were injected into the channels of the chip as the binding phase and then the mobile phase as the dissociation phase.

Experimental Setup:

| Ligand | Antigen |
|---|---|
| Grasping Time (s) | 30 |

(continued)

| Ligand | Antigen |
|---|---|
| Binding Time (s) | 120 |
| Disassociation Time (s) | 360 |
| Flow Rate ($\mu$L/min) | 30 |
| Sample Concentration (nM) | 200, 100, 50, 25, 12.5, 6.25, 3.125(F11) 2000, 1000, 500, 250, 125, 62.5, 31.25(1D6) |

The results are as follows:

| Murine Antibody Name | $k_{on}$ (1/Ms) | $k_{off}$(1/s) | $K_D$ (M) | Antigen |
|---|---|---|---|---|
| 1D6 | 2.48 E+03 | 1.19 E-05 | 4.82 E-09 | Zzs01 |
| F11 | 1.37 E+05 | 9.49 E-04 | 6.90 E-09 | Zzs05 |

### 3.4. Humanization of mouse antibodies

Protein Blast of NIH was used to find the amino acid sequences of Protein Data Bank (PDB) human antibody VH and VL close to 1D6 or F11. After the amino acid sequences were aligned, the corresponding spatial structures were used as templates of 1D6 VH, 1D6 VL, F11 VH, and F11 VL, and the spatial structure models of 1D6 VH, 1D6 VL, F11 VH, and F11 VL were constructed by replacing the amino acid sequences different from their respective amino acid sequences in PDB on the model establishment and structure display software WinCoot in CCP4[5]. The relative positions of the VH and VL aligned to PDB of each antibody were assembled to form a 3D spatial model of the variable regions. The extreme value of the lowest energy of the structure was calculated by software GROMACS[6], so as to obtain the spatial structure models of the 1D6 variable regions and the F11 variable regions simulated by the computer.

### 1D6 humanization

The structure and amino acid sequence in PDB corresponding to 1D6 VH is 1IHI, and the structure and amino acid sequence in PDB corresponding to 1D6 VL is 5MYX. Amino acid residues different from the 1D6 variable region were replaced on WinCoot. Each replacement was examined to check whether the structure is reasonable, and all the results were reasonable, and none will affect the secondary structure of the model. The extreme value of the lowest energy of the structure was calculated by using GROMACS, and the 3D spatial structure of 1D6 was obtained by computer simulation.
BioPhi[4]Software was used for humanization of 1D6 variable region:

Humanization settings: Chothia[1] for amino acid numbering and IMGT[2, 3] for defining CDRs
Humanization method 1: Sapiens, humanized for 4 iterations, with a threshold greater than or equal to 10%.

Sapiens Humanized 1D6 Variable Region:

>Antibody1 VH (Humanized Antibody 1D6 Sapiens 4iter parental Imgt CDRs BioPhi)

QVQLVQSGSELKKPGASVKISCKASGYTFTNYAVNWVRQAPGQGLEWMGWINA
YTGEPTYAQDFTGRFVFSLDTSVSTAYLQINNLKAEDTAIYFCARGGYDTYALDYWG
QGTLVTVSS (SEQ ID NO:23)

>Antibody1 VL (Humanized Antibody 1D6 Sapiens 4iter parental Imgt CDRs BioPhi)

DVVMTQSPLSLPVTPGQPASISCRSSQSLLYSNGKTYLSWFLQRPGQSPRRLIYLV
SKRDSGVPDRFSGSGSGTDFTLKISRVEAEDVGVYYCVQGTHFPFTFGQGTKLDIK(SE
Q ID NO:24)

Humanization method 2: Graft, the germlines of the heavy and light chains were automatic, the threshold was greater than or equal to 10%, and the result is:

>Antibody1 VH (Humanized Antibody 1D6 Imgt Straight graft auto auto BioPhi)

QVQLVQSGSELKKPGASVKVSCKASGYTFTNYAMNWVRQAPGQGLEWMGWIN
AYTGEPTYAQGFTGRFVFSLDTSVSTAYLQISSLKAEDTAVYYCARGGYDTYALDYW
GQGTTVTVSS(SEQ ID NO:25)

>Antibody1 VL (Humanized Antibody 1D6 Imgt Straight graft auto auto BioPhi)

DVVMTQSPLSLPVTLGQPASISCRSSQSLLYSNGKTYLNWFQQRPGQSPRRLIYLV
SNRDSGVPDRFSGSGSGTDFTLKISRVEAEDVGVYYCVQGTHFPFTFGPGTKVDIK(SE
Q ID NO:26)

**F11 humanization**

The structure and amino acid sequence in PDB corresponding to F11 VH is 6TKC, and the structure and amino acid sequence in PDB corresponding to F11 VL is 5FB8. Amino acid residues different from the variable region of F11 were replaced on WinCoot. Each replacement was examined to check whether the structure is reasonable, and all the results were reasonable, and none will affect the secondary structure of the model. The extreme value of the lowest energy of the structure was calculated by GROMACS, and the 3-D spatial structure of F 11 was obtained by computer simulation.

BioPhi software was used for F11 variable region humanization
Humanization settings: Chothia for amino acid numbering and IMGT for definition of CDRs
Humanization method 1: Sapiens, humanized for 4 iterations, with a threshold greater than or equal to 10%

Sapiens Humanized F11 Variable Region:

>Antibody1 VH (Humanized Antibody F11 Sapiens 4iter parental Imgt CDRs BioPhi)

QVQLVQSGAEVKKPGASVKVSCKTSGYTFTEYTMHWVRQAPGQGLEWMGWI
YPNNSGTNYAQKFQGRVTLTADTSTSTAYMELRSLTSDDTAVYYCHRYELYWYFDVW
GRGTLVTVSS(SEQ ID NO:27)

>Antibody1 VL (Humanized Antibody1 Sapiens 4iter parental Imgt CDRs BioPhi)

DIVLTQSPASLAVSPGERATISCRASKSVSTAGYSYLHWYQQKPGQPPKLLIYLTS
NRASGVPDRFSGSGSGTDFTLTISRVEAEDVAVYYCQHSREVPYTFGQGTKLEIK(SEQ
ID NO:28)

Humanization method 2: Graft, the germlines of the heavy and light chains were automatic, the threshold was greater than or equal to 10%, and the result is:

>Antibody1 VH (Humanized Antibody F11 Imgt Straight graft auto auto BioPhi)

QVQLVQSGAEVKKPGASVKVSCKASGYTFTEYTMHWVRQAPGQRLEWMGWI YPNNSGTKYSQKFQGRVTITRDTSASTAYMELSSLRSEDTAVYYCHRYELYWYFDVW GRGTLVTVSS(SEQ ID NO:29)

>Antibody1 VL (Humanized Antibody1 Imgt Straight graft auto auto BioPhi)

DIVLTQSPASLAVSPGQRATITCRASKSVSTAGYSYIHWYQQKPGQPPKLLIYLTS NKDTGVPARFSGSGSGTDFTLTINPVEANDTANYYCQHSREVPYTFGQGTKLEIK(SEQ ID NO:30)

The humanized sequences of 1D6 and F11 were brought into the 3-D structures of 1D6 and F11 simulated by computer, the secondary structure was not changed, and the secondary structure calculated by the energy extreme value was not significantly changed. Humanized 1D6 and F11 were able to retain the binding capacity of their parent antibodies.

**Example 4: Monoclonal Antibodies Inhibit Tumor Growth in Mice**

In this example, the effect of antibody F11 on tumor proliferation in human malignant melanoma or human triple negative breast cancer CB-17 SCID mouse subcutaneous transplanted tumor model was studied.

A-375 cells (malignant melanoma cell line) and HCC1937 cells (human breast cancer cells) were purchased from American Tissue Culture Collection (ATCC, Rockville, MD, USA).

CB-17 SCID mice, weighing 18-20 g, female, provided by Beijing Vital River (license number: SCXK (Beijing) 2016-0006), fed under SPF grade feeding environment, indoor temperature controlled at $23\pm2°$ C., free diet and water intake. The total number of animals was 32. 3 days of adaptive feeding before inoculation.

A-375 cells or HCC1937 cells in the logarithmic growth phase were taken to prepare a cell suspension at a concentration of $2.5\times10^6$ cells/ml, and nude mice were inoculated at a right underarm of 0.2 ml/mouse to establish a A-375 cell or HCC1937 mouse transplanted tumor model. On the next day of tumor cell inoculation, each tumor mouse was randomly divided into an experimental group (8 mice) and a control group (8 mice), marked, and then administered.

Administration design: F11 or buffer was injected into the tail vein for 4 times. The first administration on the next day of tumor cell inoculation was the first day of administration, followed by administration on days 3, 7, 13. The experimental group was given F11 (10 mg/Kg), and the blank control group was given buffer (equal volume to F11). See Table 5.

Table 5. Mouse tumor experiment design

| Inoculated Tumor | Experimental Method | Administration |
|---|---|---|
| A-375 | Experimental Group | F11 |
| A-375 | Blank Control Group | Buffer of F11 |
| HCC1937 | Experimental Group | F11 |
| HCC1937 | Blank Control Group | Buffer of F11 |

Mice transplanted tumor diameter was measured with a vernier caliper. The anti-tumor effect of the test substance was dynamically observed by using the method for measuring the diameter of the tumor. The tumor diameter was measured once every 3 days, and the body weight of mice was examined once every 3 days. After 60 days of tumor cell inoculation, all mice were sacrificed by carbon dioxide method, observed by laparotomy, tumors were removed, irrelevant tissues were carefully removed, washed 2-3 times with D-Hanks solution, blood was washed, water was drained and stored, and the weight and volume were measured. The calculation formula of tumor volume (TV) is:

$$\text{Tumor volume (mm}^3) = 0.5[w1\times(w2)^2],$$

W1 is the largest diameter (mm), and W2 is the diameter perpendicular to W1(mm).

Statistical differences between data groups were tested by one-way ANOVA and Tukey's test, and P values less than 0.05 were considered significant differences.

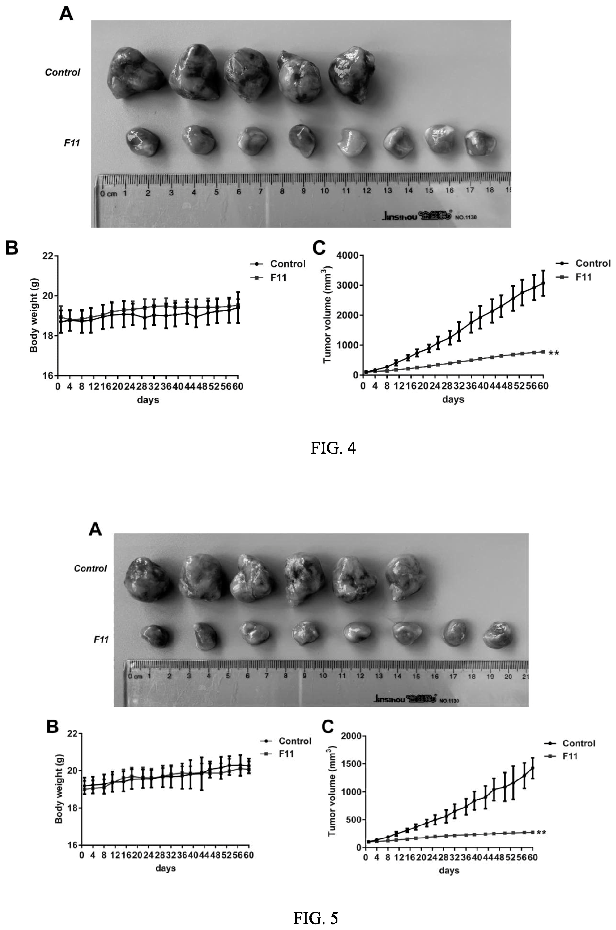

The experimental results of A-375 cell transplanted tumors were shown in FIG. 4, and there was no significant difference between the body weights of mice in each group; compared with the blank control group, the tumor volume in the F11 group was significantly reduced. It is suggested that the monoclonal antibody **F11** can inhibit the growth of A-375 cell transplanted tumors. (3 control mice died within 60 days after tumor cell inoculation)

The experimental results of HCC1937 cell transplanted tumors were shown in FIG. 5, and there was no significant difference between the body weights of mice in each group; compared with the blank control group, the tumor volume in the F11 group was significantly reduced. It is suggested that the monoclonal antibody F11 can inhibit the growth of HCC1937 cell transplanted tumors. (2 control mice died within 60 days after tumor cell inoculation)

**Example 5: Preparation and Characterization of Human Monoclonal Antibody**

CAMouse (Immune Gene Humanized Mice from Chongqing Jiangbo Biotechnology Co., Ltd., Patent Application Publication No.: CN 108486126 A) were respectively immunized with the fusion proteins GST- ZZS01 and GST- ZZS05. After the conventional immunization was completed, the spleen and bone marrow of the mice were taken and resuspended by grinding, and the monocytes were enriched by density gradient centrifugation using Histopaque ® -1083. CD138+ B cells were isolated using the CD138+ kit. After isolating the plasma cells, human antibodies were obtained by the following two methods:

1. The isolated plasma cells were introduced into a Beacon instrument (manufacturer: BLI, model: BSN0079). The positive clone obtained by BEACON against the target protein was followed by gene sequencing of the antibody.

2. The SP2/0 cell suspension and the spleen (plasma) cell suspension were mixed for cell fusion. After fusion, the fused cells were resuspended with a fusion medium, mixed uniformly to prepare a cell suspension, and then spread into a 96-well cell plate. The fused 96-well cell plate was placed in a 5.5% $CO_2$, 37°C carbon dioxide incubator for 5-8 days. The supernatant in the cell culture plate was inhaled into the corresponding ELISA detection plate (coated with immune antigen, test antigen, reverse sieve protein), the hybridoma parent clone supernatant (primary antibody) was added, incubated at 37° C for 60 minutes, and then the secondary antibody Anti-Human IgG (H & L) (GOAT) Antibody Peroxidase Conjugated (Min X Bv Ch Gt GP Ham Ms Rb Rt & Sh Serum Proteins) was added, incubated at 37° C for 30 minutes. Then TMB chromogenic solution was added, incubated at 25° C for 15 minutes, and HCl was added to terminate the reaction. The cells in the screened positive wells were subcloned by a limited dilution method. The subcloned 96-well plate was placed in a 37° C, 5%-8% carbon dioxide incubator for 6-11 days. After screening out monoclonal antibody expressing cells, gene sequencing of antibodies was performed.

After combining the monoclonal antibody expressing cells obtained by the two methods, the gene sequences of the antibody variable region were measured by NGS high-throughput sequencing method to obtain the antibody variable region sequences.

The measured antibody genes were compared with the gene sequences in the IMGT database[2, 3] to confirm that all antibody gene sequences were human antibody gene sequences. The recombinant human antibody was expressed by CHO, and the corresponding antigen affinity was measured using the Biacore method (see the above examples). The CDR sequences were obtained using BioPhi[4] software. The amino acid sequence numbering of the antibody variable region was defined by the method of Chothia[1] and CDRs were defined by the method IMGT[2, 3]. The results are shown in Table 6 and Table 7.

Table 6. human antibodies against Zzs01

| Antibody | Description | SEQ ID NO | Sequence | Affinity (K_D) |
|---|---|---|---|---|
| h1 | VH | 31 | EVQLVESGGGLVKPGGSLRLSCAASGFTFSSYSMSWVRQAPGK GLEWVSSISSRSNYIYYADSVKGRFTISRDNAKNSLYLQVNSLR AEDTAVYYCARGSGSYYVFDYWGQGTLVTVSS | 2.89E-10 |
| | VL | 32 | EIVMTQSPATLSVSPGDRATLSCRASQSISSDLAWYQQKPGQAP RLLIYAASTRATGFPARFSGSGSGTEFTLTISSLQPEDFAVYYCQ QYNNWPMYAFGQGTKLEIK | |
| | HCDR1 | 33 | GFTFSSYS | |
| | HCDR2 | 34 | ISSRSNYI | |
| | HCDR3 | 35 | ARGSGSYYVFDY | |
| | LCDR1 | 36 | QSISSD | |
| | LCDR2 | 37 | AAS | |
| | LCDR3 | 38 | QQYNNWPMYA | |
| h2 | VH | 39 | QVQLVESGGGLVKPGGSLRLSCAASGFTFSAHYMSWIRQAPGK GLEWVSYISPSSSYTVYADSVKGRFTISRDNAKNSLFLQMNSLR AEDTAVYYCAREGSPGGQGTLVTVSS | 5.24E-09 |
| | VL | 40 | DVVMTQSPLSLPVTLGQPASISCRPSQSLVDSDGNTYLSWFQQR PGQSPRRLIYKVSDRDSGVPDRFSGSGSVTDFTLKISRVEAEDV GIYYCMQGTHWPFTFGPGTKVDIK | |
| | HCDR1 | 41 | GFTFSAHY | |
| | HCDR2 | 42 | ISPSSSYT | |
| | HCDR3 | 43 | AREGSP | |
| | LCDR1 | 44 | QSLVDSDGNTY | |
| | LCDR2 | 45 | KVS | |
| | LCDR3 | 46 | MQGTHWPFT | |
| h3 | VH | 47 | QVQLVQSGAEVKQPGASVKVSCKASGYTFTSYAMHWVRQAP GQRLEWMGWIHAGMGVTKSSQKFQGRITITRDTSASTAYMEL SSLRSEDTAVYSCVRQQLGFDYWGQGTLVTVSS | 3.29E-10 |
| | VL | 48 | DIVMTQSPLSLPVTPGEPASIFCRSSQSLLHSNGNKYLDWYLQK PGQSPQLLIYLGSNRASGVPDRFSGSGSGTDFTLKISRVEAEDV GVYYCMQALQTPYTFGQGTKLEIK | |
| | HCDR1 | 49 | GYTFTSYA | |
| | HCDR2 | 50 | IHAGMGVT | |
| | HCDR3 | 51 | VRQQLGFDY | |
| | LCDR1 | 52 | QSLLHSNGNKY | |
| | LCDR2 | 53 | LGS | |
| | LCDR3 | 54 | MQALQTPYT | |

(continued)

| Antibody | Description | SEQ ID NO | Sequence | Affinity (K$_D$) |
|---|---|---|---|---|
| h4 | VH | 55 | QVQLVESGGGLVKPGGSLRLSCAASGFTFSDHYMSWIRQTPGKGLEWVSYISSSGTYTNYADSVKGRFIISRDNAKNSLFLQMNSLRAEDTAVYYCAREGSPGGQGTLVTVSS | 2.86E-09 |
| | VL | 56 | DVVMTQSPLSLPVTLGQPASISCRSSQSLVFSDGNTYLNWFQQRPGQSPRRLIYKVSDRDPGVPERFSGSGSGTDFTLKISRVEAEDIGVYYCMQGSHWPFTFGPGTKVDIK | |
| | HCDR1 | 57 | GFTFSDHY | |
| | HCDR2 | 58 | ISSSGTYT | |
| | HCDR3 | 59 | AREGSP | |
| | LCDR1 | 60 | QSLVFSDGNTY | |
| | LCDR2 | 61 | KVS | |
| | LCDR3 | 62 | MQGSHWPFT | |
| h5 | VH | 63 | QVQLVESGGGLVKPGGSLRLSCAASGFTFSDHYMSWIRQAPGKGLEWVAYISSSSTYTKYGDSVKGRFTISRDNAKNSLYLQMNSLRAEDTAVYYCAREGSPGGQGTLVTVSS | 1.01E-08 |
| | VL | 64 | DVVMTQSPLSLPVTLGQPASISCRSSQSLVFSDGNTYLSWFQQRPGQSPRRLIFKVSDRDSGVPDRFSGSGSGTDFTLKISRMEAEDVGVYYCMQGTQWPFTFGPGTKVDIK | |
| | HCDR1 | 65 | GFTFSDHY | |
| | HCDR2 | 66 | ISSSSTYTK | |
| | HCDR3 | 67 | CAREGSP | |
| | LCDR1 | 68 | QSLVFSDGNTY | |
| | LCDR2 | 69 | KVS | |
| | LCDR3 | 70 | MQGTQWPFT | |
| h6 | VH | 71 | QVQLVESGGGLVKPGGTLRLSCEASGFTFSDHYMSWIRQAPGKGLEWVSYISSVSGYINYADSVKGRFTISRDNAKKSLYLQMNSLRAEDTAVYYCARGGERFFDYWGQGTLVTVSS | 1.20E-09 |
| | VL | 72 | DIVMTQSPDSLAVSLGERATINCKSSQSVLYNFNNKIYLAWYQQKPGQPPKLLISWASTRESGVPDRFSGSGSGTDFTLTISSLQAEDVAVYYCQQHYSTPLTFGGGTKVEIK | |
| | HCDR1 | 73 | GFTFSDHY | |
| | HCDR2 | 74 | ISSVSGYI | |
| | HCDR3 | 75 | ARGGERFFDY | |
| | LCDR1 | 76 | QSVLYNFNNKIY | |
| | LCDR2 | 77 | WAS | |
| | LCDR3 | 78 | QQHYSTPLT | |

(continued)

| Antibody | Description | SEQ ID NO | Sequence | Affinity ($K_D$) |
|---|---|---|---|---|
| h7 | VH | 79 | QVQLVESGGGLVKPGGTLRLSCEASGFTFSDHYMSWIRQAPGK GLEWVSYISSVSGYINYADSVKGRFTISRDNAKKSLYLQMNSL RAEDTAVYYCARGGERFFDYWGQGTLVTVSS | 1.67E-09 |
| | VL | 80 | DIVMTQSPDSLAVSLGERATINCKSSQNVLYNFNDKNYLAWY QQKPGQPPKLLISWASTRESGVPDRFSGSGSGTDFTLTISSLQAE DVAVYYCQQHYSTPLTFGGGTKVEIK | |
| | HCDR1 | 81 | GFTFSDHY | |
| | HCDR2 | 82 | ISSVSGYI | |
| | HCDR3 | 83 | ARGGERFFDY | |
| | LCDR1 | 84 | QNVLYNFNDKNY | |
| | LCDR2 | 85 | WAS | |
| | LCDR3 | 86 | QQHYSTPLT | |
| h8 | VH | 87 | QVQLVESGGGLVKPGGSLRLSCEASGFTFSDHYMSWIRQAPGK GLEWVSYISSSSGYTNYADSVKGRFTISRDNAKNSLYLQMNSL RAEDTAVYYCARGGERFFDYWGQGTLVTVSS | 3.90E-09 |
| | VL | 88 | DIVMTQSPDSLAVSLGERATINCKSSQSVLYSFNNKNYLAWYQ QKPGQPPKLLIYWASTRESGVPDRFSGSGSGTDFTLTISSLQAED VAVYYCQQHYSTPLTFGGGTKVEIK | |
| | HCDR1 | 89 | GFTFSDHY | |
| | HCDR2 | 90 | ISSSSGYT | |
| | HCDR3 | 91 | ARGGERFFDY | |
| | LCDR1 | 92 | QSVLYSFNNKNY | |
| | LCDR2 | 93 | WAS | |
| | LCDR3 | 94 | QQHYSTPLT | |
| h9 | VH | 95 | QVQLVESGGGLVKPGGSLRLSCEASGFTFSDHYMSWIRQAPGK GLEWVSYISSSSGYTNYADSVKGRFTISRDNAKKSLYLQMNSL RAEDTAVYYCARGGERFFDYWGQGTLVTVSS | 2.46E-09 |
| | VL | 96 | DIVMTQSPDSLAVSLGERATINCKSSQSVLYSFNNKNYLAWYQ QKSGQPPKLLISWASTRESGVPDRFSGSGSGTDFTLTISSLQAED VAVYYCQQHYSTPLTFGGGTKVEIK | |
| | HCDR1 | 97 | GFTFSDHY | |
| | HCDR2 | 98 | ISSSSGYT | |
| | HCDR3 | 99 | ARGGERFFDY | |
| | LCDR1 | 100 | QSVLYSFNNKNY | |
| | LCDR2 | 101 | WAS | |
| | LCDR3 | 102 | QQHYSTPLT | |

(continued)

| Antibody | Description | SEQ ID NO | Sequence | Affinity (K_D) |
|---|---|---|---|---|
| h10 | VH | 103 | QVQLVESGGGLVKPGGSLRLSCEASGFTFSAHYMSWIRQAPGKGLEYVSYISSSSSYTNYVDSVKGRFTISRDNAKNSLYLQMNSLRVEDTAVYYCAREGSPGGQGTLVTVSS | 8.47E-11 |
| | VL | 104 | DVVLTQSPLSLPVTLGQPASISCRSSQSLVDSDGNTYLSWFHQRPGQSPRRLIYKVSDRDSGVPDRFSGSGSGTDFTLKISRVEAEDVGVYYCMEGTHWPFTFGPGTKVDIK | |
| | HCDR1 | 105 | GFTFSAHY | |
| | HCDR2 | 106 | ISSSSSYT | |
| | HCDR3 | 107 | AREGSP | |
| | LCDR1 | 108 | QSLVDSDGNTY | |
| | LCDR2 | 109 | KVS | |
| | LCDR3 | 110 | MEGTHWPFT | |
| h11 | VH | 111 | QVQLVESGGGLVKPGGSLRLSCEASGFTFSAHYMSWIRQAPGKGLEYVSYISGSSSYTNYVDSVKGRFTISRDNAKNSLYLQMNSLRVEDTAVYYCAREGSPGGQGTLVTVSS | 6.52E-10 |
| | VL | 112 | DVVLTQSPLSLPVTLGQPASISCRSSQSLVDSDGNTYLSWFHQRPGQSPRRLIYKVSDRDSGVPDRFSGSGSGTDFTLKISRVEAEDVGVYYCMEGTHWPFTFGPGTKVDIK | |
| | HCDR1 | 113 | GFTFSAHY | |
| | HCDR2 | 114 | ISGSSSYT | |
| | HCDR3 | 115 | AREGSP | |
| | LCDR1 | 116 | QSLVDSDGNTY | |
| | LCDR2 | 117 | KVS | |
| | LCDR3 | 118 | MEGTHWPFT | |
| h12 | VH | 119 | QVQLVESGGGLVKPGGSLRLSCAASGFSFSDSYMSWLRQAPGKGLEWVSYISSSSSYTKYTDSVKGRFTISRDNAKNSLYLQMSSLRAEDTAVYYCAREGSPGGQGTLVTVSS | 6.03E-09 |
| | VL | 120 | DVVMTQSPLSLPVTLGQPASFSCRSSQSLVFTDGNTFLSWFLQRPGQSPRRLIYRVSNRDPGVPDRFSGSGSGTDFTLKISRVEAEDVGVYYCMQGTQWPFTFGPGTKVDIK | |
| | HCDR1 | 121 | GFSFSDSY | |
| | HCDR2 | 122 | ISSSSSYT | |
| | HCDR3 | 123 | AREGSP | |
| | LCDR1 | 124 | QSLVFTDGNTF | |
| | LCDR2 | 125 | RVS | |
| | LCDR3 | 126 | MQGTQWPFT | |

(continued)

| Antibody | Description | SEQ ID NO | Sequence | Affinity (K$_D$) |
|---|---|---|---|---|
| h13 | VH | 127 | QVQLVESGGGLVKPGGSLRLSCAASGFSFSDSYMSWLRQAPGKGLEWVSYISSSSSYTKYADSVKGRFTISRDNAKNSLYLQMNSLRAEDTAVYYCAREGSPGGQGTLVTVSS | 3.66E-09 |
| | VL | 128 | DVVLTQSPLSLPVTLGQPASFSCRSSQSLVFTDGNTFLSWFLQRPGQSPRRLIYRVSNRDPGVPDRFSGSGSGTDFTLKISRVEAEDVGVYYCMQGTQWPFTFGPGTKVDIK | |
| | HCDR1 | 129 | GFSFSDSY | |
| | HCDR2 | 130 | ISSSSSYT | |
| | HCDR3 | 131 | AREGSP | |
| | LCDR1 | 132 | QSLVFTDGNTF | |
| | LCDR2 | 133 | RVS | |
| | LCDR3 | 134 | MQGTQWPFT | |
| h14 | VH | 135 | QVQLVESGGGLVEPGGSLRLSCAASGFTFSDHYMSWIRQAPGRGLEWVSYISNSGGYTNYADSVKGRFTISRDNAKNSLYLQMNSLRAEDTAVYYCAREGSPGGQGTLVTVSS | 4.39E-09 |
| | VL | 136 | DVVMTQSPLSLPVTLGQPASMSCRSSQSLVDSDGNTYLSWFQQRPGQSPRRLIYKVSDRDSGVPDRFSGGGSGTDFTLKISRVEAEDVGIYYCMQGTRWPFTFGPGTKVDIK | |
| | HCDR1 | 137 | GFTFSDHY | |
| | HCDR2 | 138 | ISNSGGYT | |
| | HCDR3 | 139 | AREGSP | |
| | LCDR1 | 140 | QSLVDSDGNTY | |
| | LCDR2 | 141 | KVS | |
| | LCDR3 | 142 | MQGTRWPFT | |
| h15 | VH | 143 | QVQLVESGGGLVRPGGSLRLSCAASGFTFSDHYMSWIRQAPGKGLEWVSYISGSSSYTKYADSVKGRFTISRDNAKNSLYLQMNSLRAEDTAVYYCAREGSPGGQGTLVTVSS | 5.78E-09 |
| | VL | 144 | DVVLTQSPLSLPVTLGQPASISCRSSQSLVFSDGNTFLAWFQQRPGQSPRRLIYRISNRDSGVPDRFSGSGSDTDFTLKISRVEAEDVGVYYCMQGTQWPFTFGPGTKVDIK | |
| | HCDR1 | 145 | GFTFSDHY | |
| | HCDR2 | 146 | ISGSSSYT | |
| | HCDR3 | 147 | AREGSP | |
| | LCDR1 | 148 | QSLVFSDGNTF | |
| | LCDR2 | 149 | RIS | |
| | LCDR3 | 150 | MQGTQWPFT | |

(continued)

| Antibody | Description | SEQ ID NO | Sequence | Affinity (K$_D$) |
|---|---|---|---|---|
| h16 | VH | 151 | QVQLVESGGGLVKPGGSLRLSCEASGFTFSAHYMSWIRQAPGK GLEYVSYISSSSSYTNYADSVKGRFTISRDNAKNSLYLQMNSLR AEDTAVYYCAREGSPGGQGTLVTVSS | 2.17E-09 |
| | VL | 152 | DVVLTQSPLSLPVTLGQPASISCRSSQSLVDSDGNTYLSWFHQR PGQSPRRLIYKVSDRDSGVPDRFSGSGSGTDFTLKISRVEAEDV GVYYCMEGTHWPFTFGPGTKVDIK | |
| | HCDR1 | 153 | GFTFSAHY | |
| | HCDR2 | 154 | ISSSSSYT | |
| | HCDR3 | 155 | AREGSP | |
| | LCDR1 | 156 | QSLVDSDGNTY | |
| | LCDR2 | 157 | KVS | |
| | LCDR3 | 158 | MEGTHWPFT | |
| h17 | VH | 159 | QVQLVESGGGLVEPGGSLRLSCAASGFTFSDHYMSWIRQAPGR GLEWVSYISNSGGYTNYADSVKGRFTISRDNAKNSLYLQMNSL RAEDTAVYYCAREGSPGGQGTLVTVSS | 3.18E-09 |
| | VL | 160 | DVVMTQSPLSLPVTLGQPASMSCRSSQSLVDSDGNTYLSWFQQ RPGQSPRRLIYKVSDRDSGVPDRFSGGGSGTDFTLKIRRVEAED VGIYYCMQGTRWPFTFGPGTKVDIK | |
| | HCDR1 | 161 | GFTFSDHY | |
| | HCDR2 | 162 | ISNSGGYT | |
| | HCDR3 | 163 | AREGSP | |
| | LCDR1 | 164 | QSLVDSDGNTY | |
| | LCDR2 | 165 | KVS | |
| | LCDR3 | 166 | MQGTRWPFT | |
| 18 | VH | 167 | QVQLVESGGGLVKPGGSLRLSCAASGFTFSASYMSWIRQAPGK GLEWVSYISSSSTYTNYADSVKGRFTISRDNAKNSLYLQMDSL RAEDTAMYFCAREGSPGGQGTLVTVSS | 3.46E-09 |
| | VL | 168 | DVVMTQSPLSLPVTLGQSASISCRSSQSLVYSDGDTFLSWFQQR PGQSPRRLIYMVSNRDPGVPDRFSGSGSGTDFTLKISRVEAEDV GIYYCMQGTNWPFTFGPGTRVDIK | |
| | HCDR1 | 169 | GFTFSASY | |
| | HCDR2 | 170 | ISSSSTYT | |
| | HCDR3 | 171 | AREGSP | |
| | LCDR1 | 172 | QSLVYSDGDTF | |
| | LCDR2 | 173 | MVS | |
| | LCDR3 | 174 | MQGTNWPFT | |

(continued)

| Antibody | Description | SEQ ID NO | Sequence | Affinity (K_D) |
|---|---|---|---|---|
| h19 | VH | 175 | QVQLVESGGGLVKPGGSLRLSCAASGFSFSDSYMSWIRQAPGK GLEWVSYISSSSSYTNYADSVKGRFTISRDNAKNSLYLQMNSL RVEDTAVYYCAREGSPGGQGTLVTVSS | 3.53E-09 |
| | VL | 176 | DVVLTQSPLSLPVTLGQPASISCRSSQSLVDSDGNTYLSWFQQR PGQSPRRLIYRVSNRDSGVPDRFSGSGSGTDFTLKISRVEAEDV GVYYCMQGTHWPFTFGPGTKVDIK | |
| | HCDR1 | 177 | GFSFSDSY | |
| | HCDR2 | 178 | ISSSSSYT | |
| | HCDR3 | 179 | AREGSP | |
| | LCDR1 | 180 | QSLVDSDGNTY | |
| | LCDR2 | 181 | RVS | |
| | LCDR3 | 182 | MQGTHWPFT | |
| h20 | VH | 183 | QVQLVESGGGLVKPGGSLRLSCAASGFSFSDSYMSWIRQAPGK GLEWVSYISSSSSYTNYVDSVKGRFTISRDNAKNSLYLQMNSL RVEDTAVYYCAREGSPGGQGTLVTVSS | 2.00E-09 |
| | VL | 184 | DVVMTQSPLSLPVTLGQPASISCRSSQSLVDSDGNTYLSWFQQR PGQSPRRLIYRVSNRDSGVPDRFSGSGSGTDFTLKISRVEAEDV GVYYCMQGTHWPFTFGPGTKVDIR | |
| | HCDR1 | 185 | GFSFSDSY | |
| | HCDR2 | 186 | ISSSSSYT | |
| | HCDR3 | 187 | AREGSP | |
| | LCDR1 | 188 | QSLVDSDGNTY | |
| | LCDR2 | 189 | RVS | |
| | LCDR3 | 190 | MQGTHWPFT | |
| h21 | VH | 191 | QVQLVESGGGLVKPGGSLRLSCAASGFTFSAHYMSWIRQAPGK GLEWVSYISPSSSYTVYADSVKGRFTISRDNAKNSLFLQMNSLR AEDTAVYYCAREGSPGGQGTLVTVSS | 1.48E-09 |
| | VL | 192 | DVVMTQSPLSLPVTLGQPASISCRSTQSLVDSDGNTYLSWFQQ RPGQSPRRLIYKVSDRDSGVPDRFSGSGSGTDFTLKISRVEAEDI GIYYCMQGTHWPFTFGPGTKVDIK | |
| | HCDR1 | 193 | GFTFSAHY | |
| | HCDR2 | 194 | ISPSSSYT | |
| | HCDR3 | 195 | AREGSP | |
| | LCDR1 | 196 | QSLVDSDGNTY | |
| | LCDR2 | 197 | KVS | |
| | LCDR3 | 198 | MQGTHWPFT | |

(continued)

| Antibody | Description | SEQ ID NO | Sequence | Affinity ($K_D$) |
|---|---|---|---|---|
| h22 | VH | 199 | QVQLVESGGGLVKPGGSLRLSCAASGFTFSAHYMSWIRQAPGK GLEWVSYISPSSSYTVYADSVKGRFTISRDNAKNSLFLQMNSLR AEDTAVYYCAREGSPGGQGTLVTVSS | 1.04E-10 |
| | VL | 200 | DVVMTQSPLSLPVTLGQPASISCRSSQNLVDSDGNTYLSWFQQ RPGQSPRRLIYKVSDRDSGVPDRFSGSGSGTDFTLKINRVEAEDI GIYYCMQGSHWPFTFGPGTKVDIK | |
| | HCDR1 | 201 | GFTFSAHY | |
| | HCDR2 | 202 | ISPSSSYT | |
| | HCDR3 | 203 | AREGSP | |
| | LCDR1 | 204 | QNLVDSDGNTY | |
| | LCDR2 | 205 | KVS | |
| | LCDR3 | 206 | MQGSHWPFT | |
| h23 | VH | 207 | EVQLVESGGGLVQPGGSLRLSCAASGFTFSSYAMSWVRQAPG KGLEWVSGISGSVDITHHADSVKGRFTISRDNSKNTLYLQMNS LRAEDTAVYYCVRGGFDYWGQGSLVTVSS | 4.90E-10 |
| | VL | 208 | DIQMTQSPSSVSASVGDRVTISCRASQDISTWLAWYQQKPGKA PRLLIYAASTLLIGVPSRFSGSGSGTDFTLTISSLQPEDFATYYCQ QTNSFPITFGQGTRLEIK | |
| | HCDR1 | 209 | GFTFSSYA | |
| | HCDR2 | 210 | ISGSVDIT | |
| | HCDR3 | 211 | VRGGFDY | |
| | LCDR1 | 212 | QDISTW | |
| | LCDR2 | 213 | AAS | |
| | LCDR3 | 214 | QQTNSFPIT | |
| h24 | VH | 215 | EVQLVESGGGLVQPGGSLRLSCAASGFTFSSYAMTWVRQAPG KGLEWVSGISGSIGITHHADSVKGRFTISRDNSKNTLYLQMNSL RAEDTAVYYCVRGGFDYWGQGSLVTVSS | 7.79E-10 |
| | VL | 216 | DIQMTQSPSSVSASVGDRVTITCRASQDINTWLAWYQQKPGKA PKLLIYTSSTLLGGVPSRFSGSGSGTDFTLTISSLQPEDFGTYYCQ QTNTFPITFGQGTRLEIK | |
| | HCDR1 | 217 | GFTFSSYA | |
| | HCDR2 | 218 | ISGSIGIT | |
| | HCDR3 | 219 | VRGGFDY | |
| | LCDR1 | 220 | QDINTW | |
| | LCDR2 | 221 | TSS | |
| | LCDR3 | 222 | QQTNTFPIT | |

(continued)

| Antibody | Description | SEQ ID NO | Sequence | Affinity (K$_D$) |
|---|---|---|---|---|
| h25 | VH | 223 | EVQLVESGGGLVKRGGSLRLSCAASGFTFSSYSMHWVRQAPG KGLEWVSSISSRSTYIYYADSVKGRFTISRDNAKNSLYLQMNSL SAEDTAVYYCARGSGSYYVFDYWGQGTLVTVSS | 3.20E-10 |
| | VL | 224 | ELVMTQSPATLSVSPGERATLSCRASQSVSSNLAWYQQKPGQA PRLLIYGASSRATNIPARFSGSGSGTEFTLTISSLQSEDFAVYYCQ QYDNWPMYSFGQGTKLEIK | |
| | HCDR1 | 225 | GFTFSSYS | |
| | HCDR2 | 226 | ISSRSTYI | |
| | HCDR3 | 227 | ARGSGSYYVFDY | |
| | LCDR1 | 228 | QSVSSN | |
| | LCDR2 | 229 | GAS | |
| | LCDR3 | 230 | QQYDNWPMYS | |
| h26 | VH | 231 | EVQLVESGGGLVQPGGSLRLSCAASGFTFSSYAMTWVRQAPG KGLEWVSGISGSVGITHHADSVKGRFTISRDNSKNTLYLQMNS LRVEDTAVYYCVRGGFDYWGQGSLVTVSS | 4.31E-10 |
| | VL | 232 | DIQMTQSPSSVSASVGDRVTITCRASQDINTWLAWYQQKPGKA PKLLIYTTSTLLSGVPSRFSGSGSGTDFTLTISSLQPEDFATYYCQ QTNTFPITFGQGTRLEIK | |
| | HCDR1 | 233 | GFTFSSYA | |
| | HCDR2 | 234 | ISGSVGIT | |
| | HCDR3 | 235 | VRGGFDY | |
| | LCDR1 | 236 | QDINTW | |
| | LCDR2 | 237 | TTS | |
| | LCDR3 | 238 | QQTNTFPIT | |
| h27 | VH | 239 | EVQLVESGGGLVKPGGSLRLSCAASGFTFSSYSMSWVRQAPGK GLEWVSSISSRSNYIYYADSVKGRITISRDNAKNSLYLQMNSLR AEDTAVYYCARGSGSYYVFDYWGQGTLVTVSS | 1.60E-10 |
| | VL | 240 | ELVMTQSPATLSMSPGDRATLSCRASQSIRSDLAWYQQKPGQA PRLLIYDASTRATGIPARFSGSGSGTEFTLSISSLQSEDFAVYYCQ QYNNWPMYTFGQGTKLEIK | |
| | HCDR1 | 241 | GFTFSSYS | |
| | HCDR2 | 242 | ISSRSNYI | |
| | HCDR3 | 243 | ARGSGSYYVFDY | |
| | LCDR1 | 244 | QSIRSD | |
| | LCDR2 | 245 | DAS | |
| | LCDR3 | 246 | QQYNNWPMYT | |

Table 7. human antibodies against Zzs05

| Anti-body | Description | SEQ ID NO | Sequence | Affinity (K_D) |
|---|---|---|---|---|
| h28 | VH | 247 | QVQLVESGGGVVQPGRSLRLSCAASGFTFSRYGIHWVRQAPGKGLEWVAVITYDGRKKFYADSVQGRFTISRDNSRNTLSLQMNSLRAEDTAVYYCAKDLLIGYGMDVWGQGTTVTVSS | 1.09E-09 |
| | VL | 248 | DIVMTQSPDSLAVSLGERATINCKSSQSVLFSSNNKNFLAWYQQKPGQPPKLLIYWASTRESGVPDRFSGSGSGTDFTLTISSLQPEDVALYFCQQCYSTPYTFGQGTKLEIK | |
| | HCDR1 | 249 | GFTFSRYG | |
| | HCDR2 | 250 | ITYDGRKK | |
| | HCDR3 | 251 | AKDLLIGYGMDV | |
| | LCDR1 | 252 | QSVLFSSNNKNF | |
| | LCDR2 | 253 | WAS | |
| | LCDR3 | 254 | QQCYSTPYT | |
| h29 | VH | 255 | QVQLVESGGGVVQPGRSLTLSCAASGFTFSRYGMHWVRQAPGKGLEWVTVITYDGRKKYYADSVKGRFTISRDNSQNTLSLQMNSLRAEDTAVYYCAKDLLIGYGMDVWGQGTTVTVSS | 1.20E-09 |
| | VL | 256 | DIVMTQSPDSLAVSLGERATIKCKSSQSVFFSSSNKNFLTWYQQKPGQPPKLLIYWASTRESGVPDRFSGSGSGTDFTLTINNLQAEDVTVYYCQQCFSTPYSFGQGTKLEIK | |
| | HCDR1 | 257 | GFTFSRYG | |
| | HCDR2 | 258 | ITYDGRKK | |
| | HCDR3 | 259 | AKDLLIGYGMDV | |
| | LCDR1 | 260 | QSVFFSSSNKNF | |
| | LCDR2 | 261 | WAS | |
| | LCDR3 | 262 | QQCFSTPYS | |
| h30 | VH | 263 | QVQLVESGGGVVQPGRSLRLSCAASGFTFSRYGIHWVRQAPGKGLEWVAVITYDGRKKFYADSVKGRFTISRDNSRNTLSLQMNSLRAEDTAVYYCAKDLLIGYGMDVWGQGTTVTVSS | 8.32E-10 |
| | VL | 264 | DIVMTQSPDSLAVSLGERATINCKSSQSVLFSSNNKNFLAWYQQKPGQPPKLLIYWASTRESGVPDRFSGSGSGTDFTLTISSLQPEDVALYFCQQCYSTPYTFGQGTKLEIK | |
| | HCDR1 | 265 | GFTFSRYG | |
| | HCDR2 | 266 | ITYDGRKK | |
| | HCDR3 | 267 | AKDLLIGYGMDV | |
| | LCDR1 | 268 | QSVLFSSNNKNF | |
| | LCDR2 | 269 | WAS | |
| | LCDR3 | 270 | QQCYSTPYT | |

(continued)

| Anti-body | Description | SEQ ID NO | Sequence | Affinity (K_D) |
|---|---|---|---|---|
| h31 | VH | 271 | QVQLVESGGGVVQPGRSLRLSCAASGFTFSSYGMHWVRQAPGKGLEWVAVISYDGSNKYYADSVKGRFTISKDNSKNTLYLQMNSLRAEDTAVYYCAKDLLAGYGMDVWGQGTTVTVSS | 1.87E-07 |
| | VL | 272 | DIVMTQSPDSLAVSLGERATINCKSSQSVLYSSNNKNFLAWYQQKPGQPPKLLIYWASTRESGVPDRFSGSGSGTDFTLTISSLQAEDVAVYYCQQYYSTPYTFGQGTKLEIK | |
| | HCDR1 | 273 | GFTFSSYG | |
| | HCDR2 | 274 | ISYDGSNK | |
| | HCDR3 | 275 | AKDLLAGYGMDV | |
| | LCDR1 | 276 | QSVLYSSNNKNF | |
| | LCDR2 | 277 | WAS | |
| | LCDR3 | 278 | QQYYSTPYT | |
| h32 | VH | 279 | QVQLVESGGGVVQPGRSLRLSCAASGFTFSSYGMHWVRQAPGKGLEWVTVITYDGSRKFYADSVKGRFTISRDSSKNTLYLQMNSLRVEDTAVYYCAKDHLRGYGMDVWGQGTTVTVSS | 8.17E-10 |
| | VL | 280 | DIVMTQSPDSLAVSLGERATINCKSSQSVLFSSNNKNFLAWYQQKPGQPPVLLIKWASTRYSGVPDRFSGSGSGTDFTLTISSLQAEDVAVYYCQQYYSTPYTFGQGTKLEIK | |
| | HCDR1 | 281 | GFTFSSYG | |
| | HCDR2 | 282 | ITYDGSRK | |
| | HCDR3 | 283 | AKDHLRGYGMDV | |
| | LCDR1 | 284 | QSVLFSSNNKNF | |
| | LCDR2 | 285 | WAS | |
| | LCDR3 | 286 | QQYYSTPYT | |
| h33 | VH | 287 | QVQLVESGGGVVQPGRSLRLSCAASGFTFSRYGIHWVRQAPGKGLEWVAVITYDGRKKYYADSVKGRFTISRDNSQNTLSLQMNSLTTEDTGVYYCAKDLLIGYGMDVWGQGTTVTVSS | 1.04E-09 |
| | VL | 288 | DIVMTQSPDSLAVSLGERATINCKSSQSVLFSSNKKNFLTWYQQKPGQPPKLLIYWASTRESGVPDRFSGSGSGTDFTLTISSLQAEDVAVYYCQQCYSTPYSFGRGTKLEIK | |
| | HCDR1 | 289 | GFTFSRYG | |
| | HCDR2 | 290 | ITYDGRKK | |
| | HCDR3 | 291 | AKDLLIGYGMDV | |
| | LCDR1 | 292 | QSVLFSSNKKNF | |
| | LCDR2 | 293 | WAS | |
| | LCDR3 | 294 | QQCYSTPYS | |

(continued)

| Anti-body | Description | SEQ ID NO | Sequence | Affinity (K_D) |
|---|---|---|---|---|
| h34 | VH | 295 | QVQLVESGGGVVQPGRSLRLSCAASGFTFSRYGIHWVRQAPGKGLEWVAVITYDGRKKFYADSVKGRFTISRDNSRNTLSLQMNSLRAEDTAVYYCAKDLLIGYGLDVWGQGTTVTVSS | 5.74E-10 |
| | VL | 296 | DIVMTQSPDSLAVSLGERATINCKSSQSVLFSSNNKNFLAWYQQKPGQPPKLLIYWASTRESGVPDRFSGSGSGTDFTLTISSLQPEDVALYFCQQCYSTPYSFGQGTKLEIK | |
| | HCDR1 | 297 | GFTFSRYG | |
| | HCDR2 | 298 | ITYDGRKK | |
| | HCDR3 | 299 | AKDLLIGYGLDV | |
| | LCDR1 | 300 | QSVLFSSNNKNF | |
| | LCDR2 | 301 | WAS | |
| | LCDR3 | 302 | QQCYSTPYS | |
| h35 | VH | 303 | QVQLVESGGGVVQPGRSLRLSCAASGFTFSSYGMHWVRQAPGKGLEWVTVITYDGSRKFYADSVKGRFTISRDSSKNTLFLQMNSLRVEDTAVYYCAKDHLRGYGMDVWGQGTTVTVSS | 7.93E-10 |
| | VL | 304 | DIVMTQSPDSLAVSLGERATINCKSSQSVLFSSNNKNFLAWYQQKPGQPPVLLIKWASTRYSGVPDRFSGSGSGTDFTLTISSLQAEDVAVYYCQQYYSTPYTFGQGTKLEIK | |
| | HCDR1 | 305 | GFTFSSYG | |
| | HCDR2 | 306 | ITYDGSRK | |
| | HCDR3 | 307 | AKDHLRGYGMDV | |
| | LCDR1 | 308 | QSVLFSSNNKNF | |
| | LCDR2 | 309 | WAS | |
| | LCDR3 | 310 | QQYYSTPYT | |
| | VH | 311 | QVQLVESGGGVVQPGRSLRLSCAASGFTFSSYGMHWVRQAPGKGLEWVAVITYDGSKKYYADSVKGRFTISRDNSKNTLYLQMNSLRAEDTAVYYCAKDRAVGYGMDAWGQGTTVTVSS | |
| | VL | 312 | DIVMTQSPDSLAVSLGERATINCKSSQSVLFSSNNKNFLAWYQQKSGQPPKQLIFWASIRESGVPDRFSGSGSGTDFTLTISSLQAEDVAVYSCQQYYSTPYTFGQGTKLEIK | |
| | HCDR1 | 313 | GFTFSSYG | |
| | HCDR2 | 314 | ITYDGSKK | |
| | HCDR3 | 315 | AKDRAVGYGMDA | |
| | LCDR1 | 316 | QSVLFSSNNKNF | |
| | LCDR2 | 317 | WAS | |
| | LCDR3 | 318 | QQYYSTPYT | |
| h37 | VH | 319 | QVQLVESGGGVVQPGRSLRLSCAASGFTFSSYGMHWVRQAPGKGLEWVAVITYDGSKKYYADSVKGRFTISKDNSKNTLYLQMNSLRAEDTAVFYCAKDRALGYGMDVWGQGTTVTVSS | 6.25E-09 |

(continued)

| Anti-body | Description | SEQ ID NO | Sequence | Affinity (K$_D$) |
|---|---|---|---|---|
| | VL | 320 | DIVMTQSPDSLAVSLGERATINCKSSQTVLFSSNNKNFLTWYQ QKSGQPPKQIIYWASTRESGVPDRFSGSGSGTDFTLTINSLQAE DVAVYYCQQYFSTPYTFGQGTKLEIK | |
| | HCDR1 | 321 | GFTFSSYG | |
| | HCDR2 | 322 | ITYDGSKK | |
| | HCDR3 | 323 | AKDRALGYGMDV | |
| | LCDR1 | 324 | QTVLFSSNNKNF | |
| | LCDR2 | 325 | WAS | |
| | LCDR3 | 326 | QQYFSTPYT | |
| h38 | VH | 327 | QVQLVESGGGVVQPGRSLRLSCAASGFTFNSYGMHWVRQAP GKGLEWVAVISKDGSKKYYADSVKGRFTISRDKSKNTLYLQ MNSLRAEDTAVYYCAKDRALGYGMDVWGQGTTVTVSS | 1.72E-09 |
| | VL | 328 | DIVMTQSPDSLAVSLGERATINCKSSQSVLYSVNNKNFLTWY QQKPGQPPKQLIYWASTRESGVPDRFSGSGSGTDFTLTISSLQ AEDVAVYYCQHYYSTPYTFGQGTKLEIK | |
| | HCDR1 | 329 | GFTFNSYG | |
| | HCDR2 | 330 | ISKDGSKK | |
| | HCDR3 | 331 | AKDRALGYGMDV | |
| | LCDR1 | 332 | QSVLYSVNNKNF | |
| | LCDR2 | 333 | WAS | |
| | LCDR3 | 334 | QHYYSTPYT | |
| h39 | VH | 335 | QVQLVESGGGVVQPGRSLRLSCAASGFTFSSYGMHWVRQAP GKGLEWVAVITYDGSKKYYADSVKGRFTISKDNSKNTLYLQ MNSLRAEDTAVFYCAKDRALGYGMDVWGQGTTVTVSS | 3.57E-09 |
| | VL | 336 | DIVMTQSPDSLAVSLGERATINCKSSQTVLFSSNNKNFLTWYQ QKSGQPPKQIIYWASTRESGVPDRFSGSGSGTDFTLTISSLQAE DVAVYYCQQYFSTPYTFGQGTKLEIK | |
| | HCDR1 | 337 | GFTFSSYG | |
| | HCDR2 | 338 | ITYDGSKK | |
| | HCDR3 | 339 | AKDRALGYGMDV | |
| | LCDR1 | 340 | QTVLFSSNNKNF | |
| | LCDR2 | 341 | WAS | |
| | LCDR3 | 342 | QQYFSTPYT | |

(continued)

| Anti-body | Description | SEQ ID NO | Sequence | Affinity (K$_D$) |
|---|---|---|---|---|
| h40 | VH | 343 | QVQLVESGGGVVQPGRSLRLSCAASGFTFNSYGMHWVRQAPGKGLEWVAVISKDGSKKYYADSVKGRFTISRDKSKNTLYLQMNSLRAEDTAVYYCAKDRALGYGMDVWGQGTTVTVSS | 2.59E-09 |
| | VL | 344 | DIVMTQSPDSLAVSLGERATINCKSSQSVLYSVNNKNFLTWYQQKPGQPPKQLIYWASTRESGVPDRFSGSGSGTDFSLTISGLQAEDVAVYYCQHYYSAPYTFGQGTKLEIK | |
| | HCDR1 | 345 | GFTFNSYG | |
| | HCDR2 | 346 | ISKDGSKK | |
| | HCDR3 | 347 | AKDRALGYGMDV | |
| | LCDR1 | 348 | QSVLYSVNNKN | |
| | LCDR2 | 349 | WAS | |
| | LCDR3 | 350 | QHYYSAPYT | |
| h41 | VH | 351 | QVQLVESGGGVVQPGRSLRLSCVASGFTFSSYGMHWVRQAPGKGLEWVAVITYDGSKKYYADSVKGRFTISRDSSKNTLYLQMNSLRAEDTAVYYCAKDRAVGYGMDVWGQGTTVTVSS | 3.00E-09 |
| | VL | 352 | DIVMTQSPDSLAVSLGERATINCKSSQSVLYRSNNKNFLTWYQQKSGQSPKQLIYWASTRESGVPDRFSGSGSGTDFTLTISSLQAEDVAVYYCQQYYSTPYTFGQGTKLEIK | |
| | HCDR1 | 353 | GFTFSSYG | |
| | HCDR2 | 354 | ITYDGSKK | |
| | HCDR3 | 355 | AKDRAVGYGMDV | |
| | LCDR1 | 356 | QSVLYRSNNKNF | |
| | LCDR2 | 357 | WAS | |
| | LCDR3 | 358 | QQYYSTPYT | |
| h42 | VH | 359 | QVQLVESGGGVVQPGRSLRLSCAASGFTFSRYGIHWVRQAPGKGLEWVAVISYHGSNKYYADSVKGRFTISRDNSKNTLYLQMNSLRAEDTAVYYCAKDRALGYGMDVWGQGTTVTVSS | 2.98E-09 |
| | VL | 360 | DIVVTQSPDSLAVSLGERATINCKSSQSVLFNSNNKNFLTWYQQKPGQPPKQLIYWASTRESGVPDRFSGSGSGTDFTLTISSLQAEDVAVYYCQQYYSTPYAFGQGTKLEIK | |
| | HCDR1 | 361 | GFTFSRYG | |
| | HCDR2 | 362 | ISYHGSNK | |
| | HCDR3 | 363 | AKDRALGYGMDV | |
| | LCDR1 | 364 | QSVLFNSNNKNF | |
| | LCDR2 | 365 | WAS | |
| | LCDR3 | 366 | QQYYSTPYA | |

(continued)

| Anti-body | Description | SEQ ID NO | Sequence | Affinity (K_D) |
|---|---|---|---|---|
| h43 | VH | 367 | QVQLVESGGGAVQPGRSLRLSCAASGFTFSRYGMHWVRQAP GKGLEWVAVITYDGSKKYYADSVKGRFTISRDNSKNTLSLQ MNSLRAEDTAVYYCAKDRALGYGMDVWGQGTTVTVSS | 5.42E-09 |
| | VL | 368 | DIVMTQSPDSLAVSLGERATINCKSSQSVLYTANNKNFLTWY QQKSGQPPKQLIYWASTRESGVPDRFSGSGSGTDFTLTISSLQ AEDVAVYYCQQYYSTPYTFGQGTKLEIK | |
| | HCDR1 | 369 | GFTFSRYG | |
| | HCDR2 | 370 | ITYDGSKK | |
| | HCDR3 | 371 | AKDRALGYGMDV | |
| | LCDR1 | 372 | QSVLYTANNKNF | |
| | LCDR2 | 373 | WAS | |
| | LCDR3 | 374 | QQYYSTPYT | |
| h44 | VH | 375 | QVQLVESGGGVVQPGRSLRLSCVASGFTFSSYGMHWVRQAP GKGLEWVAVITYDGSKKYYADSVKGRFTISRDSSKNTLYLQ MNSLRAEDTAVYYCAKDRAVGYGMDVWGQGTTVTVSS | 3.48E-09 |
| | VL | 376 | DIVMTQSPDSLAVSLGERATINCKSSQSVLYRSNNKNFLTWY QQKSGQSPKQLIYWASTRESGVPDRFSGSGSGTDFTLTISSLQ AEDVAVYYCQQYYSTPYTFGQGTKLEIK | |
| | HCDR1 | 377 | GFTFSSYG | |
| | HCDR2 | 378 | ITYDGSKK | |
| | HCDR3 | 379 | AKDRAVGYGMDV | |
| | LCDR1 | 380 | QSVLYRSNNKN | |
| | LCDR2 | 381 | WAS | |
| | LCDR3 | 382 | QQYYSTPYT | |
| h45 | VH | 383 | QVQLVESGGGVVQPGRSLRLSCAASGFTFSSYGMHWVRQAP GKGLEWVAVISYDGSKKYYADSVKGRFTISRDISKNTLYLQM NSLRAEDTAVYYCAKDRALGYGMDVWGQGTTVTVSS | 5.56E-09 |
| | VL | 384 | DIVMTQSPDSLAVSLGERATINCKSSQSVLYSANNKNFLTWY QQKPGQPPKQLIYWASTRESGVPDRFSGSGSGTDFTLIISSLQA EDVAVYYCQQYYSTPYTFGQGTKLEIK | |
| | HCDR1 | 385 | GFTFSSYG | |
| | HCDR2 | 386 | ISYDGSKK | |
| | HCDR3 | 387 | AKDRALGYGMDV | |
| | LCDR1 | 388 | QSVLYSANNKN | |
| | LCDR2 | 389 | WAS | |
| | LCDR3 | 390 | QQYYSTPYT | |

(continued)

| Anti-body | Description | SEQ ID NO | Sequence | Affinity (K_D) |
|---|---|---|---|---|
| h46 | VH | 391 | QVQLVESGGGVVQPGRSLRLSCAASGFTFSRYGIHWVRQAPGKGLEWVAVISYHGSNKYYADSVKGRFTISRDNSKNTLYLQMNSLRAEDTAVYYCAKDRALGYGMDVWGQGTTVTVSS | 3.95E-09 |
| | VL | 392 | DIVVTQSPDSLAVSLGERATINCKSSQSVLFNSNNKNFLTWYQQKPGQPPKQLIYWASTRESGVPDRFSGSGSGTDFTLTISSLQAEDVAIYYCQQYYSTPYAFGQGTKLEIK | |
| | HCDR1 | 393 | GFTFSRYG | |
| | HCDR2 | 394 | ISYHGSNK | |
| | HCDR3 | 395 | AKDRALGYGMDV | |
| | LCDR1 | 396 | QSVLFNSNNKNF | |
| | LCDR2 | 397 | WAS | |
| | LCDR3 | 398 | QQYYSTPYA | |
| h47 | VH | 399 | QVQLVESGGGVVQPGRSLRLSCAASGFTFSSYGMHWVRQAPGKGLEWVAVISYDGSKKYYADSVKGRFTISRDISKNTLYLQMNSLRAEDTAVYYCAKDRALGYGMDVWGQGTTVTVSS | 5.55E-09 |
| | VL | 400 | DIVMTQSPDSLAVSLGERATINCKSSQSVLYSANNKNFLTWYQQKPGQPPKQLIYWASTRESGVPDRFSGSGSGTDFTLIISSLQAEDVAVYYCQQYYSTPYTFGQGTKLEIK | |
| | HCDR1 | 401 | GFTFSSYG | |
| | HCDR2 | 402 | ISYDGSKK | |
| | HCDR3 | 403 | AKDRALGYGMDV | |
| | LCDR1 | 404 | QSVLYSANNKN | |
| | LCDR2 | 405 | WAS | |
| | LCDR3 | 406 | QQYYSTPYT | |
| h48 | VH | 407 | QVQLVESGGGVVQPGRSLRLSCAASGFTFNSYGMHWVRQAPGKGLEWVAVISKDGSKKYYADSVKGRFTISRDKSKNTLYLQMNSLRAEDTAVYYCAKDRALGYGMDVWGQGTTVTVSS | 3.63E-09 |
| | VL | 408 | DIVMTQSPDSLAVSLGERATINCKSSQSVLYSVNNKNFLTWYQQKPGQPPKQLIYWASTRESGVPDRFSGSGSGTDFTLTISSLQAEDVAVYYCQHYYSTPYTFGQGTKLEIK | |
| | HCDR1 | 409 | GFTFNSYG | |
| | HCDR2 | 410 | ISKDGSKK | |
| | HCDR3 | 411 | AKDRALGYGMDV | |
| | LCDR1 | 412 | QSVLYSVNNKN | |
| | LCDR2 | 413 | WAS | |
| | LCDR3 | 414 | QHYYSTPYT | |

The corresponding heavy chain constant region and light chain constant region sequences are as follows:

Heavy chain constant region (SEQ ID NO: 417):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGK

Light chain constant region (SEQ ID NO: 418):

RTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

**Example 6: Human Monoclonal Antibody-Mediated ADCC Effect on Tumor Organoid Cells**

6.1 The human anti-ZZS05 antibody h32(also named A-1P13) mediated ADCC effect by NK cells on tumor organoid cells of pancreatic cancer and small cell lung cancer (Beijing Big Run Technology Co., Ltd.).

Experimental Cells:

Organoids: pancreatic cancer organoid DXPC-ZY- 001(from stage III patients with medium and low differentiated pancreatic cancer), small cell lung cancer organoid DXSCLC-ZY-001(from stage IV patients with low differentiated small cell lung cancer)
NK cells: commercially available PBMC expanded NK cells DXNK-ZY- 001

Experimental Antibodies:

Human anti-ZZS05 antibody A-1P13(Experimental Group)
IgG1 (H7N9(A/Anhui/2013)) of Humanized Antibody against Influenza A Protein HA (Control)

Summary of Experiments

A co-culture model of tumor organoids and immune cells was constructed based on a 384-well plate. The cultured tumor organoids were taken and digested into single cells, followed by live cell tracer staining (Calcein, AM), the organoid tumor cells and the A-1P13 to be tested were incubated together for 20 minutes, NK cells and tumor organoid cells were co-cultured according to an effect-target ratio of 5:1, and after incubation for 5 hours, cell tracer staining (SYTOX-Red) was performed, and the cells were detected by an on-board flow cytometer.

Experimental Grouping:

| No. | Group | Drug | Drug concentration (μg/ml) | Effect-target ratio | Biological repeat | Co-culture time |
|---|---|---|---|---|---|---|
| 1 | Organoid only | PBS | NA | 5:1 | 3 | 5h |
| 2 | NK+organoid | PBS | NA | | | |
| 3 | NK+organoid | IgG1 | 1 | | | |
| 4 | NK+organoid | A-1P13 | 10 | | | |
| 5 | NK+organoid | A-1P13 | 1 | | | |
| 6 | NK+organoid | A-1P13 | 0.1 | | | |

Experimental Results:

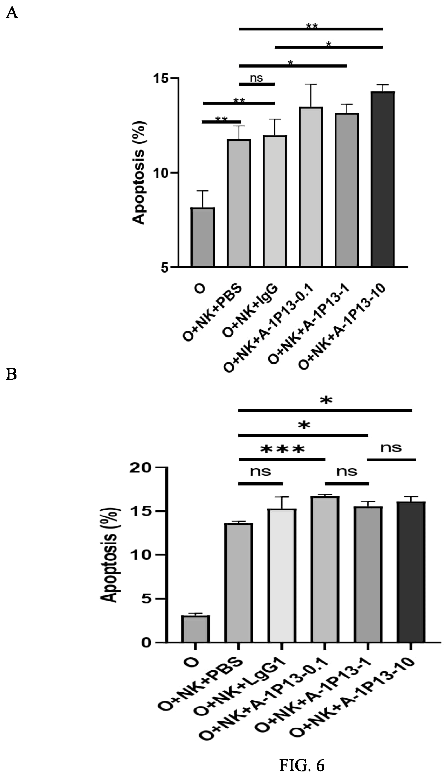

The results of pancreatic cancer organoids are shown in FIG. 6A. The apoptosis of tumor organoids in the ADCC model was statistically analyzed. T Test "*":P<0.05,"**":P<0.01. Compared with the organoid only group (O), the organoid apoptosis in the O+NK+PBS/IgG group was significantly increased, and there was no significant difference between the O+NK+PBS and O+NK+IgG groups. Compared with O+NK+PBS and O+NK+IgG, the increase of organoid apoptosis in the 10μg/mL dosing group was most significant. There was no significant difference between the dosing groups.

The results of small cell lung cancer organoids are shown in FIG. 6B. The apoptosis of tumor organoids in the ADCC model was statistically analyzed. T Test "*":P<0.05,"**":P<0.01. Compared with the O+NK+PBS group, the organoid apoptosis in the A-1P13 group was significantly increased, and there was no significant difference between the O+NK+PBS group and the O+NK+IgG group. Compared with O+NK+PBS, the increase of organoid apoptosis in the 0.1 μg/mL dosing group was most significant. There was no significant difference between the dosing groups.

6.2 The effect of human anti- ZZS05 antibody h35(also named as A-1P22) on different tumor organoids (Shanghai Cedyi Biotechnology Co., Ltd.).

**Experimental Design:**

1) Construction of colorectal cancer (CRC), lung cancer (LC), gastric cancer (GC), endometrial cancer (EC), breast cancer (BC), pancreatic cancer (PADC), liver cancer (HCC), ovarian cancer (AOC) organoids;
2) After the organoids were constructed, they were divided into organoid+PBS group, organoid+NK cell group, organoid+NK cell+candidate antibody (h35) group, organoid+NK cell+tumor-independent antibody (rabbit anti-human PSA antibody) group;
3) Co-culture of organoids and candidate antibodies and NK cells: The organoids were cultured in 96-well plates and divided into organoid+PBS group, organoid+NK cell group, organoid+NK cells+candidate antibody group, organoid+NK cells+tumor-independent antibody group. In vivo cell imaging, the growth of organoids was continuously photographed for bright field images; after 48 hours of continuous administration, the cells were stained for living and death, photographed; the organoid activity and LDH release were detected;
4) Three wells for each group were biological repeats.

**Test Method:**

Primary Organoid Culture

(1) The obtained cancer tissues must be washed at 2-8° C; several culture dishes were prepared, and a primary culture buffer pre-cooled at 4° C was added for later use (dual antibodies and gentamicin are added).
(2) The tissues were placed in a culture dish, washed three times with primary culture buffer B to remove impurities, and the cancer tissues were gently pressed with an ophthalmic shear or scalpel to remove the red blood cells and cut into tissue blocks with a volume of about 1-3 mm$^3$.
(3) The tissues were digested with primary tissue digestion solution C and subjected to shaking digestion at 37° C for 25-30 min (the digestion was observed at any time during the digestion).

(4) A small amount of liquid was taken and observed under a microscope; when more crypts were observed under the microscope, three times the volume of primary culture buffer was added to terminate digestion.

(5) After filtration with a filter of 100μm pore size, the filtrate was collected, concentrated and centrifuged at 300g for 5 min, the supernatant was removed, and the primary culture buffer B was added for resuspension and centrifugation.

(6) Matrix gel calculation: the tissue volume collected after step 6 was observed, and 40 times of the tissue volume was added to resuspend.

(7) Taking a 24 well cell culture plate as an example, 25 ul of tissue matrix gel mixture was dispensed per well for plating (operated at 4° C.).

(8) The plated culture plate was placed in an incubator at 37° C for 10-15 min, and an intestinal cancer organoid culture medium A (restored to room temperature) was added for culture.

Organoid passage

(1) The culture medium was pipetted off, and 1-2 ml of 4° C organoid passage buffer G was added to each well for 2 min.

(2) The matrix gel was gently blown by a pipette, collected in a 15 ml centrifuge tube, and allowed to stand at 4°C for 10 min. (Every 6-8 wells was one group) After 300 g centrifugation for 5 min, the supernatant was discarded , 2 ml of organoid passage digestion solution D was added for digestion for 3 min, organoid passage culture buffer G was added to terminate digestion, and the liquid was discarded by centrifugation for 5 min at 300g for step 4.

(3) After organoids were collected, matrix gel was added for resuspension, 30μl of matrix gel mixture per well was plated in a 24-well cell culture plate, and 500μl of organoid medium A was added in an incubator for 10-15 min.

Co-culturing organoids with candidate antibodies and NK cells

(1) The organoids were incubated with an organoid medium containing 200 ng/mL IFNγ for 24 h.

(2) The culture medium was pipetted off, and 1-2 ml of 4°C organoid passage buffer G was added to each well for 2 min.

(3) The matrix glue was gently blown by pipetting, collected in a 15 ml centrifuge tube, and stood at 4°C for 10 min. (Every 6-8 well was a group).

(4) After collected, the organoids were resuspended with the NK cell culture medium , and 50 ul of the organoids mixture was added to a 96-well cell culture plate (the number of organoids was preferably 10-20 per well). Note: organoids were resuspended with PBS in organoid blank group.

(5) The collected NK cells were co-incubated with candidate antibodies (5 ug/ml) and tumor-independent antibodies (5 ug/ml), and supplemented with NK cell culture medium to 100 ul, and the ratio of NK cells to organoids was 500:1.

(6) High content continuous photographing for 48h.

(7) The cells were stained and photographed according to the instructions of the kit.

(8) LDH (lactate dehydrogenase) release was detected.

**Experimental Results:**

The video results of continuous photographing of organoids co-cultured with candidate antibodies and NK cells:

Compared with the organoid+PBS group, the organoid+NK group, and the organoid+NK+candidate antibody group, after 48 h, the NK cell recognition in the following groups was increased: AOC: ovarian cancer organoid; BC breast cancer organoid; CRC colorectal cancer organoid; EC: endometrial cancer organoid; GC gastric cancer organoid; HCC liver cancer organoid; LC lung cancer organoid; PADC: pancreatic cancer organoid.

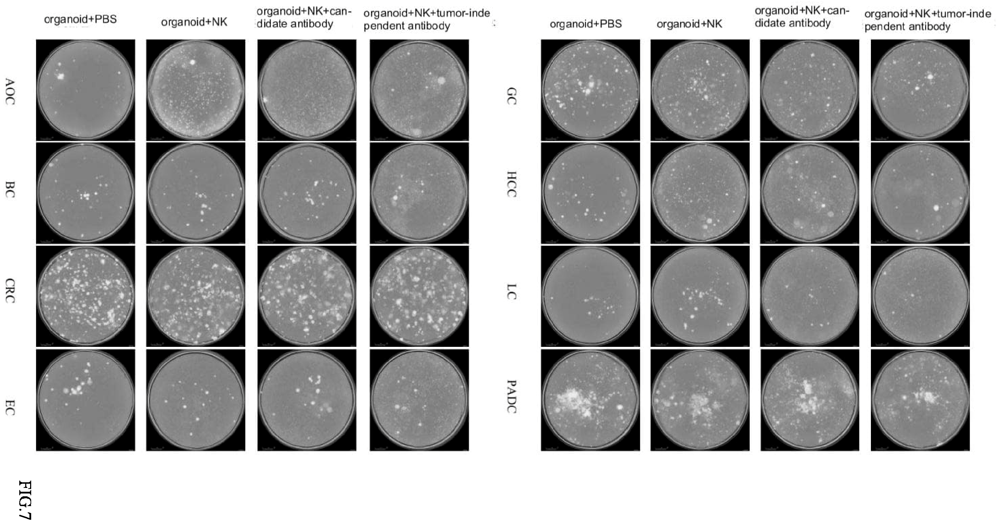

The results of fluorescence staining of organoids co-cultured with candidate antibodies and NK cells (FIG. 7):

Compared with the organoid+PBS group and the organoid+NK group, 48h later, in the organoid+NK+candidate antibody group, the organoid cell apoptosis of the following increased: AOC: ovarian cancer organoid; BC breast cancer organoid; EC: endometrial cancer organoid; HCC liver cancer organoid; LC lung cancer organoid; PADC: pancreatic cancer organoid.

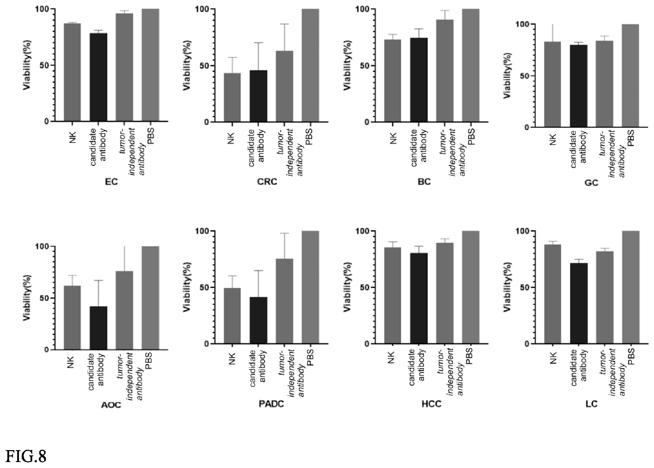

LDH Release Results (FIG. 8):

EC: endometrial cancer organoid

Compared with the organoid+PBS group; the organoid+NK group, the organoid+NK+candidate antibody group had significantly increased LDH release after 48h, and the organoid+NK+tumor-independent antibody group had no significant change; compared with the organoid+NK group, the organoid+NK+candidate antibody group had significantly increased LDH release after 48h.

CRC: colorectal cancer organoid

Compared with the organoid+PBS group; the organoid+NK group, the organoid+NK+candidate antibody group had significantly increased LDH release after 48h, and the organoid+NK+tumor-independent antibody had no significant change; compared with the organoid+NK group, the organoid+NK+candidate antibody group had no significant change in LDH release after 48h n.

BC: breast cancer organoid

Compared with the organoid+PBS group; the organoid+NK group, the organoid+NK+candidate antibody group had significantly increased LDH release after 48h, and the organoid+NK+tumor-independent antibody had no significant change; compared with the organoid+NK group, the organoid+NK+candidate antibody group had no significant change in LDH release after 48h.

GC: gastric cancer organoids

Compared with the organoid+PBS group; the organoid+NK group, the organoid+NK+candidate antibody group, the organoid+NK+tumor independent antibody group had no significant change in LDH release after 48h of action; compared with the organoid+NK group, the organoid+NK+candidate antibody group had no significant change in LDH release after 48h.

AOC: ovarian cancer organoid

Compared with the organoid+PBS group, the LDH release of the organoid+NK+candidate antibody group was significantly increased after 48h, and there was no significant change in the LDH release of the organoid+NK group and the organoid+NK+tumor-independent antibody; compared with the organoid+NK group, the LDH release of the organoid+NK+candidate antibody group was not significantly changed after 48h.

PADC: pancreatic cancer organoids

Compared with the organoid+PBS group, the organoid+NK group, the organoid+NK+candidate antibody group had significantly increased LDH release after 48h, and the organoid+NK+tumor-independent antibody had no significant change; compared with the organoid+NK group, the organoid+NK+candidate antibody group had no significant change in LDH release after 48h.

HCC: liver cancer organoids

Compared with the organoid+PBS group, the organoid+NK group, the organoid+NK+candidate antibody group, the organoid+NK+tumor independent antibody group had significantly increased LDH release after 48h; compared with the organoid+NK group, the organoid+NK+candidate antibody group had no significant change in LDH release after 48h.

LC: lung cancer organoid

Compared with the organoid+PBS group, the organoid+NK group, the organoid+NK+candidate antibody group, the organoid+NK+tumor independent antibody group significantly increased the LDH release after 48h; compared with the organoid+NK group, the organoid+NK+candidate antibody group significantly increased the LDH release after 48h.

**References:**

**EP 4 752 156 A1**

1. Chothia C, Lesk a M. Canonical structures for the hypervariable regions of immunoglobulins. J Mol Biol. (1987) 196:901-17. doi: 10.1016/0022-2836(87)90412-8

2. Lefranc, M.P. IMGT, the international ImMunoGeneTics information system: A standardized approach for immunogenetics and immunoinformatics. Immunome Res 2005, 1, 3.

3. Lefranc, M.P.; Giudicelli, V.; Kaas, Q.; Duprat, E.; Jabado-Michaloud, J.; Scaviner, D.; Ginestoux, C.; Clement, O.; Chaume, D.; Lefranc, G. IMGT, the international ImMunoGeneTics information system. Nucleic Acids Res. 2005, 33, D593-D597.

4. David Prihoda, Jad Maamaryc, Andrew Waightd, Veronica Juand, Laurence Fayadat-Dilmand, Daniel Svozil a,e, and Danny A. Bitton :BioPhi: A platform for antibody design, humanization, and humanness evaluation based on natural antibody repertoires and deep learning.

5. Collaborative Computational Project No. 4(CCP4)-Software for Macromolecular X-Ray Crystallography.

6. A free and open-source software suite for high-performance molecular dynamics and output analysis.

**Claims**

1. An antibody or antigen-binding fragment thereof, wherein the antibody i) specifically binds to the extracellular region of the cTAGE5 protein comprising an amino acid sequence shown in SEQ ID NO: 1 or 2, or ii) specifically binds to the immunogenic polypeptide comprising the amino acid sequence shown in SEQ ID NO: 1 or 2.

2. The antibody or antigen-binding fragment thereof of claim 1, wherein the antibody is a polyclonal antibody, or the antibody is a monoclonal antibody.

3. The antibody or antigen-binding fragment thereof according to claim 1, wherein the antibody comprises a heavy chain variable region and a light chain variable region, and comprises heavy chain CDRs and light chain CDRs selected from any one of the following groups:

| Group | Description | SEQ ID NO: |
|---|---|---|
| 1 | HCDR1 | 7 |
| | HCDR2 | 8 |
| | HCDR3 | 9 |
| | LCDR1 | 10 |
| | LCDR2 | 11 |
| | LCDR3 | 12 |
| 2 | HCDR1 | 17 |
| | HCDR2 | 18 |
| | HCDR3 | 19 |
| | LCDR1 | 20 |
| | LCDR2 | 21 |
| | LCDR3 | 22 |
| 3 | HCDR1 | 33 |
| | HCDR2 | 34 |
| | HCDR3 | 35 |
| | LCDR1 | 36 |
| | LCDR2 | 37 |
| | LCDR3 | 38 |

(continued)

| Group | Description | SEQ ID NO: |
|---|---|---|
| 4 | HCDR1 | 41 |
| | HCDR2 | 42 |
| | HCDR3 | 43 |
| | LCDR1 | 44 |
| | LCDR2 | 45 |
| | LCDR3 | 46 |
| 5 | HCDR1 | 49 |
| | HCDR2 | 50 |
| | HCDR3 | 51 |
| | LCDR1 | 52 |
| | LCDR2 | 53 |
| | LCDR3 | 54 |
| 6 | HCDR1 | 57 |
| | HCDR2 | 58 |
| | HCDR3 | 59 |
| | LCDR1 | 60 |
| | LCDR2 | 61 |
| | LCDR3 | 62 |
| 7 | HCDR1 | 65 |
| | HCDR2 | 66 |
| | HCDR3 | 67 |
| | LCDR1 | 68 |
| | LCDR2 | 69 |
| | LCDR3 | 70 |
| 8 | HCDR1 | 73 |
| | HCDR2 | 74 |
| | HCDR3 | 75 |
| | LCDR1 | 76 |
| | LCDR2 | 77 |
| | LCDR3 | 78 |
| 9 | HCDR1 | 81 |
| | HCDR2 | 82 |
| | HCDR3 | 83 |
| | LCDR1 | 84 |
| | LCDR2 | 85 |
| | LCDR3 | 86 |

(continued)

| Group | Description | SEQ ID NO: |
|---|---|---|
| 10 | HCDR1 | 89 |
| | HCDR2 | 90 |
| | HCDR3 | 91 |
| | LCDR1 | 92 |
| | LCDR2 | 93 |
| | LCDR3 | 94 |
| 11 | HCDR1 | 97 |
| | HCDR2 | 98 |
| | HCDR3 | 99 |
| | LCDR1 | 100 |
| | LCDR2 | 101 |
| | LCDR3 | 102 |
| 12 | HCDR1 | 105 |
| | HCDR2 | 106 |
| | HCDR3 | 107 |
| | LCDR1 | 108 |
| | LCDR2 | 109 |
| | LCDR3 | 110 |
| 13 | HCDR1 | 113 |
| | HCDR2 | 114 |
| | HCDR3 | 115 |
| | LCDR1 | 116 |
| | LCDR2 | 117 |
| | LCDR3 | 118 |
| 14 | HCDR1 | 121 |
| | HCDR2 | 122 |
| | HCDR3 | 123 |
| | LCDR1 | 124 |
| | LCDR2 | 125 |
| | LCDR3 | 126 |
| 15 | HCDR1 | 129 |
| | HCDR2 | 130 |
| | HCDR3 | 131 |
| | LCDR1 | 132 |
| | LCDR2 | 133 |
| | LCDR3 | 134 |

(continued)

| Group | Description | SEQ ID NO: |
|---|---|---|
| 16 | HCDR1 | 137 |
| | HCDR2 | 138 |
| | HCDR3 | 139 |
| | LCDR1 | 140 |
| | LCDR2 | 141 |
| | LCDR3 | 142 |
| 17 | HCDR1 | 145 |
| | HCDR2 | 146 |
| | HCDR3 | 147 |
| | LCDR1 | 148 |
| | LCDR2 | 149 |
| | LCDR3 | 150 |
| 18 | HCDR1 | 153 |
| | HCDR2 | 154 |
| | HCDR3 | 155 |
| | LCDR1 | 156 |
| | LCDR2 | 157 |
| | LCDR3 | 158 |
| 19 | HCDR1 | 161 |
| | HCDR2 | 162 |
| | HCDR3 | 163 |
| | LCDR1 | 164 |
| | LCDR2 | 165 |
| | LCDR3 | 166 |
| 20 | HCDR1 | 169 |
| | HCDR2 | 170 |
| | HCDR3 | 171 |
| | LCDR1 | 172 |
| | LCDR2 | 173 |
| | LCDR3 | 174 |
| 21 | HCDR1 | 177 |
| | HCDR2 | 178 |
| | HCDR3 | 179 |
| | LCDR1 | 180 |
| | LCDR2 | 181 |
| | LCDR3 | 182 |

(continued)

| Group | Description | SEQ ID NO: |
|---|---|---|
| 22 | HCDR1 | 185 |
| | HCDR2 | 186 |
| | HCDR3 | 187 |
| | LCDR1 | 188 |
| | LCDR2 | 189 |
| | LCDR3 | 190 |
| 23 | HCDR1 | 193 |
| | HCDR2 | 194 |
| | HCDR3 | 195 |
| | LCDR1 | 196 |
| | LCDR2 | 197 |
| | LCDR3 | 198 |
| 24 | HCDR1 | 201 |
| | HCDR2 | 202 |
| | HCDR3 | 203 |
| | LCDR1 | 204 |
| | LCDR2 | 205 |
| | LCDR3 | 206 |
| 25 | HCDR1 | 209 |
| | HCDR2 | 210 |
| | HCDR3 | 211 |
| | LCDR1 | 212 |
| | LCDR2 | 213 |
| | LCDR3 | 214 |
| 26 | HCDR1 | 217 |
| | HCDR2 | 218 |
| | HCDR3 | 219 |
| | LCDR1 | 220 |
| | LCDR2 | 221 |
| | LCDR3 | 222 |
| 27 | HCDR1 | 225 |
| | HCDR2 | 226 |
| | HCDR3 | 227 |
| | LCDR1 | 228 |
| | LCDR2 | 229 |
| | LCDR3 | 230 |

(continued)

| Group | Description | SEQ ID NO: |
|---|---|---|
| 28 | HCDR1 | 233 |
| | HCDR2 | 234 |
| | HCDR3 | 235 |
| | LCDR1 | 236 |
| | LCDR2 | 237 |
| | LCDR3 | 238 |
| 29 | HCDR1 | 241 |
| | HCDR2 | 242 |
| | HCDR3 | 243 |
| | LCDR1 | 244 |
| | LCDR2 | 245 |
| | LCDR3 | 246 |
| 30 | HCDR1 | 249 |
| | HCDR2 | 250 |
| | HCDR3 | 251 |
| | LCDR1 | 252 |
| | LCDR2 | 253 |
| | LCDR3 | 254 |
| 31 | HCDR1 | 257 |
| | HCDR2 | 258 |
| | HCDR3 | 259 |
| | LCDR1 | 260 |
| | LCDR2 | 261 |
| | LCDR3 | 262 |
| 32 | HCDR1 | 265 |
| | HCDR2 | 266 |
| | HCDR3 | 267 |
| | LCDR1 | 268 |
| | LCDR2 | 269 |
| | LCDR3 | 270 |
| 33 | HCDR1 | 273 |
| | HCDR2 | 274 |
| | HCDR3 | 275 |
| | LCDR1 | 276 |
| | LCDR2 | 277 |
| | LCDR3 | 278 |

(continued)

| Group | Description | SEQ ID NO: |
|---|---|---|
| 34 | HCDR1 | 281 |
| | HCDR2 | 282 |
| | HCDR3 | 283 |
| | LCDR1 | 284 |
| | LCDR2 | 285 |
| | LCDR3 | 286 |
| 35 | HCDR1 | 289 |
| | HCDR2 | 290 |
| | HCDR3 | 291 |
| | LCDR1 | 292 |
| | LCDR2 | 293 |
| | LCDR3 | 294 |
| 36 | HCDR1 | 297 |
| | HCDR2 | 298 |
| | HCDR3 | 299 |
| | LCDR1 | 300 |
| | LCDR2 | 301 |
| | LCDR3 | 302 |
| 37 | HCDR1 | 305 |
| | HCDR2 | 306 |
| | HCDR3 | 307 |
| | LCDR1 | 308 |
| | LCDR2 | 309 |
| | LCDR3 | 310 |
| 38 | HCDR1 | 313 |
| | HCDR2 | 314 |
| | HCDR3 | 315 |
| | LCDR1 | 316 |
| | LCDR2 | 317 |
| | LCDR3 | 318 |
| 39 | HCDR1 | 321 |
| | HCDR2 | 322 |
| | HCDR3 | 323 |
| | LCDR1 | 324 |
| | LCDR2 | 325 |
| | LCDR3 | 326 |

(continued)

| Group | Description | SEQ ID NO: |
|---|---|---|
| 40 | HCDR1 | 329 |
| | HCDR2 | 330 |
| | HCDR3 | 331 |
| | LCDR1 | 332 |
| | LCDR2 | 333 |
| | LCDR3 | 334 |
| 41 | HCDR1 | 337 |
| | HCDR2 | 338 |
| | HCDR3 | 339 |
| | LCDR1 | 340 |
| | LCDR2 | 341 |
| | LCDR3 | 342 |
| 42 | HCDR1 | 345 |
| | HCDR2 | 346 |
| | HCDR3 | 347 |
| | LCDR1 | 348 |
| | LCDR2 | 349 |
| | LCDR3 | 350 |
| 43 | HCDR1 | 353 |
| | HCDR2 | 354 |
| | HCDR3 | 355 |
| | LCDR1 | 356 |
| | LCDR2 | 357 |
| | LCDR3 | 358 |
| 44 | HCDR1 | 361 |
| | HCDR2 | 362 |
| | HCDR3 | 363 |
| | LCDR1 | 364 |
| | LCDR2 | 365 |
| | LCDR3 | 366 |
| 45 | HCDR1 | 369 |
| | HCDR2 | 370 |
| | HCDR3 | 371 |
| | LCDR1 | 372 |
| | LCDR2 | 373 |
| | LCDR3 | 374 |

(continued)

| Group | Description | SEQ ID NO: |
|---|---|---|
| 46 | HCDR1 | 377 |
| | HCDR2 | 378 |
| | HCDR3 | 379 |
| | LCDR1 | 380 |
| | LCDR2 | 381 |
| | LCDR3 | 382 |
| 47 | HCDR1 | 385 |
| | HCDR2 | 386 |
| | HCDR3 | 387 |
| | LCDR1 | 388 |
| | LCDR2 | 389 |
| | LCDR3 | 390 |
| 48 | HCDR1 | 393 |
| | HCDR2 | 394 |
| | HCDR3 | 395 |
| | LCDR1 | 396 |
| | LCDR2 | 397 |
| | LCDR3 | 398 |
| 49 | HCDR1 | 401 |
| | HCDR2 | 402 |
| | HCDR3 | 403 |
| | LCDR1 | 404 |
| | LCDR2 | 405 |
| | LCDR3 | 406 |
| 50 | HCDR1 | 409 |
| | HCDR2 | 410 |
| | HCDR3 | 411 |
| | LCDR1 | 412 |
| | LCDR2 | 413 |
| | LCDR3 | 414 |

4. The antibody or antigen-binding fragment thereof according to claim 3, wherein the antibody comprises a light chain variable region and a heavy chain variable region, and the heavy chain variable region (VH) and the light chain variable region (VL) respectively comprise an amino acid sequence selected from any one of the following groups or respectively comprise an amino acid sequence having at least 70%, at least 75%, at least 80%, at least 85%, at least 90%, at least 95% or higher sequence identity with an amino acid sequence selected from any one of the following groups:

| Group | Description | SEQ ID NO: |
|---|---|---|
| 1 | VH | 5 |
| | VL | 6 |

(continued)

| Group | Description | SEQ ID NO: |
|---|---|---|
| 2 | VH | 15 |
| | VL | 16 |
| 3 | VH | 31 |
| | VL | 32 |
| 4 | VH | 39 |
| | VL | 40 |
| 5 | VH | 47 |
| | VL | 48 |
| 6 | VH | 55 |
| | VL | 56 |
| 7 | VH | 63 |
| | VL | 64 |
| 8 | VH | 71 |
| | VL | 72 |
| 9 | VH | 79 |
| | VL | 80 |
| 10 | VH | 87 |
| | VL | 88 |
| 11 | VH | 95 |
| | VL | 96 |
| 12 | VH | 103 |
| | VL | 104 |
| 13 | VH | 111 |
| | VL | 112 |
| 14 | VH | 119 |
| | VL | 120 |
| 15 | VH | 127 |
| | VL | 128 |
| 16 | VH | 135 |
| | VL | 136 |
| 17 | VH | 143 |
| | VL | 144 |
| 18 | VH | 151 |
| | VL | 152 |
| 19 | VH | 159 |
| | VL | 160 |
| 20 | VH | 167 |
| | VL | 168 |

(continued)

| Group | Description | SEQ ID NO: |
|-------|-------------|------------|
| 21 | VH | 175 |
| 21 | VL | 176 |
| 22 | VH | 183 |
| 22 | VL | 184 |
| 23 | VH | 191 |
| 23 | VL | 192 |
| 24 | VH | 199 |
| 24 | VL | 200 |
| 25 | VH | 207 |
| 25 | VL | 208 |
| 26 | VH | 215 |
| 26 | VL | 216 |
| 27 | VH | 223 |
| 27 | VL | 224 |
| 28 | VH | 231 |
| 28 | VL | 232 |
| 29 | VH | 239 |
| 29 | VL | 240 |
| 30 | VH | 247 |
| 30 | VL | 248 |
| 31 | VH | 255 |
| 31 | VL | 256 |
| 32 | VH | 263 |
| 32 | VL | 264 |
| 33 | VH | 271 |
| 33 | VL | 272 |
| 34 | VH | 279 |
| 34 | VL | 280 |
| 35 | VH | 287 |
| 35 | VL | 288 |
| 36 | VH | 295 |
| 36 | VL | 296 |
| 37 | VH | 303 |
| 37 | VL | 304 |
| 38 | VH | 311 |
| 38 | VL | 312 |
| 39 | VH | 319 |
| 39 | VL | 320 |

(continued)

| Group | Description | SEQ ID NO: |
|---|---|---|
| 40 | VH | 327 |
| | VL | 328 |
| 41 | VH | 335 |
| | VL | 336 |
| 42 | VH | 343 |
| | VL | 344 |
| 43 | VH | 351 |
| | VL | 352 |
| 44 | VH | 359 |
| | VL | 360 |
| 45 | VH | 367 |
| | VL | 368 |
| 46 | VH | 375 |
| | VL | 376 |
| 47 | VH | 383 |
| | VL | 384 |
| 48 | VH | 391 |
| | VL | 392 |
| 49 | VH | 399 |
| | VL | 400 |
| 50 | VH | 407 |
| | VL | 408 |

5. The antibody or antigen-binding fragment thereof according to any one of claims 1-4, wherein the antibody is a humanized antibody.

6. The antibody or antigen-binding fragment thereof according to claim 5, wherein the humanized antibody

i) the heavy chain variable region comprises the amino acid sequence set forth in SEQ ID NO: 23, and the light chain variable region comprises the amino acid sequence set forth in SEQ ID NO: 24;
ii) the heavy chain variable region comprises the amino acid sequence set forth in SEQ ID NO: 25, and the light chain variable region comprises the amino acid sequence set forth in SEQ ID NO: 26;
iii) the heavy chain variable region comprises the amino acid sequence set forth in SEQ ID NO: 27, and the light chain variable region comprises the amino acid sequence set forth in SEQ ID NO: 28; or
iv) the heavy chain variable region comprises the amino acid sequence set forth in SEQ ID NO: 29, and the light chain variable region comprises the amino acid sequence set forth in SEQ ID NO: 30.

7. The antibody or antigen-binding fragment thereof according to any one of claims 1-4, wherein the antibody comprises a heavy chain set forth in SEQ ID NO: 3 and a light chain set forth in SEQ ID NO: 4; or the antibody comprises a heavy chain set forth in SEQ ID NO: 13 and a light chain set forth in SEQ ID NO: 14.

8. The antibody or antigen-binding fragment thereof according to any one of claims 1-7, wherein the antibody or antigen-binding fragment thereof is capable of inhibiting tumor growth by at least about 10%, preferably at least about 20%, more preferably at least about 30%, more preferably at least about 40%, more preferably at least about 50%, more preferably at least about 60%, more preferably at least about 70%, more preferably at least about 80%.

9. The antibody or antigen-binding fragment thereof according to any one of claims 1-8, wherein the antibody or antigen-binding fragment thereof binds to the extracellular region of the cTAGE5 protein comprising an amino acid sequence set forth in SEQ ID NO: 1 or 2 or the immunogenic polypeptide comprising an amino acid sequence set forth in SEQ ID NO: 1 or 2 with a $K_D$ value of less than $1 \times 10^{-7}$M, preferably less than $1 \times 10^{-8}$M, more preferably less than $1 \times 10^{-9}$M, more preferably less than $1 \times 10^{-10}$M.

10. An antibody conjugate comprising the antibody or antigen-binding fragment thereof according to any one of claims 1-9, and a therapeutic moiety selected from the group consisting of a cytotoxin, a radioisotope or a biologically active protein conjugated to the antibody or antigen-binding fragment thereof.

11. A pharmaceutical composition comprising the antibody or antigen-binding fragment thereof of any one of claims 1-9 or the antibody conjugate of claim 10, and a pharmaceutically acceptable carrier.

12. A method of treating and/or preventing a tumor in a subject, comprising administering to the subject an effective amount of the antibody or antigen-binding fragment thereof of any one of claims 1-9, or the antibody conjugate of claim 10, or the pharmaceutical composition of claim 11.

13. The method of claim 12, wherein the tumor is a cTAGE5 protein-associated tumor, such as a tumor overexpressing a cTAGE5 protein, wherein the cTAGE5 protein has an extracellular region, and the extracellular region of the cTAGE5 protein comprises an amino acid sequence shown in SEQ ID NO: 1 or 2.

14. The method of claim 12 or 13, wherein the tumor is selected from the group consisting of nasopharyngeal cancer, malignant melanoma, lung cancer, skin cancer, glossopharyngeal cancer, lymphoma (including B-cell lymphoma or T-cell lymphoma), liver cancer, cervical cancer, thyroid cancer, colon cancer, ovarian cancer, brain tumor, prostate cancer, breast cancer, esophageal cancer, gastric cancer, pancreatic cancer, rectal cancer.

15. The method of any one of claims 12-14, further comprising administering to the subject other anti-tumor treatment means, such as administering a chemotherapeutic agent, an antibody targeting other tumor-specific antigen, or radiotherapy.

16. Use of the antibody or antigen-binding fragment thereof according to any one of claims 1-9, the antibody conjugate according to claim 10 or the pharmaceutical composition according to claim 11 in the preparation of a medicament for treating and/or preventing a tumor.

17. The use according to claim 16, wherein the tumor is a cTAGE5 protein-related tumor, such as a tumor overexpressing a cTAGE5 protein, wherein the cTAGE5 protein has an extracellular region, and the extracellular region of the cTAGE5 protein comprises an amino acid sequence shown in SEQ ID NO: 1 or 2.

18. The method of claim 17, wherein the tumor is selected from the group consisting of nasopharyngeal cancer, malignant melanoma, lung cancer, glioma, skin cancer, glossopharyngeal cancer, lymphoma (including B-cell lymphoma or T-cell lymphoma), liver cancer, cervical cancer, thyroid cancer, colon cancer, ovarian cancer, brain tumor, prostate cancer, breast cancer, esophageal cancer, gastric cancer, pancreatic cancer, rectal cancer, endometrial cancer, and colorectal cancer.

19. A method for detecting the presence or expression level of cTAGE5 protein in a biological sample, comprising:

   contacting the biological sample and optionally a control sample with an antibody, such as a monoclonal antibody or an antigen-binding fragment thereof of the present invention, under a condition allowing the formation of a complex between the antibody or the antigen-binding fragment thereof of any one of claims 1-9 and a cTAGE5 protein; and
   detecting the formation of the complex,
   wherein the formation of the complex in the biological sample or the difference in the formation of the complex between the biological sample and the control sample indicates the presence of a cTAGE5 protein or the expression level of cTAGE5 protein in the sample,
   wherein the cTAGE5 protein has an extracellular region comprising the amino acid sequence set forth in SEQ ID NO: 1 or 2.

20. The method of claim 19, wherein the biological sample is an ex vivo sample, such as a tissue sample, a blood sample, a lymphatic sample, and the like.

21. The method of claim 19 or 20, which is an immunohistochemical assay or an immunofluorescence assay.

22. A method of detecting the presence of a tumor in a subject, comprising detecting, for example by the method of any one of claims 19-21, the expression level of cTAGE5 protein in a biological sample from the subject,

wherein the presence of cTAGE5 in the biological sample from the subject or the expression level of cTAGE5 in the biological sample from the subject being higher than the expression level of cTAGE5 from the control biological sample indicates the presence of a tumor in the subject,
wherein the cTAGE5 protein has an extracellular region comprising an amino acid sequence set forth in SEQ ID NO: 1 or 2.

23. The method of claim 22, wherein the tumor is a cTAGE5 protein-associated tumor, such as a tumor overexpressing a cTAGE5 protein, wherein the cTAGE5 protein has an extracellular region, and the extracellular region of the cTAGE5 protein comprises the amino acid sequence shown in SEQ ID NO: 1 or 2.

24. The method of claim 23, wherein the tumor is selected from the group consisting of nasopharyngeal cancer, malignant melanoma, lung cancer, glioma, skin cancer, glossopharyngeal cancer, lymphoma (including B-cell lymphoma or T-cell lymphoma), liver cancer, cervical cancer, thyroid cancer, colon cancer, ovarian cancer, brain tumor, prostate cancer, breast cancer, esophageal cancer, gastric cancer, pancreatic cancer, rectal cancer, endometrial cancer, and colorectal cancer.

25. An isolated immunogenic polypeptide, comprising the amino acid sequence shown in SEQ ID NO: 1 or SEQ ID NO: 2, or comprising the amino acid sequence having at least 80%, at least 90%, or at least 95% sequence identity with SEQ ID NO: 1 or SEQ ID NO: 2, or comprising an amino acid sequence having one or more (e.g., 1, 2, 3, 4, or 5) amino acid substitutions, deletions, or additions relative to SEQ ID NO: 1 or SEQ ID NO: 2.

26. Use of the isolated immunogenic polypeptide of claim 25 in generating an antibody.

27. A method of generating an antibody comprising immunizing an animal with the isolated immunogenic polypeptide of claim 25, and then isolating an antibody that specifically binds to the polypeptide from the animal or obtaining sequence information of an antibody that specifically binds to the polypeptide.

28. A pharmaceutical composition comprising the isolated immunogenic polypeptide of claim 25 and a pharmaceutically acceptable carrier.

29. Use of the isolated immunogenic polypeptide of claim 25 in the manufacture of a medicament, for example, a medicament for treating and/or preventing a tumor.

30. A method of preventing and/or treating a tumor in a subject in need thereof, comprising administering to the subject an effective amount of the isolated immunogenic polypeptide of claim 25 or the pharmaceutical composition of claim 28.

31. A method of detecting the presence and/or amount of an antibody that specifically binds to cTAGE5 protein in a biological sample comprising 1) contacting the isolated immunogenic polypeptide of claim 25 with the biological sample, and 2) detecting the presence and/or amount of the antibody that binds to the isolated immunogenic polypeptide.

32. Use of the isolated immunogenic polypeptide of claim 25 in the manufacture of a kit for detecting the presence and/or amount of an antibody that specifically binds to cTAGE5 protein in a biological sample.

33. Use of a cTAGE5 protein comprising the amino acid sequence shown in SEQ ID NO: 1 or 2, or comprising an amino acid sequence having at least 80%, at least 90%, or at least 95% sequence identity with SEQ ID NO: 1, or comprising an amino acid sequence having one or more (e.g., 1, 2, 3, 4, or 5) amino acid substitutions, deletions, or additions relative to SEQ ID NO: 1, preferably comprising the amino acid sequence shown in SEQ ID NO: 2, as a tumor marker.

34. A method for screening a tumor, comprising detecting the presence or amount of a cTAGE5 protein in a biological sample from a subject, wherein the cTAGE5 protein comprises an amino acid sequence shown in SEQ ID NO: 1 or 2, or comprises an amino acid sequence having at least 80%, at least 90%, or at least 95% sequence identity to SEQ ID NO: 1, or comprises an amino acid sequence having one or more (e.g., 1, 2, 3, 4, or 5) amino acid substitutions, deletions, or additions relative to SEQ ID NO: 1, preferably comprising the amino acid sequence shown in SEQ ID NO: 2.

35. A method for screening a tumor, comprising detecting the presence and/or amount of an antibody that specifically binds to cTAGE5 protein in a biological sample from a subject, wherein the antibody specifically binds to an extracellular region of the cTAGE5 protein, and the extracellular region of the cTAGE5 protein comprises an amino acid sequence as shown in SEQ ID NO: 1 or 2, or comprises an amino acid sequence having at least 80%, at least 90%, or at least 95% sequence identity with SEQ ID NO: 1, or comprises an amino acid sequence having one or more (e.g., 1, 2, 3, 4, or 5) amino acid substitutions, deletions, or additions relative to SEQ ID NO: 1, preferably comprising an amino acid sequence as shown in SEQ ID NO: 2.

FIG. 1

Anti-ZZS01, malignant melanoma    anti-ZZS05, esophageal cancer    anti-ZZS05, T-cell lymphoma

FIG. 2

A.

Nasopharyngeal Cancer               Malignant Melanoma

ID6 IHC Representative Results

B.

Lung adenocarcinoma               gastric cancer

F11 IHC Representative Results

FIG. 3

FIG. 4

FIG. 5

A

B

FIG. 6

FIG.7

FIG.8

## INTERNATIONAL SEARCH REPORT

| International application No. |
|---|
| **PCT/CN2024/107513** |

| A. | CLASSIFICATION OF SUBJECT MATTER |
|---|---|

C07K 16/18(2006.01)i; C12Q1/68(2018.01)i; A61K39/395(2006.01)i; A61K31/713(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC:C07K,C12Q,A61K,A61P

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, EBI, ISI Web of Science, CNKI, BAIDU, BING, STN: cTAGE5, MEA6, MGEA6, MEA11, MGEA, MTEA, 抗体, 单克隆抗体, CDR, antibody, monoclonal antibody, 肿瘤, 癌, tumor, cancer, sequences 1-414

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2011156970 A1 (XU, Zhiheng et al.) 22 December 2011 (2011-12-22) claims 1-12, description, page 3, paragraph 1, page 9, last paragraph and page 12, paragraph 1, and embodiment 1 | 1-2, 5, 8-35 |
| Y | WO 2011156970 A1 (XU, Zhiheng et al.) 22 December 2011 (2011-12-22) claims 1-12, description, page 3, paragraph 1, page 9, last paragraph and page 12, paragraph 1, and embodiment 1 | 28-30, 33-35 |
| X | NCBI. "GenBank Accession NO: XP_016876803.1" *NCBI GenBank*, 20 March 2023 (2023-03-20), specific sequence information | 25-27, 31-32 |
| Y | NCBI. "GenBank Accession NO: XP_016876803.1" *NCBI GenBank*, 20 March 2023 (2023-03-20), specific sequence information | 28-30, 33-35 |
| X | NCBI. "GenBank Accession NO: XP_016876805.1" *NCBI GenBank*, 20 March 2023 (2023-03-20), specific sequence information | 25-27, 31-32 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **31 October 2024** | **06 November 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

EP 4 752 156 A1

**INTERNATIONAL SEARCH REPORT**

International application No.

**PCT/CN2024/107513**

**C.    DOCUMENTS CONSIDERED TO BE RELEVANT**

| Category* | Citation of document, with indication, where appropriate, of the relevant passages | Relevant to claim No. |
|---|---|---|
| Y | NCBI. "GenBank Accession NO: XP_016876805.1" <br> *NCBI GenBank*, 20 March 2023 (2023-03-20), <br> specific sequence information | 28-30, 33-35 |
| A | CN 103347898 A (CT ATLANTIC LTD. et al.) 09 October 2013 (2013-10-09) <br> entire document | 1-35 |
| A | US 2022155305 A1 (YEDA RESEARCH AND DEVELOPMENT CO., LTD.) 19 May 2022 (2022-05-19) <br> entire document | 1-35 |
| A | WO 2023034864 A1 (YALE UNIVERSITY et al.) 09 March 2023 (2023-03-09) <br> entire document | 1-35 |

Form PCT/ISA/210 (second sheet) (July 2022)

**INTERNATIONAL SEARCH REPORT**

International application No.

**PCT/CN2024/107513**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2024/107513**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **12-15, 30**
   because they relate to subject matter not required to be searched by this Authority, namely:

   Claims 12-15 relate to a method for treating and/or preventing tumors in a patient, and claim 30 relates to a method for preventing and/or treating tumors in a subject in need thereof, which fall within disease treatment methods as defined in PCT Rule 39.1(iv). The present report is provided on the basis of the corresponding pharmaceutical use of products such as the antibody.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/107513**

| Patent document cited in search report | | | Publication date (day/month/year) | Patent family member(s) | | | Publication date (day/month/year) |
|---|---|---|---|---|---|---|---|
| WO | 2011156970 | A1 | 22 December 2011 | None | | | |
| CN | 103347898 | A | 09 October 2013 | KR | 20130114196 | A | 16 October 2013 |
| | | | | KR | 101614997 | B1 | 22 April 2016 |
| | | | | WO | 2012095412 | A1 | 19 July 2012 |
| | | | | HUE | 031197 | T2 | 28 June 2017 |
| | | | | US | 2018353603 | A1 | 13 December 2018 |
| | | | | JP | 2014505050 | A | 27 February 2014 |
| | | | | JP | 5828909 | B2 | 09 December 2015 |
| | | | | ES | 2617983 | T3 | 20 June 2017 |
| | | | | IL | 226675 | A0 | 31 July 2013 |
| | | | | EP | 2663580 | A1 | 20 November 2013 |
| | | | | EP | 2663580 | B1 | 07 December 2016 |
| | | | | DK | 2663580 | T3 | 13 March 2017 |
| | | | | PL | 2663580 | T3 | 31 August 2017 |
| | | | | CA | 2821117 | A1 | 19 July 2012 |
| | | | | CA | 2821117 | C | 12 February 2019 |
| | | | | US | 2013295091 | A1 | 07 November 2013 |
| | | | | PT | 2663580 | T | 10 March 2017 |
| US | 2022155305 | A1 | 19 May 2022 | WO | 2021019526 | A1 | 04 February 2021 |
| | | | | CA | 3147575 | A1 | 04 February 2021 |
| | | | | EP | 4004548 | A1 | 01 June 2022 |
| WO | 2023034864 | A1 | 09 March 2023 | AU | 2022340804 | A1 | 21 March 2024 |
| | | | | EP | 4395832 | A1 | 10 July 2024 |
| | | | | JP | 2024532621 | A | 05 September 2024 |

Form PCT/ISA/210 (patent family annex) (July 2022)

# EP 4 752 156 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7229619 B

- CN 108486126 A

### Non-patent literature cited in the description

- Methods in Molecular Biology. **KIPRIYANOV**. Recombinant Antibodies for Cancer Therapy Methods and Protocols. 2003, vol. 207, 3-25
- **SMITH et al.** *J. Clin. Pathol*, 2004, vol. 57, 912-917
- **NELSON et al.** *J Clin Pathol*, 2000, vol. 53, 111-117
- **KABAT, E.A. et al.** Sequences of Proteins of Immunological Interest. U.S. Department of Health and Human Services, 1991
- **CHOTHIA, C et al.** *J. Mol. Biol.*, 1987, vol. 196, 901-917
- **RICH** ; **MYSZKA**. *Curr. Opin. Biotechnol*, 2000, vol. 11, 54
- **ENGLEBIENNE**. *Analyst*, 1998, vol. 123, 1599
- Fundamental Immunology. Raven Press, 1989, 332-336
- **MALMQVIST**. *Biochem. Soc. Trans*, 2000, vol. 27, 335
- **WATSON et al.** Molecular Biology of the Gene. The Benjamin/Cummings Pub. co., 1987, 224
- Computational Molecular Biology. Oxford University Press, 1988
- Biocomputing: Informatics and Genome Projects. Academic Press, 1993
- Computer Analysis of Sequence Data. Humana Press, 1994, vol. I
- **VON HEINJE, G.** Sequence Analysis in Molecular Biology. Academic Press, 1987

- Sequence Analysis Primer. M Stockton Press, 1991
- **CARRILLO, H** ; **LIPMAN, D.** *SIAM J Applied Math*, 1988, vol. 48, 1073
- **NAKAMURA Y**. Codon usage tabulated from the international DNA sequence databases: status for the year2000. *Nucl.Acids Res*, 2000, vol. 28, 292
- Sustained and Controlled Release Drug Delivery Systems. Marcel Dekker, Inc., 1978
- **CHOTHIA C** ; **LESK A M**. Canonical structures for the hypervariable regions of immunoglobulins. *J Mol Biol.*, 1987, vol. 196, 901-17
- **LEFRANC, M.P**. IMGT, the international ImMunoGeneTics information system: A standardized approach for immunogenetics and immunoinformatics. *Immunome Res*, 2005, vol. 1, 3
- **LEFRANC, M.P.** ; **GIUDICELLI, V.** ; **KAAS, Q.** ; **DUPRAT, E.** ; **JABADO-MICHALOUD, J.** ; **SCAVINER, D.** ; **GINESTOUX, C.** ; **CLEMENT, O.** ; **CHAUME, D.** ; **LEFRANC, G**. IMGT, the international ImMunoGeneTics information system. *Nucleic Acids Res.*, 2005, vol. 33, D593-D597
- **DAVID PRIHODA** ; **JAD MAAMARYC** ; **ANDREW WAIGHTD** ; **VERONICA JUAND** ; **LAURENCE FAYADAT-DILMAND** ; **DANIEL SVOZIL A,E** ; **DANNY A**. *Bitton :BioPhi: A platform for antibody design, humanization, and humanness evaluation based on natural antibody repertoires and deep learning*